# EUROPEAN PATENT SPECIFICATION

(11) **EP 1 522 586 B1**
(45) Date of publication and mention of the grant of the patent: **15.08.2007**
(21) Application number: 04024045.9
(22) Date of filing: 08.10.2004
(51) Int. Cl.: C12N 15/82, C07K 14/415, C12N 5/10

(54) **Transformed cell co-expressing cytokinin receptor and cytokinin biosynthesis enzyme**
Transformierte Zellen, die einen Cytokinin Rezeptor und ein Cytokinin Biosynthese-Enzym koexprimieren
Cellules transformées qui co-expriment un récepteur de cytokinine et une enzyme de biosynthèse de cytokinine

(30) Priority: 10.10.2003 JP 2003351758
(43) Date of publication of application: 13.04.2005
(73) Proprietor: Sumitomo Chemical Company, Limited, Tokyo 104-8260 (JP)
(72) Inventor: Kakimoto, Tatsuo, Toyonaka-shi, Osaka (JP); Higuchi, Masayuki, Okayama-shi, Okayama (JP)
(74) Representative: Vossius & Partner

(56) References cited:
- EP-A- 1 241 182
- EP-A- 1 384 776
- WO-A-99/06579
- KAKIMOTO TATSUO: "Perception and signal transduction of cytokinins." ANNUAL REVIEW OF PLANT BIOLOGY. VOLUME 54 ANNUAL REVIEWS, 4139 EL CAMINO WAY, P. O. BOX 10139, PALO ALTO, CA, 94303-0139, USA SERIES : ANNUAL REVIEW OF PLANT BIOLOGY, 2003, pages 605-627, XP002313108 ISSN: 0-8243-0654-6
- KAKIMOTO TATSUO: "Biosynthesis of cytokinins." JOURNAL OF PLANT RESEARCH, vol. 116, no. 3, June 2003 (2003-06), pages 233-239, XP002313109 ISSN: 0918-9440
- TATSUYA MAEDA ET AL: "A two-component system that regulates an osmosensing MAP kinase cascade in yeast" NATURE, MACMILLAN JOURNALS LTD. LONDON, GB, vol. 369, no. 6477, 19 May 1994 (1994-05-19), pages 242-245, XP002129354 ISSN: 0028-0836

### SEQUENCE LISTING

<110> Sumitomo Chemical Company Limited
<120> TRANSFORMED CELL CO-EXPRESSING CYTOKININ RECEPTOR AND CYTOKININ BIOSYNTHESIS ENZYME
<130>
<160> 38
<210> 1
   <211> 3174
   <212> DNA
   <213> Arabidopsis thaliana
<220>
   <221> CDS
   <222> (1).. (3174)
<400> 1
   atg aac tgg gca ctc aac aat cat caa gaa gaa gaa gaa gag cca cga 48
   Met Asn Trp Ala Leu Asn Asn His Gln Glu Glu Glu Glu Glu Pro Arg
   1 5 10 15
   aga att gaa att tct gat tcc gag tca cta gaa aac ttg aaa agc agc 96
   Arg Ile Glu Ile Ser Asp Ser Glu Ser Leu Glu Asn Leu Lys Ser Ser
   20 25 30
   gat ttt tat caa ctg ggt ggt ggt ggt gct ctg aat tcg tca gaa aag 144
   Asp Phe Tyr Gln Leu Gly Gly Gly Gly Ala Leu Asn Ser Ser Glu Lys
   35 40 45
   ccg aga aag atc gat ttt tgg cgt tcg ggg ttg atg ggt ttt gcg aag 192
   Pro Arg Lys Ile Asp Phe Trp Arg Ser Gly Leu Met Gly Phe Ala Lys
   50 55 60
   atg cag cag cag caa cag ctt cag cat tca gtg gcg gtg aag atg aac 240
   Met Gln Gln Gln Gln Gln Leu Gln His Ser Val Ala Val Lys Met Asn
   65 70 75 80
   aat aat aat aat aac gat cta atg ggt aat aaa aaa ggg tca act ttc 288
   Asn Asn Asn Asn Asn Asp Leu Met Gly Asn Lys Lys Gly Ser Thr Phe
   85 90 95
   ata caa gaa cat cga gca ttg tta cca aaa gct ttg att ctg tgg atc 336
   Ile Gln Glu His Arg Ala Leu Leu Pro Lys Ala Leu Ile Leu Trp Ile
   100 105 110
   atc att gtt ggg ttt ata agc agt ggg att tat cag tgg atg gat gat 384
   Ile Ile Val Gly Phe Ile Ser Ser Gly Ile Tyr Gln Trp Met Asp Asp
   115 120 125
   gct aat aag att aga agg gaa gag gtt ttg gtc agc atg tgt gat caa 432
   Ala Asn Lys Ile Arg Arg Glu Glu Val Leu Val Ser Met Cys Asp Gln
   130 135 140
   aga gct aga atg ttg cag gat caa ttt agt gtt agt gtt aat cat gtt 480
   Arg Ala Arg Met Leu Gln Asp Gln Phe Ser Val Ser Val Asn His Val
   145 150 155 160
   cat gct ttg gct att ctc gtc tcc act ttt cat tac cac aag aac cct 528
   His Ala Leu Ala Ile Leu Val Ser Thr Phe His Tyr His Lys Asn Pro
   165 170 175
   tct gca att gat cag gag aca ttt gcg gag tac acg gca aga aca gca 576
   Ser Ala Ile Asp Gln Glu Thr Phe Ala Glu Tyr Thr Ala Arg Thr Ala
   180 185 190
   ttt gag aga ccg ttg cta agt gga gtg gct tat gct gaa aaa gtt gtg 624
   Phe Glu Arg Pro Leu Leu Ser Gly Val Ala Tyr Ala Glu Lys Val Val
   195 200 205
   aat ttt gag agg gag atg ttt gag cgg cag cac aat tgg gtt ata aag 672
   Asn Phe Glu Arg Glu Met Phe Glu Arg Gln His Asn Trp Val Ile Lys
   210 215 220
   aca atg gat aga gga gag cct tca ccg gtt agg gat gag tat gct cct 720
   Thr Met Asp Arg Gly Glu Pro Ser Pro Val Arg Asp Glu Tyr Ala Pro
   225 230 235 240
   gtt ata ttc tct caa gat agt gtc tct tac ctt gag tca ctc gat atg 768
   Val Ile Phe Ser Gln Asp Ser Val Ser Tyr Leu Glu Ser Leu Asp Met
   245 250 255
   atg tca ggc gag gag gat cgt gag aat att ttg cga gct aga gaa acc 816
   Met Ser Gly Glu Glu Asp Arg Glu Asn Ile Leu Arg Ala Arg Glu Thr
   260 265 270
   gga aaa gct gtc ttg act agc cct ttt agg ttg ttg gaa act cac cat 864
   Gly Lys Ala Val Leu Thr Ser Pro Phe Arg Leu Leu Glu Thr His His
   275 280 285
   ctc gga gtt gtg ttg aca ttc cct gtc tac aag tct tct ctt cct gaa 912
   Leu Gly Val Val Leu Thr Phe Pro Val Tyr Lys Ser Ser Leu Pro Glu
   290 295 300
   aat ccg act gtc gaa gag cgt att gca gcc act gca ggg tac ctt ggt 960
   Asn Pro Thr Val Glu Glu Arg Ile Ala Ala Thr Ala Gly Tyr Leu Gly
   305 310 315 320
   ggt gcg ttt gat gtg gag tct cta gtc gag aat tta ctt ggt cag ctt 1008
   Gly Ala Phe Asp Val Glu Ser Leu Val Glu Asn Leu Leu Gly Gln Leu
   325 330 335
   gct ggt aac caa gca ata gtt gtg cat gtg tat gat atc acc aat gca 1056
   Ala Gly Asn Gln Ala Ile Val Val His Val Tyr Asp Ile Thr Asn Ala
   340 345 350
   tca gat cca ctt gtc atg tat ggt aat caa gat gaa gaa gcc gac aga 1104
   Ser Asp Pro Leu Val Met Tyr Gly Asn Gln Asp Glu Glu Ala Asp Arg
   355 360 365
   tct ctc tct cat gag agc aag ctc gat ttt gga gac ccc ttc agg aaa 1152
   Ser Leu Ser His Glu Ser Lys Leu Asp Phe Gly Asp Pro Phe Arg Lys
   370 375 380
   cat aag atg ata tgc agg tac cac caa aag gca cca ata cca ttg aat 1200
   His Lys Met Ile Cys Arg Tyr His Gln Lys Ala Pro Ile Pro Leu Asn
   385 390 395 400
   gtg ctc aca act gtg cca ttg ttc ttt gcg att ggt ttc ttg gtg ggt 1248
   Val Leu Thr Thr Val Pro Leu Phe Phe Ala Ile Gly Phe Leu Val Gly
   405 410 415
   tat ata ctg tat ggt gca gct atg cac ata gta aaa gtc gaa gat gat 1296
   Tyr Ile Leu Tyr Gly Ala Ala Met His Ile Val Lys Val Glu Asp Asp
   420 425 430
   ttc cat gaa atg caa gag ctt aaa gtg cga gca gaa gct gct gat gtc 1344
   Phe His Glu Met Gln Glu Leu Lys Val Arg Ala Glu Ala Ala Asp Val
   435 440 445
   gct aaa tcg cag ttt ctt gct acc gtg tct cac gag atc agg aca cca 1392
   Ala Lys Ser Gln Phe Leu Ala Thr Val Ser His Glu Ile Arg Thr Pro
   450 455 460
   atg aat ggc att ctc gga atg ctt gct atg ctc cta gat aca gaa cta 1440
   Met Asn Gly Ile Leu Gly Met Leu Ala Met Leu Leu Asp Thr Glu Leu
   465 470 475 480
   agc tcg aca cag aga gat tac gct caa acc gct caa gta tgt ggt aaa 1488
   Ser Ser Thr Gln Arg Asp Tyr Ala Gln Thr Ala Gln Val Cys Gly Lys
   485 490 495
   gct ttg att gca ttg ata aat gag gtt ctt gat cgc gcc aag att gaa 1536
   Ala Leu Ile Ala Leu Ile Asn Glu Val Leu Asp Arg Ala Lys Ile Glu
   500 505 510
   gct gga aag ctg gag ttg gaa tca gta cca ttt gat atc cgt tca ata 1584
   Ala Gly Lys Leu Glu Leu Glu Ser Val Pro Phe Asp Ile Arg Ser Ile
   515 520 525
   ttg gat gat gtc ctt tct cta ttc tct gag gag tca agg aac aaa ggc 1632
   Leu Asp Asp Val Leu Ser Leu Phe Ser Glu Glu Ser Arg Asn Lys Gly
   530 535 540
   att gag ctc gcg gtt ttc gtt tca gac aaa gta cca gag ata gtc aaa 1680
   Ile Glu Leu Ala Val Phe Val Ser Asp Lys Val Pro Glu Ile Val Lys
   545 550 555 560
   gga gat tca ggg aga ttt aga cag ata atc ata aac ctt gtt gga aat 1728
   Gly Asp Ser Gly Arg Phe Arg Gln Ile Ile Ile Asn Leu Val Gly Asn
   565 570 575
   tcg gtt aaa ttc aca gag aaa gga cat atc ttt gtt aaa gtc cat ctt 1776
   Ser Val Lys Phe Thr Glu Lys Gly His Ile Phe Val Lys Val His Leu
   580 585 590
   gcg gaa caa tca aaa gat gaa tct gaa ccg aaa aat gca ttg aat ggt 1824
   Ala Glu Gln Ser Lys Asp Glu Ser Glu Pro Lys Asn Ala Leu Asn Gly
   595 600 605
   gga gtg tct gaa gaa atg atc gtt gtt tcc aaa cag tca agt tac aac 1872
   Gly Val Ser Glu Glu Met Ile Val Val Ser Lys Gln Ser Ser Tyr Asn
   610 615 620
   aca ttg agc ggt tac gaa gct gct gat ggt cgg aat agc tgg gat tca 1920
   Thr Leu Ser Gly Tyr Glu Ala Ala Asp Gly Arg Asn Ser Trp Asp Ser
   625 630 635 640
   ttc aag cat ttg gtc tct gag gag cag tca tta tcg gag ttt gat att 1968
   Phe Lys His Leu Val Ser Glu Glu Gln Ser Leu Ser Glu Phe Asp Ile
   645 650 655
   tct agc aat gtt agg ctt atg gtt tca atc gaa gac acg ggt att gga 2016
   Ser Ser Asn Val Arg Leu Met Val Ser Ile Glu Asp Thr Gly Ile Gly
   660 665 670
   atc cct tta gtt gca caa ggc cgt gtg ttt atg ccg ttt atg caa gca 2064
   Ile Pro Leu Val Ala Gln Gly Arg Val Phe Met Pro Phe Met Gln Ala
   675 680 685
   gat agc tcg act tca aga aac tat gga ggt act ggt att ggt ttg agt 2112
   Asp Ser Ser Thr Ser Arg Asn Tyr Gly Gly Thr Gly Ile Gly Leu Ser
   690 695 700
   ata agc aag tgt ctt gtt gaa ctt atg cgt ggt cag ata aat ttc ata 2160
   Ile Ser Lys Cys Leu Val Glu Leu Met Arg Gly Gln Ile Asn Phe Ile
   705 710 715 720
   agc cgg cct cat att gga agc acg ttc tgg ttc acg gct gtt tta gag 2208
   Ser Arg Pro His Ile Gly Ser Thr Phe Trp Phe Thr Ala Val Leu Glu
   725 730 735
   aaa tgc gat aaa tgc agt gcg att aac cat atg aag aaa cct aat gtg 2256
   Lys Cys Asp Lys Cys Ser Ala Ile Asn His Met Lys Lys Pro Asn Val
   740 745 750
   gaa cac ttg cct tct act ttt aaa gga atg aaa gct ata gtt gtt gat 2304
   Glu His Leu Pro Ser Thr Phe Lys Gly Met Lys Ala Ile Val Val Asp
   755 760 765
   gct aag cct gtt aga gct gct gtg act aga tac cat atg aaa aga ctc 2352
   Ala Lys Pro Val Arg Ala Ala Val Thr Arg Tyr His Met Lys Arg Leu
   770 775 780
   gga atc aat gtt gat gtc gtg aca agt ctc aaa acc gct gtt gtt gca 2400
   Gly Ile Asn Val Asp Val Val Thr Ser Leu Lys Thr Ala Val Val Ala
   785 790 795 800
   gct gct gcg ttt gaa aga aac ggt tct cct ctc cca aca aaa ccg caa 2448
   Ala Ala Ala Phe Glu Arg Asn Gly Ser Pro Leu Pro Thr Lys Pro Gln
   805 810 815
   ctt gat atg atc tta gta gag aaa gat tca tgg att tca act gaa gat 2496
   Leu Asp Met Ile Leu Val Glu Lys Asp Ser Trp Ile Ser Thr Glu Asp
   820 825 830
   aat gac tca gag att cgt tta ttg aat tca aga acc aac gga aac gtt 2544
   Asn Asp Ser Glu Ile Arg Leu Leu Asn Ser Arg Thr Asn Gly Asn Val
   835 840 845
   cat cac aag tct ccg aaa cta gct cta ttc gca aca aac atc aca aat 2592
   His His Lys Ser Pro Lys Leu Ala Leu Phe Ala Thr Asn Ile Thr Asn
   850 855 860
   tcg gag ttc gac aga gct aaa tcc gca gga ttt gca gat acg gta ata 2640
   Ser Glu Phe Asp Arg Ala Lys Ser Ala Gly Phe Ala Asp Thr Val Ile
   865 870 875 880
   atg aaa ccg tta aga gca agc atg att ggg gcg tgt ctg caa caa gtt 2688
   Met Lys Pro Leu Arg Ala Ser Met Ile Gly Ala Cys Leu Gln Gln Val
   885 890 895
   ctc gag ctg aga aaa aca aga caa caa cat cca gaa gga tca tca ccc 2736
   Leu Glu Leu Arg Lys Thr Arg Gln Gln His Pro Glu Gly Ser Ser Pro
   900 905 910
   gca act ctc aag agc ttg ctt aca ggg aag aag att ctt gtg gtt gat 2784
   Ala Thr Leu Lys Ser Leu Leu Thr Gly Lys Lys Ile Leu Val Val Asp
   915 920 925
   gat aat ata gtt aac agg aga gta gct gca gga gct ctc aag aaa ttt 2832
   Asp Asn Ile Val Asn Arg Arg Val Ala Ala Gly Ala Leu Lys Lys Phe
   930 935 940
   gga gca gaa gtg gtt tgt gca gag agt ggt caa gtt gct ttg ggt ttg 2880
   Gly Ala Glu Val Val Cys Ala Glu Ser Gly Gln Val Ala Leu Gly Leu
   945 950 955 960
   ctt cag att cca cac act ttc gat gct tgc ttc atg gat att caa atg 2928
   Leu Gln Ile Pro His Thr Phe Asp Ala Cys Phe Met Asp Ile Gln Met
   965 970 975
   cca cag atg gac gga ttt gaa gca act cgt cag ata aga atg atg gag 2976
   Pro Gln Met Asp Gly Phe Glu Ala Thr Arg Gln Ile Arg Met Met Glu
   980 985 990
   aag gaa gct aaa gag aag acg aat ctc gaa tgg cat tta ccg att cta 3024
   Lys Glu Ala Lys Glu Lys Thr Asn Leu Glu Trp His Leu Pro Ile Leu
   995 1000 1005
   gcg atg act gcg gat gtg ata cac gcg acc tac gag gaa tgt ctg aaa 3072
   Ala Met Thr Ala Asp Val Ile His Ala Thr Tyr Glu Glu Cys Leu Lys
   1010 1015 1020
   agt ggg atg gat ggt tac gtc tcc aaa cct ttt gaa gaa gag aat ctc 3120
   Ser Gly Met Asp Gly Tyr Val Ser Lys Pro Phe Glu Glu Glu Asn Leu
   1025 1030 1035 1040
   tat aaa tcc gtt gcc aaa tca ttc aaa cct aat cct atc tca cct tcg 3168
   Tyr Lys Ser Val Ala Lys Ser Phe Lys Pro Asn Pro Ile Ser Pro Ser
   1045 1050 1055
   tcg taa 3174
   Ser
<210> 2
   <211> 1057
   <212> PRT
   <213> Arabidopsis thaliana
<400> 2
   Met Asn Trp Ala Leu Asn Asn His Gln Glu Glu Glu Glu Glu Pro Arg
   1 5 10 15
   Arg Ile Glu Ile Ser Asp Ser Glu Ser Leu Glu Asn Leu Lys Ser Ser
   20 25 30
   Asp Phe Tyr Gln Leu Gly Gly Gly Gly Ala Leu Asn Ser Ser Glu Lys
   35 40 45
   Pro Arg Lys Ile Asp Phe Trp Arg Ser Gly Leu Met Gly Phe Ala Lys
   50 55 60
   Met Gln Gln Gln Gln Gln Leu Gln His Ser Val Ala Val Lys Met Asn
   65 70 75 80
   Asn Asn Asn Asn Asn Asp Leu Met Gly Asn Lys Lys Gly Ser Thr Phe
   85 90 95
   Ile Gln Glu His Arg Ala Leu Leu Pro Lys Ala Leu Ile Leu Trp Ile
   100 105 110
   Ile Ile Val Gly Phe Ile Ser Ser Gly Ile Tyr Gln Trp Met Asp Asp
   115 120 125
   Ala Asn Lys Ile Arg Arg Glu Glu Val Leu Val Ser Met Cys Asp Gln
   130 135 140
   Arg Ala Arg Met Leu Gln Asp Gln Phe Ser Val Ser Val Asn His Val
   145 150 155 160
   His Ala Leu Ala Ile Leu Val Ser Thr Phe His Tyr His Lys Asn Pro
   165 170 175
   Ser Ala Ile Asp Gln Glu Thr Phe Ala Glu Tyr Thr Ala Arg Thr Ala
   180 185 190
   Phe Glu Arg Pro Leu Leu Ser Gly Val Ala Tyr Ala Glu Lys Val Val
   195 200 205
   Asn Phe Glu Arg Glu Met Phe Glu Arg Gln His Asn Trp Val Ile Lys
   210 215 220
   Thr Met Asp Arg Gly Glu Pro Ser Pro Val Arg Asp Glu Tyr Ala Pro
   225 230 235 240
   Val Ile Phe Ser Gln Asp Ser Val Ser Tyr Leu Glu Ser Leu Asp Met
   245 250 255
   Met Ser Gly Glu Glu Asp Arg Glu Asn Ile Leu Arg Ala Arg Glu Thr
   260 265 270
   Gly Lys Ala Val Leu Thr Ser Pro Phe Arg Leu Leu Glu Thr His His
   275 280 285
   Leu Gly Val Val Leu Thr Phe Pro Val Tyr Lys Ser Ser Leu Pro Glu
   290 295 300
   Asn Pro Thr Val Glu Glu Arg Ile Ala Ala Thr Ala Gly Tyr Leu Gly
   305 310 315 320
   Gly Ala Phe Asp Val Glu Ser Leu Val Glu Asn Leu Leu Gly Gln Leu
   325 330 335
   Ala Gly Asn Gln Ala Ile Val Val His Val Tyr Asp Ile Thr Asn Ala
   340 345 350
   Ser Asp Pro Leu Val Met Tyr Gly Asn Gln Asp Glu Glu Ala Asp Arg
   355 360 365
   Ser Leu Ser His Glu Ser Lys Leu Asp Phe Gly Asp Pro Phe Arg Lys
   370 375 380
   His Lys Met Ile Cys Arg Tyr His Gln Lys Ala Pro Ile Pro Leu Asn
   385 390 395 400
   Val Leu Thr Thr Val Pro Leu Phe Phe Ala Ile Gly Phe Leu Val Gly
   405 410 415
   Tyr Ile Leu Tyr Gly Ala Ala Met His Ile Val Lys Val Glu Asp Asp
   420 425 430
   Phe His Glu Met Gln Glu Leu Lys Val Arg Ala Glu Ala Ala Asp Val
   435 440 445
   Ala Lys Ser Gln Phe Leu Ala Thr Val Ser His Glu Ile Arg Thr Pro
   450 455 460
   Met Asn Gly Ile Leu Gly Met Leu Ala Met Leu Leu Asp Thr Glu Leu
   465 470 475 480
   Ser Ser Thr Gln Arg Asp Tyr Ala Gln Thr Ala Gln Val Cys Gly Lys
   485 490 495
   Ala Leu Ile Ala Leu Ile Asn Glu Val Leu Asp Arg Ala Lys Ile Glu
   500 505 510
   Ala Gly Lys Leu Glu Leu Glu Ser Val Pro Phe Asp Ile Arg Ser Ile
   515 520 525
   Leu Asp Asp Val Leu Ser Leu Phe Ser Glu Glu Ser Arg Asn Lys Gly
   530 535 540
   Ile Glu Leu Ala Val Phe Val Ser Asp Lys Val Pro Glu Ile Val Lys
   545 550 555 560
   Gly Asp Ser Gly Arg Phe Arg Gln Ile Ile Ile Asn Leu Val Gly Asn
   565 570 575
   Ser Val Lys Phe Thr Glu Lys Gly His Ile Phe Val Lys Val His Leu
   580 585 590
   Ala Glu Gln Ser Lys Asp Glu Ser Glu Pro Lys Asn Ala Leu Asn Gly
   595 600 605
   Gly Val Ser Glu Glu Met Ile Val Val Ser Lys Gln Ser Ser Tyr Asn
   610 615 620
   Thr Leu Ser Gly Tyr Glu Ala Ala Asp Gly Arg Asn Ser Trp Asp Ser
   625 630 635 640
   Phe Lys His Leu Val Ser Glu Glu Gln Ser Leu Ser Glu Phe Asp Ile
   645 650 655
   Ser Ser Asn Val Arg Leu Met Val Ser Ile Glu Asp Thr Gly Ile Gly
   660 665 670
   Ile Pro Leu Val Ala Gln Gly Arg Val Phe Met Pro Phe Met Gln Ala
   675 680 685
   Asp Ser Ser Thr Ser Arg Asn Tyr Gly Gly Thr Gly Ile Gly Leu Ser
   690 695 700
   Ile Ser Lys Cys Leu Val Glu Leu Met Arg Gly Gln Ile Asn Phe Ile
   705 710 715 720
   Ser Arg Pro His Ile Gly Ser Thr Phe Trp Phe Thr Ala Val Leu Glu
   725 730 735
   Lys Cys Asp Lys Cys Ser Ala Ile Asn His Met Lys Lys Pro Asn Val
   740 745 750
   Glu His Leu Pro Ser Thr Phe Lys Gly Met Lys Ala Ile Val Val Asp
   755 760 765
   Ala Lys Pro Val Arg Ala Ala Val Thr Arg Tyr His Met Lys Arg Leu
   770 775 780
   Gly Ile Asn Val Asp Val Val Thr Ser Leu Lys Thr Ala Val Val Ala
   785 790 795 800
   Ala Ala Ala Phe Glu Arg Asn Gly Ser Pro Leu Pro Thr Lys Pro Gln
   805 810 815
   Leu Asp Met Ile Leu Val Glu Lys Asp Ser Trp Ile Ser Thr Glu Asp
   820 825 830
   Asn Asp Ser Glu Ile Arg Leu Leu Asn Ser Arg Thr Asn Gly Asn Val
   835 840 845
   His His Lys Ser Pro Lys Leu Ala Leu Phe Ala Thr Asn Ile Thr Asn
   850 855 860
   Ser Glu Phe Asp Arg Ala Lys Ser Ala Gly Phe Ala Asp Thr Val Ile
   865 870 875 880
   Met Lys Pro Leu Arg Ala Ser Met Ile Gly Ala Cys Leu Gln Gln Val
   885 890 895
   Leu Glu Leu Arg Lys Thr Arg Gln Gln His Pro Glu Gly Ser Ser Pro
   900 905 910
   Ala Thr Leu Lys Ser Leu Leu Thr Gly Lys Lys Ile Leu Val Val Asp
   915 920 925
   Asp Asn Ile Val Asn Arg Arg Val Ala Ala Gly Ala Leu Lys Lys Phe
   930 935 940
   Gly Ala Glu Val Val Cys Ala Glu Ser Gly Gln Val Ala Leu Gly Leu
   945 950 955 960
   Leu Gln Ile Pro His Thr Phe Asp Ala Cys Phe Met Asp Ile Gln Met
   965 970 975
   Pro Gln Met Asp Gly Phe Glu Ala Thr Arg Gln Ile Arg Met Met Glu
   980 985 990
   Lys Glu Ala Lys Glu Lys Thr Asn Leu Glu Trp His Leu Pro Ile Leu
   995 1000 1005
   Ala Met Thr Ala Asp Val Ile His Ala Thr Tyr Glu Glu Cys Leu Lys
   1010 1015 1020
   Ser Gly Met Asp Gly Tyr Val Ser Lys Pro Phe Glu Glu Glu Asn Leu
   1025 1030 1035 1040
   Tyr Lys Ser Val Ala Lys Ser Phe Lys Pro Asn Pro Ile Ser Pro Ser
   1045 1050 1055
   Ser
<210> 3
   <211> 3531
   <212> DNA
   <213> Arabidopsis thaliana
<220>
   <221> CDS
   <222> (1).. (3531)
<400> 3
   atg tct ata act tgt gag ctc ttg aat ctt act tca aag aaa gct aag 48
   Met Ser Ile Thr Cys Glu Leu Leu Asn Leu Thr Ser Lys Lys Ala Lys
   1 5 10 15
   aag tcg tcg agc agt gac aag aaa tgg cta aag aag cct ctc ttc ttc 96
   Lys Ser Ser Ser Ser Asp Lys Lys Trp Leu Lys Lys Pro Leu Phe Phe
   20 25 30
   ctg att ttg tgt ggc tct ttg gta att gtt ttg gtt atg ttc tta cgg 144
   Leu Ile Leu Cys Gly Ser Leu Val Ile Val Leu Val Met Phe Leu Arg
   35 40 45
   tta ggt aga agt cag aag gag gag aca gat tct tgt aat gga gaa gag 192
   Leu Gly Arg Ser Gln Lys Glu Glu Thr Asp Ser Cys Asn Gly Glu Glu
   50 55 60
   aaa gtg ttg tat aga cat caa aat gtc aca aga agt gag att cat gat 240
   Lys Val Leu Tyr Arg His Gln Asn Val Thr Arg Ser Glu Ile His Asp
   65 70 75 80
   ttg gtc tct ttg ttc tct gat tca gat cag gta aca tcc ttt gaa tgt 288
   Leu Val Ser Leu Phe Ser Asp Ser Asp Gln Val Thr Ser Phe Glu Cys
   85 90 95
   cat aag gaa tca agc cct gga atg tgg aca aac tat ggt att aca tgt 336
   His Lys Glu Ser Ser Pro Gly Met Trp Thr Asn Tyr Gly Ile Thr Cys
   100 105 110
   tcc ctg agt gtg cgt tct gat aaa caa gag act aga ggg ctt ccc tgg 384
   Ser Leu Ser Val Arg Ser Asp Lys Gln Glu Thr Arg Gly Leu Pro Trp
   115 120 125
   aat ctt ggc tta gga cat tct atc tca tca aca tct tgt atg tgt ggt 432
   Asn Leu Gly Leu Gly His Ser Ile Ser Ser Thr Ser Cys Met Cys Gly
   130 135 140
   aat ctt gaa ccg att tta cag caa cct gaa aac ctt gag gaa gaa aac 480
   Asn Leu Glu Pro Ile Leu Gln Gln Pro Glu Asn Leu Glu Glu Glu Asn
   145 150 155 160
   cat gaa gaa ggg ctg gag cag ggt ttg tca tcg tat tta aga aat gca 528
   His Glu Glu Gly Leu Glu Gln Gly Leu Ser Ser Tyr Leu Arg Asn Ala
   165 170 175
   tgg tgg tgt cta atc ctt ggt gtg tta gtg tgc cat aag att tat gta 576
   Trp Trp Cys Leu Ile Leu Gly Val Leu Val Cys His Lys Ile Tyr Val
   180 185 190
   tct cat tct aaa gca cga ggt gag agg aaa gag aaa gta cat ctg caa 624
   Ser His Ser Lys Ala Arg Gly Glu Arg Lys Glu Lys Val His Leu Gln
   195 200 205
   gag gct tta gct cca aag aag cag caa caa cgt gct cag act tct tct 672
   Glu Ala Leu Ala Pro Lys Lys Gln Gln Gln Arg Ala Gln Thr Ser Ser
   210 215 220
   aga ggg gct gga aga tgg agg aag aat atc ctt ctc ctt ggt att tta 720
   Arg Gly Ala Gly Arg Trp Arg Lys Asn Ile Leu Leu Leu Gly Ile Leu
   225 230 235 240
   gga gga gtt tcc ttc tct gtt tgg tgg ttt tgg gac act aat gag gag 768
   Gly Gly Val Ser Phe Ser Val Trp Trp Phe Trp Asp Thr Asn Glu Glu
   245 250 255
   atc ata atg aaa agg agg gag act ttg gca aac atg tgt gac gaa cga 816
   Ile Ile Met Lys Arg Arg Glu Thr Leu Ala Asn Met Cys Asp Glu Arg
   260 265 270
   gca cgt gtt tta caa gat cag ttc aat gtt agc ttg aac cat gtt cat 864
   Ala Arg Val Leu Gln Asp Gln Phe Asn Val Ser Leu Asn His Val His
   275 280 285
   gcc ttg tct att ctt gta tct aca ttt cat cat ggt aaa atc cca tct 912
   Ala Leu Ser Ile Leu Val Ser Thr Phe His His Gly Lys Ile Pro Ser
   290 295 300
   gcc att gat cag aga aca ttt gaa gaa tat act gag aga aca aac ttt 960
   Ala Ile Asp Gln Arg Thr Phe Glu Glu Tyr Thr Glu Arg Thr Asn Phe
   305 310 315 320
   gag agg cca ctt act agt ggt gta gcg tat gct ttg aaa gtc cca cac 1008
   Glu Arg Pro Leu Thr Ser Gly Val Ala Tyr Ala Leu Lys Val Pro His
   325 330 335
   tca gaa aga gag aaa ttt gaa aag gag cat gga tgg gca ata aag aaa 1056
   Ser Glu Arg Glu Lys Phe Glu Lys Glu His Gly Trp Ala Ile Lys Lys
   340 345 350
   atg gaa act gag gac cag aca gtt gta caa gat tgt gtt cct gaa aac 1104
   Met Glu Thr Glu Asp Gln Thr Val Val Gln Asp Cys Val Pro Glu Asn
   355 360 365
   ttt gat ccc gca ccg att caa gac gaa tac gcg cca gtt ata ttt gct 1152
   Phe Asp Pro Ala Pro Ile Gln Asp Glu Tyr Ala Pro Val Ile Phe Ala
   370 375 380
   caa gaa act gtt tcc cat att gta tcg gtc gac atg atg tct gga gaa 1200
   Gln Glu Thr Val Ser His Ile Val Ser Val Asp Met Met Ser Gly Glu
   385 390 395 400
   gaa gac cgt gaa aac atc tta cgg gca agg gca tca gga aaa gga gtg 1248
   Glu Asp Arg Glu Asn Ile Leu Arg Ala Arg Ala Ser Gly Lys Gly Val
   405 410 415
   tta aca tct cca ttt aag ctt ctt aag tca aat cat ctt ggt gtt gtg 1296
   Leu Thr Ser Pro Phe Lys Leu Leu Lys Ser Asn His Leu Gly Val Val
   420 425 430
   ttg acc ttt gct gtc tat gac acg agc cta ccg cct gat gct aca gaa 1344
   Leu Thr Phe Ala Val Tyr Asp Thr Ser Leu Pro Pro Asp Ala Thr Glu
   435 440 445
   gaa cag cgt gtt gaa gca act att ggg tac ctt ggt gca tca tat gat 1392
   Glu Gln Arg Val Glu Ala Thr Ile Gly Tyr Leu Gly Ala Ser Tyr Asp
   450 455 460
   atg cca tcg ctg gtg gag aaa ctt ctt cac caa ctt gcc agc aaa cag 1440
   Met Pro Ser Leu Val Glu Lys Leu Leu His Gln Leu Ala Ser Lys Gln
   465 470 475 480
   aca att gct gtg gat gtt tac gac aca act aac act tca ggt cta ata 1488
   Thr Ile Ala Val Asp Val Tyr Asp Thr Thr Asn Thr Ser Gly Leu Ile
   485 490 495
   aaa atg tat ggc tca gaa att ggg gat ata agt gag cag cat ata agt 1536
   Lys Met Tyr Gly Ser Glu Ile Gly Asp Ile Ser Glu Gln His Ile Ser
   500 505 510
   agc ctt gat ttt ggt gat cca tca agg aac cat gag atg cat tgc agg 1584
   Ser Leu Asp Phe Gly Asp Pro Ser Arg Asn His Glu Met His Cys Arg
   515 520 525
   ttt aag cat aaa ctt ccc att ccc tgg aca gcg ata aca ccg tcg atc 1632
   Phe Lys His Lys Leu Pro Ile Pro Trp Thr Ala Ile Thr Pro Ser Ile
   530 535 540
   tta gtt ctg gtt att act ttt ctt gtt ggt tat att tta tat gaa gcc 1680
   Leu Val Leu Val Ile Thr Phe Leu Val Gly Tyr Ile Leu Tyr Glu Ala
   545 550 555 560
   atc aac cga att gcg aca gtt gaa gag gat tgt cag aag atg agg gaa 1728
   Ile Asn Arg Ile Ala Thr Val Glu Glu Asp Cys Gln Lys Met Arg Glu
   565 570 575
   ctc aaa gct cgt gct gag gcc gct gac att gca aag tca cag ttc cta 1776
   Leu Lys Ala Arg Ala Glu Ala Ala Asp Ile Ala Lys Ser Gln Phe Leu
   580 585 590
   gca act gtt tct cat gag ata cgg act ccg atg aat gga gtt tta gga 1824
   Ala Thr Val Ser His Glu Ile Arg Thr Pro Met Asn Gly Val Leu Gly
   595 600 605
   atg ctg aaa atg ctg atg gac acc gat ctt gat gcg aag cag atg gac 1872
   Met Leu Lys Met Leu Met Asp Thr Asp Leu Asp Ala Lys Gln Met Asp
   610 615 620
   tat gcg caa act gct cat ggc agt ggg aag gat ctt aca tca cta ata 1920
   Tyr Ala Gln Thr Ala His Gly Ser Gly Lys Asp Leu Thr Ser Leu Ile
   625 630 635 640
   aat gag gtt ctt gat cag gca aag att gaa tcc gga agg ctc gag ctt 1968
   Asn Glu Val Leu Asp Gln Ala Lys Ile Glu Ser Gly Arg Leu Glu Leu
   645 650 655
   gaa aat gtg cct ttt gat atg cgt ttt att ctt gat aat gtt tca tct 2016
   Glu Asn Val Pro Phe Asp Met Arg Phe Ile Leu Asp Asn Val Ser Ser
   660 665 670
   ctc ctc tct ggc aag gca aat gaa aaa gga att gag ttg gcc gtt tat 2064
   Leu Leu Ser Gly Lys Ala Asn Glu Lys Gly Ile Glu Leu Ala Val Tyr
   675 680 685
   gtt tct agt caa gtt cct gat gtt gta gtc ggt gat ccg agt cgg ttc 2112
   Val Ser Ser Gln Val Pro Asp Val Val Val Gly Asp Pro Ser Arg Phe
   690 695 700
   cgg cag atc att aca aac ctg gtt gga aac tca atc aaa ttc aca cag 2160
   Arg Gln Ile Ile Thr Asn Leu Val Gly Asn Ser Ile Lys Phe Thr Gln
   705 710 715 720
   gaa agg gga cac ata ttt atc tca gtg cac ctt gca gat gag gta aag 2208
   Glu Arg Gly His Ile Phe Ile Ser Val His Leu Ala Asp Glu Val Lys
   725 730 735
   gag cct ctt act att gaa gac gca gtg cta aaa cag cga cta gct tta 2256
   Glu Pro Leu Thr Ile Glu Asp Ala Val Leu Lys Gln Arg Leu Ala Leu
   740 745 750
   gga tgc agc gag tcc ggt gag aca gtt agc ggg ttt cct gcg gta aat 2304
   Gly Cys Ser Glu Ser Gly Glu Thr Val Ser Gly Phe Pro Ala Val Asn
   755 760 765
   gca tgg gga agc tgg aag aat ttc aag aca tgt tac agt act gag agt 2352
   Ala Trp Gly Ser Trp Lys Asn Phe Lys Thr Cys Tyr Ser Thr Glu Ser
   770 775 780
   cag aat tct gat caa atc aaa ttg cta gtt aca gtg gag gac act gga 2400
   Gln Asn Ser Asp Gln Ile Lys Leu Leu Val Thr Val Glu Asp Thr Gly
   785 790 795 800
   gtt ggc ata cct gtg gat gca caa ggc cga atc ttc aca cct ttt atg 2448
   Val Gly Ile Pro Val Asp Ala Gln Gly Arg Ile Phe Thr Pro Phe Met
   805 810 815
   caa gcc gac agt tcc aca tcg cgg act tat ggt gga act ggc ata ggt 2496
   Gln Ala Asp Ser Ser Thr Ser Arg Thr Tyr Gly Gly Thr Gly Ile Gly
   820 825 830
   ttg agt ata agc aaa cgt ttg gtt gaa ctc atg caa gga gag atg ggg 2544
   Leu Ser Ile Ser Lys Arg Leu Val Glu Leu Met Gln Gly Glu Met Gly
   835 840 845
   ttt gtg agt gag ccc ggg ata ggc agt act ttt tca ttt act gga gtt 2592
   Phe Val Ser Glu Pro Gly Ile Gly Ser Thr Phe Ser Phe Thr Gly Val
   850 855 860
   ttc ggg aaa gca gaa aca aat acg tcg att act aag ctg gaa cga ttc 2640
   Phe Gly Lys Ala Glu Thr Asn Thr Ser Ile Thr Lys Leu Glu Arg Phe
   865 870 875 880
   gat cta gct att cag gag ttt aca gga ttg aga gca tta gtt att gat 2688
   Asp Leu Ala Ile Gln Glu Phe Thr Gly Leu Arg Ala Leu Val Ile Asp
   885 890 895
   aac aga aac att aga gca gag gtc acc agg tac gaa ctt cgg aga ctg 2736
   Asn Arg Asn Ile Arg Ala Glu Val Thr Arg Tyr Glu Leu Arg Arg Leu
   900 905 910
   gga ata tct gca gac att gtt tca agt ctg aga atg gca tgc act tgt 2784
   Gly Ile Ser Ala Asp Ile Val Ser Ser Leu Arg Met Ala Cys Thr Cys
   915 920 925
   tgt atc agc aaa tta gaa aat ttg gct atg att cta ata gac aaa gac 2832
   Cys Ile Ser Lys Leu Glu Asn Leu Ala Met Ile Leu Ile Asp Lys Asp
   930 935 940
   gcc tgg aac aag gaa gaa ttt tca gta ctt gac gag ttg ttt acc cga 2880
   Ala Trp Asn Lys Glu Glu Phe Ser Val Leu Asp Glu Leu Phe Thr Arg
   945 950 955 960
   agc aaa gta acc ttt aca aga gtc cca aag att ttt ctt ttg gca act 2928
   Ser Lys Val Thr Phe Thr Arg Val Pro Lys Ile Phe Leu Leu Ala Thr
   965 970 975
   tct gca act ctt act gag cgc agt gag atg aag tct act ggt ctc atc 2976
   Ser Ala Thr Leu Thr Glu Arg Ser Glu Met Lys Ser Thr Gly Leu Ile
   980 985 990
   gat gag gtg gtg ata aag cct ctt cgg atg agt gtc tta ata tgt tgc 3024
   Asp Glu Val Val Ile Lys Pro Leu Arg Met Ser Val Leu Ile Cys Cys
   995 1000 1005
   ttg caa gaa acc ctt gtc aat ggc aag aag agg caa ccg aac aga cag 3072
   Leu Gln Glu Thr Leu Val Asn Gly Lys Lys Arg Gln Pro Asn Arg Gln
   1010 1015 1020
   cga aga aat ctt gga cac ttg cta aga gaa aaa cag att ctg gtt gtg 3120
   Arg Arg Asn Leu Gly His Leu Leu Arg Glu Lys Gln Ile Leu Val Val
   1025 1030 1035 1040
   gat gat aat ctt gtg aac aga cga gtt gca gaa ggt gca ctt aag aaa 3168
   Asp Asp Asn Leu Val Asn Arg Arg Val Ala Glu Gly Ala Leu Lys Lys
   1045 1050 1055
   tat gga gct att gtt aca tgc gtt gag agt ggc aaa gct gca ttg gca 3216
   Tyr Gly Ala Ile Val Thr Cys Val Glu Ser Gly Lys Ala Ala Leu Ala
   1060 1065 1070
   atg ctt aag ccg cct cat aac ttc gat gct tgc ttc atg gat ctc cag 3264
   Met Leu Lys Pro Pro His Asn Phe Asp Ala Cys Phe Met Asp Leu Gln
   1075 1080 1085
   atg cct gaa atg gat gga ttt gaa gcg aca agg aga gtc cgt gag ctg 3312
   Met Pro Glu Met Asp Gly Phe Glu Ala Thr Arg Arg Val Arg Glu Leu
   1090 1095 1100
   gag agg gaa atc aat aag aaa ata gct tct gga gaa gtt tca gct gaa 3360
   Glu Arg Glu Ile Asn Lys Lys Ile Ala Ser Gly Glu Val Ser Ala Glu
   1105 1110 1115 1120
   atg ttc tgt aaa ttt agt agt tgg cac gtc ccg ata tta gca atg aca 3408
   Met Phe Cys Lys Phe Ser Ser Trp His Val Pro Ile Leu Ala Met Thr
   1125 1130 1135
   gca gat gtt att cag gct act cat gaa gaa tgc atg aaa tgt gga atg 3456
   Ala Asp Val Ile Gln Ala Thr His Glu Glu Cys Met Lys Cys Gly Met
   1140 1145 1150
   gat ggt tat gta tca aaa ccg ttt gaa gag gaa gtg ctc tac aca gcg 3504
   Asp Gly Tyr Val Ser Lys Pro Phe Glu Glu Glu Val Leu Tyr Thr Ala
   1155 1160 1165
   gta gca aga ttc ttt gaa cct tgt taa 3531
   Val Ala Arg Phe Phe Glu Pro Cys
   1170 1175
<210> 4
   <211> 1176
   <212> PRT
   <213> Arabidopsis thaliana
<400> 4
   Met Ser Ile Thr Cys Glu Leu Leu Asn Leu Thr Ser Lys Lys Ala Lys
   1 5 10 15
   Lys Ser Ser Ser Ser Asp Lys Lys Trp Leu Lys Lys Pro Leu Phe Phe
   20 25 30
   Leu Ile Leu Cys Gly Ser Leu Val Ile Val Leu Val Met Phe Leu Arg
   35 40 45
   Leu Gly Arg Ser Gln Lys Glu Glu Thr Asp Ser Cys Asn Gly Glu Glu
   50 55 60
   Lys Val Leu Tyr Arg His Gln Asn Val Thr Arg Ser Glu Ile His Asp
   65 70 75 80
   Leu Val Ser Leu Phe Ser Asp Ser Asp Gln Val Thr Ser Phe Glu Cys
   85 90 95
   His Lys Glu Ser Ser Pro Gly Met Trp Thr Asn Tyr Gly Ile Thr Cys
   100 105 110
   Ser Leu Ser Val Arg Ser Asp Lys Gln Glu Thr Arg Gly Leu Pro Trp
   115 120 125
   Asn Leu Gly Leu Gly His Ser Ile Ser Ser Thr Ser Cys Met Cys Gly
   130 135 140
   Asn Leu Glu Pro Ile Leu Gln Gln Pro Glu Asn Leu Glu Glu Glu Asn
   145 150 155 160
   His Glu Glu Gly Leu Glu Gln Gly Leu Ser Ser Tyr Leu Arg Asn Ala
   165 170 175
   Trp Trp Cys Leu Ile Leu Gly Val Leu Val Cys His Lys Ile Tyr Val
   180 185 190
   Ser His Ser Lys Ala Arg Gly Glu Arg Lys Glu Lys Val His Leu Gln
   195 200 205
   Glu Ala Leu Ala Pro Lys Lys Gln Gln Gln Arg Ala Gln Thr Ser Ser
   210 215 220
   Arg Gly Ala Gly Arg Trp Arg Lys Asn Ile Leu Leu Leu Gly Ile Leu
   225 230 235 240
   Gly Gly Val Ser Phe Ser Val Trp Trp Phe Trp Asp Thr Asn Glu Glu
   245 250 255
   Ile Ile Met Lys Arg Arg Glu Thr Leu Ala Asn Met Cys Asp Glu Arg
   260 265 270
   Ala Arg Val Leu Gln Asp Gln Phe Asn Val Ser Leu Asn His Val His
   275 280 285
   Ala Leu Ser Ile Leu Val Ser Thr Phe His His Gly Lys Ile Pro Ser
   290 295 300
   Ala Ile Asp Gln Arg Thr Phe Glu Glu Tyr Thr Glu Arg Thr Asn Phe
   305 310 315 320
   Glu Arg Pro Leu Thr Ser Gly Val Ala Tyr Ala Leu Lys Val Pro His
   325 330 335
   Ser Glu Arg Glu Lys Phe Glu Lys Glu His Gly Trp Ala Ile Lys Lys
   340 345 350
   Met Glu Thr Glu Asp Gln Thr Val Val Gln Asp Cys Val Pro Glu Asn
   355 360 365
   Phe Asp Pro Ala Pro Ile Gln Asp Glu Tyr Ala Pro Val Ile Phe Ala
   370 375 380
   Gln Glu Thr Val Ser His Ile Val Ser Val Asp Met Met Ser Gly Glu
   385 390 395 400
   Glu Asp Arg Glu Asn Ile Leu Arg Ala Arg Ala Ser Gly Lys Gly Val
   405 410 415
   Leu Thr Ser Pro Phe Lys Leu Leu Lys Ser Asn His Leu Gly Val Val
   420 425 430
   Leu Thr Phe Ala Val Tyr Asp Thr Ser Leu Pro Pro Asp Ala Thr Glu
   435 440 445
   Glu Gln Arg Val Glu Ala Thr Ile Gly Tyr Leu Gly Ala Ser Tyr Asp
   450 455 460
   Met Pro Ser Leu Val Glu Lys Leu Leu His Gln Leu Ala Ser Lys Gln
   465 470 475 480
   Thr Ile Ala Val Asp Val Tyr Asp Thr Thr Asn Thr Ser Gly Leu Ile
   485 490 495
   Lys Met Tyr Gly Ser Glu Ile Gly Asp Ile Ser Glu Gln His Ile Ser
   500 505 510
   Ser Leu Asp Phe Gly Asp Pro Ser Arg Asn His Glu Met His Cys Arg
   515 520 525
   Phe Lys His Lys Leu Pro Ile Pro Trp Thr Ala Ile Thr Pro Ser Ile
   530 535 540
   Leu Val Leu Val Ile Thr Phe Leu Val Gly Tyr Ile Leu Tyr Glu Ala
   545 550 555 560
   Ile Asn Arg Ile Ala Thr Val Glu Glu Asp Cys Gln Lys Met Arg Glu
   565 570 575
   Leu Lys Ala Arg Ala Glu Ala Ala Asp Ile Ala Lys Ser Gln Phe Leu
   580 585 590
   Ala Thr Val Ser His Glu Ile Arg Thr Pro Met Asn Gly Val Leu Gly
   595 600 605
   Met Leu Lys Met Leu Met Asp Thr Asp Leu Asp Ala Lys Gln Met Asp
   610 615 620
   Tyr Ala Gln Thr Ala His Gly Ser Gly Lys Asp Leu Thr Ser Leu Ile
   625 630 635 640
   Asn Glu Val Leu Asp Gln Ala Lys Ile Glu Ser Gly Arg Leu Glu Leu
   645 650 655
   Glu Asn Val Pro Phe Asp Met Arg Phe Ile Leu Asp Asn Val Ser Ser
   660 665 670
   Leu Leu Ser Gly Lys Ala Asn Glu Lys Gly Ile Glu Leu Ala Val Tyr
   675 680 685
   Val Ser Ser Gln Val Pro Asp Val Val Val Gly Asp Pro Ser Arg Phe
   690 695 700
   Arg Gln Ile Ile Thr Asn Leu Val Gly Asn Ser Ile Lys Phe Thr Gln
   705 710 715 720
   Glu Arg Gly His Ile Phe Ile Ser Val His Leu Ala Asp Glu Val Lys
   725 730 735
   Glu Pro Leu Thr Ile Glu Asp Ala Val Leu Lys Gln Arg Leu Ala Leu
   740 745 750
   Gly Cys Ser Glu Ser Gly Glu Thr Val Ser Gly Phe Pro Ala Val Asn
   755 760 765
   Ala Trp Gly Ser Trp Lys Asn Phe Lys Thr Cys Tyr Ser Thr Glu Ser
   770 775 780
   Gln Asn Ser Asp Gln Ile Lys Leu Leu Val Thr Val Glu Asp Thr Gly
   785 790 795 800
   Val Gly Ile Pro Val Asp Ala Gln Gly Arg Ile Phe Thr Pro Phe Met
   805 810 815
   Gln Ala Asp Ser Ser Thr Ser Arg Thr Tyr Gly Gly Thr Gly Ile Gly
   820 825 830
   Leu Ser Ile Ser Lys Arg Leu Val Glu Leu Met Gln Gly Glu Met Gly
   835 840 845
   Phe Val Ser Glu Pro Gly Ile Gly Ser Thr Phe Ser Phe Thr Gly Val
   850 855 860
   Phe Gly Lys Ala Glu Thr Asn Thr Ser Ile Thr Lys Leu Glu Arg Phe
   865 870 875 880
   Asp Leu Ala Ile Gln Glu Phe Thr Gly Leu Arg Ala Leu Val Ile Asp
   885 890 895
   Asn Arg Asn Ile Arg Ala Glu Val Thr Arg Tyr Glu Leu Arg Arg Leu
   900 905 910
   Gly Ile Ser Ala Asp Ile Val Ser Ser Leu Arg Met Ala Cys Thr Cys
   915 920 925
   Cys Ile Ser Lys Leu Glu Asn Leu Ala Met Ile Leu Ile Asp Lys Asp
   930 935 940
   Ala Trp Asn Lys Glu Glu Phe Ser Val Leu Asp Glu Leu Phe Thr Arg
   945 950 955 960
   Ser Lys Val Thr Phe Thr Arg Val Pro Lys Ile Phe Leu Leu Ala Thr
   965 970 975
   Ser Ala Thr Leu Thr Glu Arg Ser Glu Met Lys Ser Thr Gly Leu Ile
   980 985 990
   Asp Glu Val Val Ile Lys Pro Leu Arg Met Ser Val Leu Ile Cys Cys
   995 1000 1005
   Leu Gln Glu Thr Leu Val Asn Gly Lys Lys Arg Gln Pro Asn Arg Gln
   1010 1015 1020
   Arg Arg Asn Leu Gly His Leu Leu Arg Glu Lys Gln Ile Leu Val Val
   1025 1030 1035 1040
   Asp Asp Asn Leu Val Asn Arg Arg Val Ala Glu Gly Ala Leu Lys Lys
   1045 1050 1055
   Tyr Gly Ala Ile Val Thr Cys Val Glu Ser Gly Lys Ala Ala Leu Ala
   1060 1065 1070
   Met Leu Lys Pro Pro His Asn Phe Asp Ala Cys Phe Met Asp Leu Gln
   1075 1080 1085
   Met Pro Glu Met Asp Gly Phe Glu Ala Thr Arg Arg Val Arg Glu Leu
   1090 1095 1100
   Glu Arg Glu Ile Asn Lys Lys Ile Ala Ser Gly Glu Val Ser Ala Glu
   1105 1110 1115 1120
   Met Phe Cys Lys Phe Ser Ser Trp His Val Pro Ile Leu Ala Met Thr
   1125 1130 1135
   Ala Asp Val Ile Gln Ala Thr His Glu Glu Cys Met Lys Cys Gly Met
   1140 1145 1150
   Asp Gly Tyr Val Ser Lys Pro Phe Glu Glu Glu Val Leu Tyr Thr Ala
   1155 1160 1165
   Val Ala Arg Phe Phe Glu Pro Cys
   1170 1175
<210> 5
   <211> 3111
   <212> DNA
   <213> Arabidopsis thaliana
<220>
   <221> CDS
   <222> (1) .. (3111)
<400> 5
   atg agt ctg ttc cat gtg cta ggg ttt ggt gtc aag att ggg cat ctc 48
   Met Ser Leu Phe His Val Leu Gly Phe Gly Val Lys Ile Gly His Leu
   1 5 10 15
   ttc tgg atg cta tgc tgc tgg ttt gtt tct tgg ttc gtt gat aat ggg 96
   Phe Trp Met Leu Cys Cys Trp Phe Val Ser Trp Phe Val Asp Asn Gly
   20 25 30
   atc gag gac aag tct ggt ctt tta gtt ggc tct gtc ggt gat ctt gag 144
   Ile Glu Asp Lys Ser Gly Leu Leu Val Gly Ser Val Gly Asp Leu Glu
   35 40 45
   aag act aag atg act acg ttg aag aag aag aac aag atg tgg ttc tgg 192
   Lys Thr Lys Met Thr Thr Leu Lys Lys Lys Asn Lys Met Trp Phe Trp
   50 55 60
   aat aag atc tct agc agc gga ctc aag atc ccg agt ttc tct tat cag 240
   Asn Lys Ile Ser Ser Ser Gly Leu Lys Ile Pro Ser Phe Ser Tyr Gln
   65 70 75 80
   ttt ctt ggc tct gtt aaa ttc aac aag gcg tgg tgg agg aag ctt gtg 288
   Phe Leu Gly Ser Val Lys Phe Asn Lys Ala Trp Trp Arg Lys Leu Val
   85 90 95
   gtg gtt tgg gtt gtc ttc tgg gtc ttg gtc tct att tgg acg ttt tgg 336
   Val Val Trp Val Val Phe Trp Val Leu Val Ser Ile Trp Thr Phe Trp
   100 105 110
   tac ttt agc tcg caa gct atg gag aag agg aaa gag acg cta gct agt 384
   Tyr Phe Ser Ser Gln Ala Met Glu Lys Arg Lys Glu Thr Leu Ala Ser
   115 120 125
   atg tgt gat gag aga gct cgt atg ctg cag gat cag ttc aac gtt agc 432
   Met Cys Asp Glu Arg Ala Arg Met Leu Gln Asp Gln Phe Asn Val Ser
   130 135 140
   atg aat cat gtt caa gcc atg tct atc ttg atc tca acc ttc cac cat 480
   Met Asn His Val Gln Ala Met Ser Ile Leu Ile Ser Thr Phe His His
   145 150 155 160
   ggc aag att cct tct gct atc gat cag aga aca ttc tca gag tac act 528
   Gly Lys Ile Pro Ser Ala Ile Asp Gln Arg Thr Phe Ser Glu Tyr Thr
   165 170 175
   gat aga act tcc ttt gag agg cct ctt act agc ggg gta gct tat gct 576
   Asp Arg Thr Ser Phe Glu Arg Pro Leu Thr Ser Gly Val Ala Tyr Ala
   180 185 190
   atg agg gtg ctc cat tca gag agg gaa gag ttc gag agg caa caa ggt 624
   Met Arg Val Leu His Ser Glu Arg Glu Glu Phe Glu Arg Gln Gln Gly
   195 200 205
   tgg act att agg aag atg tat tct ctt gaa caa aac cca gtt cac aag 672
   Trp Thr Ile Arg Lys Met Tyr Ser Leu Glu Gln Asn Pro Val His Lys
   210 215 220
   gat gac tat gac ctg gaa gct ttg gaa cca tcc cct gtc caa gaa gag 720
   Asp Asp Tyr Asp Leu Glu Ala Leu Glu Pro Ser Pro Val Gln Glu Glu
   225 230 235 240
   tac gct cca gtc atc ttt gct cag gac act gtt tct cac gtt gtt tct 768
   Tyr Ala Pro Val Ile Phe Ala Gln Asp Thr Val Ser His Val Val Ser
   245 250 255
   ctc gat atg ctg tct ggg aaa gaa gat cgt gaa aac gtt ttg cgg gcc 816
   Leu Asp Met Leu Ser Gly Lys Glu Asp Arg Glu Asn Val Leu Arg Ala
   260 265 270
   agg agt tca ggt aaa ggg gtt ttg aca gct cct ttc cca ttg ata aag 864
   Arg Ser Ser Gly Lys Gly Val Leu Thr Ala Pro Phe Pro Leu Ile Lys
   275 280 285
   aca aat aga ctt ggg gtg atc ctg aca ttt gca gtg tac aag aga gat 912
   Thr Asn Arg Leu Gly Val Ile Leu Thr Phe Ala Val Tyr Lys Arg Asp
   290 295 300
   ctc ccc tcc aat gca acg cca aaa gag aga att gag gct act aac ggg 960
   Leu Pro Ser Asn Ala Thr Pro Lys Glu Arg Ile Glu Ala Thr Asn Gly
   305 310 315 320
   tat ctc ggg gga gtg ttt gac att gag tcc ctg gta gaa aac ttg ctt 1008
   Tyr Leu Gly Gly Val Phe Asp Ile Glu Ser Leu Val Glu Asn Leu Leu
   325 330 335
   caa cag ctg gct agc aag caa acg att ctt gtc aat gtg tac gat atc 1056
   Gln Gln Leu Ala Ser Lys Gln Thr Ile Leu Val Asn Val Tyr Asp Ile
   340 345 350
   acc aat cac tct caa ccg att agc atg tat ggt aca aat gtg tcg gct 1104
   Thr Asn His Ser Gln Pro Ile Ser Met Tyr Gly Thr Asn Val Ser Ala
   355 360 365
   gat ggg ttg gaa cgt gtt agt cca cta atc ttt ggc gat cca ttg aga 1152
   Asp Gly Leu Glu Arg Val Ser Pro Leu Ile Phe Gly Asp Pro Leu Arg
   370 375 380
   aag cat gag atg cgt tgc aga ttt aag cag aaa cca cca tgg cca gtg 1200
   Lys His Glu Met Arg Cys Arg Phe Lys Gln Lys Pro Pro Trp Pro Val
   385 390 395 400
   cta tca atg gtg aca tca ttc ggt atc ctt gtg att gcg tta ctt gtt 1248
   Leu Ser Met Val Thr Ser Phe Gly Ile Leu Val Ile Ala Leu Leu Val
   405 410 415
   gca cat ata atc cac gca acc gtt agt cga ata cac aaa gtt gaa gaa 1296
   Ala His Ile Ile His Ala Thr Val Ser Arg Ile His Lys Val Glu Glu
   420 425 430
   gat tgt gat aaa atg aag cag ctc aag aaa aag gct gaa gca gca gat 1344
   Asp Cys Asp Lys Met Lys Gln Leu Lys Lys Lys Ala Glu Ala Ala Asp
   435 440 445
   gtt gca aag tca cag ttc ctt gcc act gtt tca cat gaa atc aga act 1392
   Val Ala Lys Ser Gln Phe Leu Ala Thr Val Ser His Glu Ile Arg Thr
   450 455 460
   cca atg aat ggt gtt cta gga atg ttg cat atg ctt atg gac aca gag 1440
   Pro Met Asn Gly Val Leu Gly Met Leu His Met Leu Met Asp Thr Glu
   465 470 475 480
   tta gat gtt acg caa cag gat tat gtt agg acc gca cag gca agt gga 1488
   Leu Asp Val Thr Gln Gln Asp Tyr Val Arg Thr Ala Gln Ala Ser Gly
   485 490 495
   aaa gct tta gtc tcg cta ata aat gag gtt ttg gac caa gca aag att 1536
   Lys Ala Leu Val Ser Leu Ile Asn Glu Val Leu Asp Gln Ala Lys Ile
   500 505 510
   gaa tct gga aag ctt gaa ctt gag gag gtg cgg ttt gat ttg aga gga 1584
   Glu Ser Gly Lys Leu Glu Leu Glu Glu Val Arg Phe Asp Leu Arg Gly
   515 520 525
   ata tta gat gat gtc ctg tca ctc ttc tct agc aag tcc caa caa aag 1632
   Ile Leu Asp Asp Val Leu Ser Leu Phe Ser Ser Lys Ser Gln Gln Lys
   530 535 540
   ggg gtg gag ttg gca gta tac ata tct gat cgt gtt cca gat atg tta 1680
   Gly Val Glu Leu Ala Val Tyr Ile Ser Asp Arg Val Pro Asp Met Leu
   545 550 555 560
   att ggt gat cct ggg agg ttt cga caa ata ctc aca aat ctt atg ggt 1728
   Ile Gly Asp Pro Gly Arg Phe Arg Gln Ile Leu Thr Asn Leu Met Gly
   565 570 575
   aat tcc att aag ttc act gag aaa gga cac atc ttt gta act gtt cat 1776
   Asn Ser Ile Lys Phe Thr Glu Lys Gly His Ile Phe Val Thr Val His
   580 585 590
   ttg gtg gat gag cta ttt gaa tct atc gat gga gag aca gca tca tct 1824
   Leu Val Asp Glu Leu Phe Glu Ser Ile Asp Gly Glu Thr Ala Ser Ser
   595 600 605
   ccg gaa agt aca ctg agt ggg ctt cca gtt gca gac cgg cag agg agc 1872
   Pro Glu Ser Thr Leu Ser Gly Leu Pro Val Ala Asp Arg Gln Arg Ser
   610 615 620
   tgg gaa aac ttt aaa gct ttc agc tcc aac ggg cat cgg agc ttt gaa 1920
   Trp Glu Asn Phe Lys Ala Phe Ser Ser Asn Gly His Arg Ser Phe Glu
   625 630 635 640
   cca tct ccc cct gat ata aac cta atc gtc tca gtt gag gat act ggc 1968
   Pro Ser Pro Pro Asp Ile Asn Leu Ile Val Ser Val Glu Asp Thr Gly
   645 650 655
   gta ggg atc cct gta gaa gcg cag tcc cgt att ttt acg cct ttc atg 2016
   Val Gly Ile Pro Val Glu Ala Gln Ser Arg Ile Phe Thr Pro Phe Met
   660 665 670
   caa gtc gga cca tcc ata tcc agg acg cat gga ggc aca gga att gga 2064
   Gln Val Gly Pro Ser Ile Ser Arg Thr His Gly Gly Thr Gly Ile Gly
   675 680 685
   ctt agc ata agc aaa tgt cta gtt gga ctg atg aag gga gaa att gga 2112
   Leu Ser Ile Ser Lys Cys Leu Val Gly Leu Met Lys Gly Glu Ile Gly
   690 695 700
   ttc tcg agt act ccc aag gtt ggg tcc aca ttc aca ttt act gct gta 2160
   Phe Ser Ser Thr Pro Lys Val Gly Ser Thr Phe Thr Phe Thr Ala Val
   705 710 715 720
   ttt tcc aat ggg atg caa cca gct gaa aga aag aat gac aac aac cag 2208
   Phe Ser Asn Gly Met Gln Pro Ala Glu Arg Lys Asn Asp Asn Asn Gln
   725 730 735
   ccc ata ttc tcg gaa ttc cgg ggc atg aaa gct gtg gtt gtg gac cat 2256
   Pro Ile Phe Ser Glu Phe Arg Gly Met Lys Ala Val Val Val Asp His
   740 745 750
   agg cct gca agg gca aaa gtc tcg tgg tac cat ttt cag cgt ctt gga 2304
   Arg Pro Ala Arg Ala Lys Val Ser Trp Tyr His Phe Gln Arg Leu Gly
   755 760 765
   att cga gtc gaa gta gtt cca cgt gtt gaa cag gct cta cat tat ctg 2352
   Ile Arg Val Glu Val Val Pro Arg Val Glu Gln Ala Leu His Tyr Leu
   770 775 780
   aag att ggt act acc act gtg aat atg ata ctc ata gag caa gaa ata 2400
   Lys Ile Gly Thr Thr Thr Val Asn Met Ile Leu Ile Glu Gln Glu Ile
   785 790 795 800
   tgg aat agg gaa gca gat gat ttc att aaa aag cta cag aaa gac cct 2448
   Trp Asn Arg Glu Ala Asp Asp Phe Ile Lys Lys Leu Gln Lys Asp Pro
   805 810 815
   ctt ttc ctt tct cct aag ttg att ttg tta gca aac tca gta gaa tcg 2496
   Leu Phe Leu Ser Pro Lys Leu Ile Leu Leu Ala Asn Ser Val Glu Ser
   820 825 830
   tca ata tca gag gct tta tgc acc ggt ata gat cct cca ata gtg ata 2544
   Ser Ile Ser Glu Ala Leu Cys Thr Gly Ile Asp Pro Pro Ile Val Ile
   835 840 845
   gtg aaa cca ttg agg gcg agt atg cta gca gca act ttg cag agg gga 2592
   Val Lys Pro Leu Arg Ala Ser Met Leu Ala Ala Thr Leu Gln Arg Gly
   850 855 860
   ttg ggt att gga atc aga gaa cca cct caa cac aag gga cct cct gct 2640
   Leu Gly Ile Gly Ile Arg Glu Pro Pro Gln His Lys Gly Pro Pro Ala
   865 870 875 880
   ttg att ctc agg aat ctt ctc ctt ggt aga aaa att tta atc gtg gat 2688
   Leu Ile Leu Arg Asn Leu Leu Leu Gly Arg Lys Ile Leu Ile Val Asp
   885 890 895
   gat aac aac gta aac ctc aga gtg gca gcg gga gct ctg aaa aag tac 2736
   Asp Asn Asn Val Asn Leu Arg Val Ala Ala Gly Ala Leu Lys Lys Tyr
   900 905 910
   gga gct gat gtg gtc tgc gct gag agt ggg ata aag gca atc tca ttg 2784
   Gly Ala Asp Val Val Cys Ala Glu Ser Gly Ile Lys Ala Ile Ser Leu
   915 920 925
   ctt aag cca cct cac gag ttt gat gct tgc ttc atg gac att cag atg 2832
   Leu Lys Pro Pro His Glu Phe Asp Ala Cys Phe Met Asp Ile Gln Met
   930 935 940
   cca gaa atg gat gga ttt gaa gct aca agg aga ata cga gat atg gaa 2880
   Pro Glu Met Asp Gly Phe Glu Ala Thr Arg Arg Ile Arg Asp Met Glu
   945 950 955 960
   gag gag atg aac aag aga ata aag aat ggg gag gct ttg ata gta gag 2928
   Glu Glu Met Asn Lys Arg Ile Lys Asn Gly Glu Ala Leu Ile Val Glu
   965 970 975
   aac ggt aac aaa aca agc tgg cat ctt ccg gta tta gca atg acg gca 2976
   Asn Gly Asn Lys Thr Ser Trp His Leu Pro Val Leu Ala Met Thr Ala
   980 985 990
   gat gtg atc caa gca acg cat gag gaa tgt ctg aag tgt gga atg gat 3024
   Asp Val Ile Gln Ala Thr His Glu Glu Cys Leu Lys Cys Gly Met Asp
   995 1000 1005
   ggg tat gta tca aaa cca ttt gaa gca gag cag ctg tac agg gaa gtt 3072
   Gly Tyr Val Ser Lys Pro Phe Glu Ala Glu Gln Leu Tyr Arg Glu Val
   1010 1015 1020
   tct cgc ttt ttc aat tcg cct tca gat aca gaa tca taa 3111
   Ser Arg Phe Phe Asn Ser Pro Ser Asp Thr Glu Ser
   1025 1030 1035
<210> 6
   <211> 1036
   <212> PRT
   <213> Arabidopsis thaliana
<400> 6
   Met Ser Leu Phe His Val Leu Gly Phe Gly Val Lys Ile Gly His Leu
   1 5 10 15
   Phe Trp Met Leu Cys Cys Trp Phe Val Ser Trp Phe Val Asp Asn Gly
   20 25 30
   Ile Glu Asp Lys Ser Gly Leu Leu Val Gly Ser Val Gly Asp Leu Glu
   35 40 45
   Lys Thr Lys Met Thr Thr Leu Lys Lys Lys Asn Lys Met Trp Phe Trp
   50 55 60
   Asn Lys Ile Ser Ser Ser Gly Leu Lys Ile Pro Ser Phe Ser Tyr Gln
   65 70 75 80
   Phe Leu Gly Ser Val Lys Phe Asn Lys Ala Trp Trp Arg Lys Leu Val
   85 90 95
   Val Val Trp Val Val Phe Trp Val Leu Val Ser Ile Trp Thr Phe Trp
   100 105 110
   Tyr Phe Ser Ser Gln Ala Met Glu Lys Arg Lys Glu Thr Leu Ala Ser
   115 120 125
   Met Cys Asp Glu Arg Ala Arg Met Leu Gln Asp Gln Phe Asn Val Ser
   130 135 140
   Met Asn His Val Gln Ala Met Ser Ile Leu Ile Ser Thr Phe His His
   145 150 155 160
   Gly Lys Ile Pro Ser Ala Ile Asp Gln Arg Thr Phe Ser Glu Tyr Thr
   165 170 175
   Asp Arg Thr Ser Phe Glu Arg Pro Leu Thr Ser Gly Val Ala Tyr Ala
   180 185 190
   Met Arg Val Leu His Ser Glu Arg Glu Glu Phe Glu Arg Gln Gln Gly
   195 200 205
   Trp Thr Ile Arg Lys Met Tyr Ser Leu Glu Gln Asn Pro Val His Lys
   210 215 220
   Asp Asp Tyr Asp Leu Glu Ala Leu Glu Pro Ser Pro Val Gln Glu Glu
   225 230 235 240
   Tyr Ala Pro Val Ile Phe Ala Gln Asp Thr Val Ser His Val Val Ser
   245 250 255
   Leu Asp Met Leu Ser Gly Lys Glu Asp Arg Glu Asn Val Leu Arg Ala
   260 265 270
   Arg Ser Ser Gly Lys Gly Val Leu Thr Ala Pro Phe Pro Leu Ile Lys
   275 280 285
   Thr Asn Arg Leu Gly Val Ile Leu Thr Phe Ala Val Tyr Lys Arg Asp
   290 295 300
   Leu Pro Ser Asn Ala Thr Pro Lys Glu Arg Ile Glu Ala Thr Asn Gly
   305 310 315 320
   Tyr Leu Gly Gly Val Phe Asp Ile Glu Ser Leu Val Glu Asn Leu Leu
   325 330 335
   Gln Gln Leu Ala Ser Lys Gln Thr Ile Leu Val Asn Val Tyr Asp Ile
   340 345 350
   Thr Asn His Ser Gln Pro Ile Ser Met Tyr Gly Thr Asn Val Ser Ala
   355 360 365
   Asp Gly Leu Glu Arg Val Ser Pro Leu Ile Phe Gly Asp Pro Leu Arg
   370 375 380
   Lys His Glu Met Arg Cys Arg Phe Lys Gln Lys Pro Pro Trp Pro Val
   385 390 395 400
   Leu Ser Met Val Thr Ser Phe Gly Ile Leu Val Ile Ala Leu Leu Val
   405 410 415
   Ala His Ile Ile His Ala Thr Val Ser Arg Ile His Lys Val Glu Glu
   420 425 430
   Asp Cys Asp Lys Met Lys Gln Leu Lys Lys Lys Ala Glu Ala Ala Asp
   435 440 445
   Val Ala Lys Ser Gln Phe Leu Ala Thr Val Ser His Glu Ile Arg Thr
   450 455 460
   Pro Met Asn Gly Val Leu Gly Met Leu His Met Leu Met Asp Thr Glu
   465 470 475 480
   Leu Asp Val Thr Gln Gln Asp Tyr Val Arg Thr Ala Gln Ala Ser Gly
   485 490 495
   Lys Ala Leu Val Ser Leu Ile Asn Glu Val Leu Asp Gln Ala Lys Ile
   500 505 510
   Glu Ser Gly Lys Leu Glu Leu Glu Glu Val Arg Phe Asp Leu Arg Gly
   515 520 525
   Ile Leu Asp Asp Val Leu Ser Leu Phe Ser Ser Lys Ser Gln Gln Lys
   530 535 540
   Gly Val Glu Leu Ala Val Tyr Ile Ser Asp Arg Val Pro Asp Met Leu
   545 550 555 560
   Ile Gly Asp Pro Gly Arg Phe Arg Gln Ile Leu Thr Asn Leu Met Gly
   565 570 575
   Asn Ser Ile Lys Phe Thr Glu Lys Gly His Ile Phe Val Thr Val His
   580 585 590
   Leu Val Asp Glu Leu Phe Glu Ser Ile Asp Gly Glu Thr Ala Ser Ser
   595 600 605
   Pro Glu Ser Thr Leu Ser Gly Leu Pro Val Ala Asp Arg Gln Arg Ser
   610 615 620
   Trp Glu Asn Phe Lys Ala Phe Ser Ser Asn Gly His Arg Ser Phe Glu
   625 630 635 640
   Pro Ser Pro Pro Asp Ile Asn Leu Ile Val Ser Val Glu Asp Thr Gly
   645 650 655
   Val Gly Ile Pro Val Glu Ala Gln Ser Arg Ile Phe Thr Pro Phe Met
   660 665 670
   Gln Val Gly Pro Ser Ile Ser Arg Thr His Gly Gly Thr Gly Ile Gly
   675 680 685
   Leu Ser Ile Ser Lys Cys Leu Val Gly Leu Met Lys Gly Glu Ile Gly
   690 695 700
   Phe Ser Ser Thr Pro Lys Val Gly Ser Thr Phe Thr Phe Thr Ala Val
   705 710 715 720
   Phe Ser Asn Gly Met Gln Pro Ala Glu Arg Lys Asn Asp Asn Asn Gln
   725 730 735
   Pro Ile Phe Ser Glu Phe Arg Gly Met Lys Ala Val Val Val Asp His
   740 745 750
   Arg Pro Ala Arg Ala Lys Val Ser Trp Tyr His Phe Gln Arg Leu Gly
   755 760 765
   Ile Arg Val Glu Val Val Pro Arg Val Glu Gln Ala Leu His Tyr Leu
   770 775 780
   Lys Ile Gly Thr Thr Thr Val Asn Met Ile Leu Ile Glu Gln Glu Ile
   785 790 795 800
   Trp Asn Arg Glu Ala Asp Asp Phe Ile Lys Lys Leu Gln Lys Asp Pro
   805 810 815
   Leu Phe Leu Ser Pro Lys Leu Ile Leu Leu Ala Asn Ser Val Glu Ser
   820 825 830
   Ser Ile Ser Glu Ala Leu Cys Thr Gly Ile Asp Pro Pro Ile Val Ile
   835 840 845
   Val Lys Pro Leu Arg Ala Ser Met Leu Ala Ala Thr Leu Gln Arg Gly
   850 855 860
   Leu Gly Ile Gly Ile Arg Glu Pro Pro Gln His Lys Gly Pro Pro Ala
   865 870 875 880
   Leu Ile Leu Arg Asn Leu Leu Leu Gly Arg Lys Ile Leu Ile Val Asp
   885 890 895
   Asp Asn Asn Val Asn Leu Arg Val Ala Ala Gly Ala Leu Lys Lys Tyr
   900 905 910
   Gly Ala Asp Val Val Cys Ala Glu Ser Gly Ile Lys Ala Ile Ser Leu
   915 920 925
   Leu Lys Pro Pro His Glu Phe Asp Ala Cys Phe Met Asp Ile Gln Met
   930 935 940
   Pro Glu Met Asp Gly Phe Glu Ala Thr Arg Arg Ile Arg Asp Met Glu
   945 950 955 960
   Glu Glu Met Asn Lys Arg Ile Lys Asn Gly Glu Ala Leu Ile Val Glu
   965 970 975
   Asn Gly Asn Lys Thr Ser Trp His Leu Pro Val Leu Ala Met Thr Ala
   980 985 990
   Asp Val Ile Gln Ala Thr His Glu Glu Cys Leu Lys Cys Gly Met Asp
   995 1000 1005
   Gly Tyr Val Ser Lys Pro Phe Glu Ala Glu Gln Leu Tyr Arg Glu Val
   1010 1015 1020
   Ser Arg Phe Phe Asn Ser Pro Ser Asp Thr Glu Ser
   1025 1030 1035
<210> 7
   <211> 1074
   <212> DNA
   <213> Arabidopsis thaliana
<220>
   <221> CDS
   <222> (1).. (1074)
<400> 7
   atg aca gaa ctc aac ttc cac ctc ctc cca ata atc tcc gat cgc ttc 48
   Met Thr Glu Leu Asn Phe His Leu Leu Pro Ile Ile Ser Asp Arg Phe
   1 5 10 15
   acg acg acg acg aca aca tca ccg tcg ttc tcg tca cat tct tct tct 96
   Thr Thr Thr Thr Thr Thr Ser Pro Ser Phe Ser Ser His Ser Ser Ser
   20 25 30
   tct tct tct ctt ctc tct ttc acc aaa cga aga cga aaa cac caa cct 144
   Ser Ser Ser Leu Leu Ser Phe Thr Lys Arg Arg Arg Lys His Gln Pro
   35 40 45
   tta gta tca tcc ata cgc atg gaa cag tca cgg tca cgg aat cgg aaa 192
   Leu Val Ser Ser Ile Arg Met Glu Gln Ser Arg Ser Arg Asn Arg Lys
   50 55 60
   gac aaa gtc gtc gtc att tta gga gca acc ggc gcc gga aaa tca aga 240
   Asp Lys Val Val Val Ile Leu Gly Ala Thr Gly Ala Gly Lys Ser Arg
   65 70 75 80
   ctt tcc gtc gat ctc gct act cgt ttc cct tca gag atc ata aac tcc 288
   Leu Ser Val Asp Leu Ala Thr Arg Phe Pro Ser Glu Ile Ile Asn Ser
   85 90 95
   gat aaa atc caa gtc tac gaa gga tta gag atc aca acg aat cag att 336
   Asp Lys Ile Gln Val Tyr Glu Gly Leu Glu Ile Thr Thr Asn Gln Ile
   100 105 110
   acg tta caa gac cgt cgc ggc gtt cct cac cat ctc ctc ggc gtc atc 384
   Thr Leu Gln Asp Arg Arg Gly Val Pro His His Leu Leu Gly Val Ile
   115 120 125
   aac ccc gaa cac ggc gaa cta acc gcc gga gag ttt cgc tcc gcc gct 432
   Asn Pro Glu His Gly Glu Leu Thr Ala Gly Glu Phe Arg Ser Ala Ala
   130 135 140
   tca aac gtc gtc aaa gag ata act tct cgt caa aag gtt ccg att atc 480
   Ser Asn Val Val Lys Glu Ile Thr Ser Arg Gln Lys Val Pro Ile Ile
   145 150 155 160
   gcc ggt gga tct aac tct ttc gtc cac gca ctc tta gct caa cga ttc 528
   Ala Gly Gly Ser Asn Ser Phe Val His Ala Leu Leu Ala Gln Arg Phe
   165 170 175
   gac cca aag ttc gat cct ttt tca tcc ggg tcg tgt tta atc agc tcc 576
   Asp Pro Lys Phe Asp Pro Phe Ser Ser Gly Ser Cys Leu Ile Ser Ser
   180 185 190
   gat ttg cgt tac gag tgt tgt ttc atc tgg gtc gat gta tcg gag act 624
   Asp Leu Arg Tyr Glu Cys Cys Phe Ile Trp Val Asp Val Ser Glu Thr
   195 200 205
   gtt ctc tac gag tat ctt ctc aga aga gtc gac gaa atg atg gat tca 672
   Val Leu Tyr Glu Tyr Leu Leu Arg Arg Val Asp Glu Met Met Asp Ser
   210 215 220
   ggt atg ttc gaa gag ctg tct aga ttc tac gac ccg gtt aaa tcc ggt 720
   Gly Met Phe Glu Glu Leu Ser Arg Phe Tyr Asp Pro Val Lys Ser Gly
   225 230 235 240
   tta gaa acc cgg ttt ggg att agg aaa gct ata ggt gta ccg gag ttt 768
   Leu Glu Thr Arg Phe Gly Ile Arg Lys Ala Ile Gly Val Pro Glu Phe
   245 250 255
   gac ggt tac ttc aaa gag tat cca ccg gag aag aag atg ata aag tgg 816
   Asp Gly Tyr Phe Lys Glu Tyr Pro Pro Glu Lys Lys Met Ile Lys Trp
   260 265 270
   gac gct tta aga aaa gcg gcg tac gat aag gcg gtt gat gat atc aaa 864
   Asp Ala Leu Arg Lys Ala Ala Tyr Asp Lys Ala Val Asp Asp Ile Lys
   275 280 285
   agg aac acg tgg acg tta gcg aag aga caa gtg aag aag att gag atg 912
   Arg Asn Thr Trp Thr Leu Ala Lys Arg Gln Val Lys Lys Ile Glu Met
   290 295 300
   cta aaa gac gct ggt tgg gaa ata gaa aga gtt gat gca acg gcg tcg 960
   Leu Lys Asp Ala Gly Trp Glu Ile Glu Arg Val Asp Ala Thr Ala Ser
   305 310 315 320
   ttt aaa gca gtg atg atg aag agt tcg tcg gag aag aag tgg aga gag 1008
   Phe Lys Ala Val Met Met Lys Ser Ser Ser Glu Lys Lys Trp Arg Glu
   325 330 335
   aat tgg gaa gag caa gtg ttg gag cca agc gta aag att gtg aag cgg 1056
   Asn Trp Glu Glu Gln Val Leu Glu Pro Ser Val Lys Ile Val Lys Arg
   340 345 350
   cat ttg gtg caa aat tag 1074
   His Leu Val Gln Asn
   355
<210> 8
   <211> 357
   <212> PRT
   <213> Arabidopsis thaliana
<400> 8
   Met Thr Glu Leu Asn Phe His Leu Leu Pro Ile Ile Ser Asp Arg Phe
   1 5 10 15
   Thr Thr Thr Thr Thr Thr Ser Pro Ser Phe Ser Ser His Ser Ser Ser
   20 25 30
   Ser Ser Ser Leu Leu Ser Phe Thr Lys Arg Arg Arg Lys His Gln Pro
   35 40 45
   Leu Val Ser Ser Ile Arg Met Glu Gln Ser Arg Ser Arg Asn Arg Lys
   50 55 60
   Asp Lys Val Val Val Ile Leu Gly Ala Thr Gly Ala Gly Lys Ser Arg
   65 70 75 80
   Leu Ser Val Asp Leu Ala Thr Arg Phe Pro Ser Glu Ile Ile Asn Ser
   85 90 95
   Asp Lys Ile Gln Val Tyr Glu Gly Leu Glu Ile Thr Thr Asn Gln Ile
   100 105 110
   Thr Leu Gln Asp Arg Arg Gly Val Pro His His Leu Leu Gly Val Ile
   115 120 125
   Asn Pro Glu His Gly Glu Leu Thr Ala Gly Glu Phe Arg Ser Ala Ala
   130 135 140
   Ser Asn Val Val Lys Glu Ile Thr Ser Arg Gln Lys Val Pro Ile Ile
   145 150 155 160
   Ala Gly Gly Ser Asn Ser Phe Val His Ala Leu Leu Ala Gln Arg Phe
   165 170 175
   Asp Pro Lys Phe Asp Pro Phe Ser Ser Gly Ser Cys Leu Ile Ser Ser
   180 185 190
   Asp Leu Arg Tyr Glu Cys Cys Phe Ile Trp Val Asp Val Ser Glu Thr
   195 200 205
   Val Leu Tyr Glu Tyr Leu Leu Arg Arg Val Asp Glu Met Met Asp Ser
   210 215 220
   Gly Met Phe Glu Glu Leu Ser Arg Phe Tyr Asp Pro Val Lys Ser Gly
   225                 230                 235                 240
   Leu Glu Thr Arg Phe Gly Ile Arg Lys Ala Ile Gly Val Pro Glu Phe
                   245                 250                 255
   Asp Gly Tyr Phe Lys Glu Tyr Pro Pro Glu Lys Lys Met Ile Lys Trp
               260                 265                 270
   Asp Ala Leu Arg Lys Ala Ala Tyr Asp Lys Ala Val Asp Asp Ile Lys
           275                 280                 285
   Arg Asn Thr Trp Thr Leu Ala Lys Arg Gln Val Lys Lys Ile Glu Met
       290                 295                 300
   Leu Lys Asp Ala Gly Trp Glu Ile Glu Arg Val Asp Ala Thr Ala Ser
   305                 310                 315                 320
   Phe Lys Ala Val Met Met Lys Ser Ser Ser Glu Lys Lys Trp Arg Glu
                   325                 330                 335
   Asn Trp Glu Glu Gln Val Leu Glu Pro Ser Val Lys Ile Val Lys Arg
               340                 345                 350
   His Leu Val Gln Asn
           355
<210> 9
   <211> 1011
   <212> DNA
   <213> Arabidopsis thaliana
<220>
   <221> CDS
   <222> (1).. (1011)
<400> 9
   atg atc atg aag ata tct atg gct atg tgc aaa caa cca ttg cct cct 48
   Met Ile Met Lys Ile Ser Met Ala Met Cys Lys Gln Pro Leu Pro Pro
   1 5 10 15
   tcg ccg act tta gac ttc cct cca gcg aga ttt ggt ccc aat atg cta 96
   Ser Pro Thr Leu Asp Phe Pro Pro Ala Arg Phe Gly Pro Asn Met Leu
   20 25 30
   act cta aac cca tac ggt cca aag gac aaa gtt gtg gtc atc atg ggt 144
   Thr Leu Asn Pro Tyr Gly Pro Lys Asp Lys Val Val Val Ile Met Gly
   35 40 45
   gct acc ggg aca ggc aag tca cga ctc tcc gtg gat ata gcc aca cgt 192
   Ala Thr Gly Thr Gly Lys Ser Arg Leu Ser Val Asp Ile Ala Thr Arg
   50 55 60
   ttt cgg gct gag atc ata aac tca gac aag ata caa gtc cac caa ggt 240
   Phe Arg Ala Glu Ile Ile Asn Ser Asp Lys Ile Gln Val His Gln Gly
   65 70 75 80
   cta gac att gta acc aac aag atc acg agc gag gag agc tgc ggg gta 288
   Leu Asp Ile Val Thr Asn Lys Ile Thr Ser Glu Glu Ser Cys Gly Val
   85 90 95
   ccg cac cat ctc ctc ggc gtc ttg ccg cct gaa gcc gac tta acc gcc 336
   Pro His His Leu Leu Gly Val Leu Pro Pro Glu Ala Asp Leu Thr Ala
   100 105 110
   gcg aat tac tgt cac atg gcg aat ctc tcc att gaa tcc gtc cta aac 384
   Ala Asn Tyr Cys His Met Ala Asn Leu Ser Ile Glu Ser Val Leu Asn
   115 120 125
   cgt gga aag ctt cca atc atc gtt gga ggt tcc aac tct tac gtg gag 432
   Arg Gly Lys Leu Pro Ile Ile Val Gly Gly Ser Asn Ser Tyr Val Glu
   130 135 140
   gct cta gtg gat gac aaa gaa aac aag ttc agg tcg aga tac gac tgt 480
   Ala Leu Val Asp Asp Lys Glu Asn Lys Phe Arg Ser Arg Tyr Asp Cys
   145 150 155 160
   tgt ttt cta tgg gtg gac gtg gca ctt ccc gtt ttg cac ggg ttc gtg 528
   Cys Phe Leu Trp Val Asp Val Ala Leu Pro Val Leu His Gly Phe Val
   165 170 175
   tct gag aga gtt gac aag atg gtg gag agt gga atg gtt gag gaa gtc 576
   Ser Glu Arg Val Asp Lys Met Val Glu Ser Gly Met Val Glu Glu Val
   180 185 190
   aga gaa ttt ttc gac ttt tcg aac tct gat tac tca aga ggg atc aag 624
   Arg Glu Phe Phe Asp Phe Ser Asn Ser Asp Tyr Ser Arg Gly Ile Lys
   195 200 205
   aaa gca atc gga ttt ccg gag ttt gac agg ttt ttc agg aac gag cag 672
   Lys Ala Ile Gly Phe Pro Glu Phe Asp Arg Phe Phe Arg Asn Glu Gln
   210 215 220
   ttc ttg aat gtg gaa gac aga gaa gaa ctg tta agt aaa gtg ttg gaa 720
   Phe Leu Asn Val Glu Asp Arg Glu Glu Leu Leu Ser Lys Val Leu Glu
   225 230 235 240
   gaa ata aag agg aat aca ttt gag tta gct tgt agg cag aga gaa aag 768
   Glu Ile Lys Arg Asn Thr Phe Glu Leu Ala Cys Arg Gln Arg Glu Lys
   245 250 255
   atc gaa cgg ttg aga aaa gtg aag aag tgg tct att cag aga gtg gat 816
   Ile Glu Arg Leu Arg Lys Val Lys Lys Trp Ser Ile Gln Arg Val Asp
   260 265 270
   gcg act cca gtc ttt aca aag cga agg tcc aag atg gat gct aac gtg 864
   Ala Thr Pro Val Phe Thr Lys Arg Arg Ser Lys Met Asp Ala Asn Val
   275 280 285
   gcc tgg gag agg ctc gtg gct gga cca agc aca gat act gtg tcg cgg 912
   Ala Trp Glu Arg Leu Val Ala Gly Pro Ser Thr Asp Thr Val Ser Arg
   290 295 300
   ttt ctg ctg gac att gcc agc cga cga ccg ctc gtg gaa gct tca aca 960
   Phe Leu Leu Asp Ile Ala Ser Arg Arg Pro Leu Val Glu Ala Ser Thr
   305 310 315 320
   gcg gtt gcg gcc gcc atg gaa cgc gag ttg tcg cgg tgt cta gtg gcg 1008
   Ala Val Ala Ala Ala Met Glu Arg Glu Leu Ser Arg Cys Leu Val Ala
   325 330 335
   tga 1011
<210> 10
   <211> 336
   <212> PRT
   <213> Arabidopsis thaliana
<400> 10
   Met Ile Met Lys Ile Ser Met Ala Met Cys Lys Gln Pro Leu Pro Pro
   1 5 10 15
   Ser Pro Thr Leu Asp Phe Pro Pro Ala Arg Phe Gly Pro Asn Met Leu
   20 25 30
   Thr Leu Asn Pro Tyr Gly Pro Lys Asp Lys Val Val Val Ile Met Gly
   35 40 45
   Ala Thr Gly Thr Gly Lys Ser Arg Leu Ser Val Asp Ile Ala Thr Arg
   50 55 60
   Phe Arg Ala Glu Ile Ile Asn Ser Asp Lys Ile Gln Val His Gln Gly
   65 70 75 80
   Leu Asp Ile Val Thr Asn Lys Ile Thr Ser Glu Glu Ser Cys Gly Val
   85 90 95
   Pro His His Leu Leu Gly Val Leu Pro Pro Glu Ala Asp Leu Thr Ala
   100 105 110
   Ala Asn Tyr Cys His Met Ala Asn Leu Ser Ile Glu Ser Val Leu Asn
   115 120 125
   Arg Gly Lys Leu Pro Ile Ile Val Gly Gly Ser Asn Ser Tyr Val Glu
   130 135 140
   Ala Leu Val Asp Asp Lys Glu Asn Lys Phe Arg Ser Arg Tyr Asp Cys
   145 150 155 160
   Cys Phe Leu Trp Val Asp Val Ala Leu Pro Val Leu His Gly Phe Val
   165 170 175
   Ser Glu Arg Val Asp Lys Met Val Glu Ser Gly Met Val Glu Glu Val
   180 185 190
   Arg Glu Phe Phe Asp Phe Ser Asn Ser Asp Tyr Ser Arg Gly Ile Lys
   195 200 205
   Lys Ala Ile Gly Phe Pro Glu Phe Asp Arg Phe Phe Arg Asn Glu Gln
   210 215 220
   Phe Leu Asn Val Glu Asp Arg Glu Glu Leu Leu Ser Lys Val Leu Glu
   225 230 235 240
   Glu Ile Lys Arg Asn Thr Phe Glu Leu Ala Cys Arg Gln Arg Glu Lys
   245 250 255
   Ile Glu Arg Leu Arg Lys Val Lys Lys Trp Ser Ile Gln Arg Val Asp
   260 265 270
   Ala Thr Pro Val Phe Thr Lys Arg Arg Ser Lys Met Asp Ala Asn Val
   275 280 285
   Ala Trp Glu Arg Leu Val Ala Gly Pro Ser Thr Asp Thr Val Ser Arg
   290 295 300
   Phe Leu Leu Asp Ile Ala Ser Arg Arg Pro Leu Val Glu Ala Ser Thr
   305 310 315 320
   Ala Val Ala Ala Ala Met Glu Arg Glu Leu Ser Arg Cys Leu Val Ala
   325 330 335
<210> 11
   <211> 957
   <212> DNA
   <213> Arabidopsis thaliana
<220>
   <221> CDS
   <222> (1).. (957)
<400> 11
   atg aag tgt aat gac aaa atg gtt gtg atc atg ggt gcc acc ggt tct 48
   Met Lys Cys Asn Asp Lys Met Val Val Ile Met Gly Ala Thr Gly Ser
   1 5 10 15
   ggc aag tca tca ctc tct gtt gat ctc gct tta cat ttt aaa gcc gag 96
   Gly Lys Ser Ser Leu Ser Val Asp Leu Ala Leu His Phe Lys Ala Glu
   20 25 30
   atc atc aac tct gac aaa atg cag ttc tac gat ggc ttg aag atc acc 144
   Ile Ile Asn Ser Asp Lys Met Gln Phe Tyr Asp Gly Leu Lys Ile Thr
   35 40 45
   acg aat caa tcg acc att gaa gat cga cgt gga gtg cca cat cac ctt 192
   Thr Asn Gln Ser Thr Ile Glu Asp Arg Arg Gly Val Pro His His Leu
   50 55 60
   ctc ggt gaa cta aac ccg gag gct gga gaa gtc aca gcg gca gaa ttt 240
   Leu Gly Glu Leu Asn Pro Glu Ala Gly Glu Val Thr Ala Ala Glu Phe
   65 70 75 80
   cgc gtt atg gcg gct gaa gcc atc tcc gag att act caa cgt aaa aag 288
   Arg Val Met Ala Ala Glu Ala Ile Ser Glu Ile Thr Gln Arg Lys Lys
   85 90 95
   ctc cca atc ctt gcc ggt gga tcc aac tca tac att cat gct ctc ctt 336
   Leu Pro Ile Leu Ala Gly Gly Ser Asn Ser Tyr Ile His Ala Leu Leu
   100 105 110
   gca aaa tct tat gac cct gaa aac tat ccg ttt tct gat cac aag ggc 384
   Ala Lys Ser Tyr Asp Pro Glu Asn Tyr Pro Phe Ser Asp His Lys Gly
   115 120 125
   tca atc tgc tcc gag ttg aaa tat gat tgt tgt ttc att tgg ata gat 432
   Ser Ile Cys Ser Glu Leu Lys Tyr Asp Cys Cys Phe Ile Trp Ile Asp
   130 135 140
   gtg gat cag tct gtg tta ttc gag tat ctt tct tta cgt ttg gat ctt 480
   Val Asp Gln Ser Val Leu Phe Glu Tyr Leu Ser Leu Arg Leu Asp Leu
   145 150 155 160
   atg atg aag tca ggt atg ttc gag gag atc gct gag ttc cac cgc tct 528
   Met Met Lys Ser Gly Met Phe Glu Glu Ile Ala Glu Phe His Arg Ser
   165 170 175
   aag aag gcc ccg aaa gag cca ttg ggg ata tgg aag gct ata gga gtg 576
   Lys Lys Ala Pro Lys Glu Pro Leu Gly Ile Trp Lys Ala Ile Gly Val
   180 185 190
   caa gag ttt gat gac tac ctc aaa atg tac aag tgg gac aat gac atg 624
   Gln Glu Phe Asp Asp Tyr Leu Lys Met Tyr Lys Trp Asp Asn Asp Met
   195 200 205
   gat aaa tgg gac cct atg aga aag gag gct tat gag aag gcg gtg aga 672
   Asp Lys Trp Asp Pro Met Arg Lys Glu Ala Tyr Glu Lys Ala Val Arg
   210 215 220
   gcc atc aaa gaa aac aca ttt cag ctc aca aag gat caa atc acg aag 720
   Ala Ile Lys Glu Asn Thr Phe Gln Leu Thr Lys Asp Gln Ile Thr Lys
   225 230 235 240
   atc aac aag ctg aga aat gcc ggg tgg gac ata aag aag gtg gat gct 768
   Ile Asn Lys Leu Arg Asn Ala Gly Trp Asp Ile Lys Lys Val Asp Ala
   245 250 255
   aca gca tcg ttt cga gag gca att agg gca gcc aag gaa ggc gaa ggt 816
   Thr Ala Ser Phe Arg Glu Ala Ile Arg Ala Ala Lys Glu Gly Glu Gly
   260 265 270
   gta gcc gag atg cag aga aag ata tgg aac aag gaa gtg ttg gaa ccg 864
   Val Ala Glu Met Gln Arg Lys Ile Trp Asn Lys Glu Val Leu Glu Pro
   275 280 285
   tgt gtg aag att gtc agg agc cac ttg gac caa ccg atc aac tat tat 912
   Cys Val Lys Ile Val Arg Ser His Leu Asp Gln Pro Ile Asn Tyr Tyr
   290 295 300
   tat tat tac ttt tat tta cta aaa aga ttt tta agt ctt aac tag 957
   Tyr Tyr Tyr Phe Tyr Leu Leu Lys Arg Phe Leu Ser Leu Asn
   305 310 315
<210> 12
   <211> 318
   <212> PRT
   <213> Arabidopsis thaliana
<400> 12
   Met Lys Cys Asn Asp Lys Met Val Val Ile Met Gly Ala Thr Gly Ser
   1 5 10 15
   Gly Lys Ser Ser Leu Ser Val Asp Leu Ala Leu His Phe Lys Ala Glu
   20 25 30
   Ile Ile Asn Ser Asp Lys Met Gln Phe Tyr Asp Gly Leu Lys Ile Thr
   35 40 45
   Thr Asn Gln Ser Thr Ile Glu Asp Arg Arg Gly Val Pro His His Leu
   50 55 60
   Leu Gly Glu Leu Asn Pro Glu Ala Gly Glu Val Thr Ala Ala Glu Phe
   65 70 75 80
   Arg Val Met Ala Ala Glu Ala Ile Ser Glu Ile Thr Gln Arg Lys Lys
   85 90 95
   Leu Pro Ile Leu Ala Gly Gly Ser Asn Ser Tyr Ile His Ala Leu Leu
   100 105 110
   Ala Lys Ser Tyr Asp Pro Glu Asn Tyr Pro Phe Ser Asp His Lys Gly
   115 120 125
   Ser Ile Cys Ser Glu Leu Lys Tyr Asp Cys Cys Phe Ile Trp Ile Asp
   130 135 140
   Val Asp Gln Ser Val Leu Phe Glu Tyr Leu Ser Leu Arg Leu Asp Leu
   145 150 155 160
   Met Met Lys Ser Gly Met Phe Glu Glu Ile Ala Glu Phe His Arg Ser
   165 170 175
   Lys Lys Ala Pro Lys Glu Pro Leu Gly Ile Trp Lys Ala Ile Gly Val
   180 185 190
   Gln Glu Phe Asp Asp Tyr Leu Lys Met Tyr Lys Trp Asp Asn Asp Met
   195 200 205
   Asp Lys Trp Asp Pro Met Arg Lys Glu Ala Tyr Glu Lys Ala Val Arg
   210 215 220
   Ala Ile Lys Glu Asn Thr Phe Gln Leu Thr Lys Asp Gln Ile Thr Lys
   225 230 235 240
   Ile Asn Lys Leu Arg Asn Ala Gly Trp Asp Ile Lys Lys Val Asp Ala
   245 250 255
   Thr Ala Ser Phe Arg Glu Ala Ile Arg Ala Ala Lys Glu Gly Glu Gly
   260 265 270
   Val Ala Glu Met Gln Arg Lys Ile Trp Asn Lys Glu Val Leu Glu Pro
   275 280 285
   Cys Val Lys Ile Val Arg Ser His Leu Asp Gln Pro Ile Asn Tyr Tyr
   290 295 300
   Tyr Tyr Tyr Phe Tyr Leu Leu Lys Arg Phe Leu Ser Leu Asn
   305 310 315
<210> 13
   <211> 993
   <212> DNA
   <213> Arabidopsis thaliana
<220>
   <221> CDS
   <222> (1) .. (993)
<400> 13
   atg aag cca tgc atg acg gct cta aga caa gtg att caa cca ttg tcg 48
   Met Lys Pro Cys Met Thr Ala Leu Arg Gln Val Ile Gln Pro Leu Ser
   1 5 10 15
   ttg aac ttc caa gga aac atg gtg gac gtt ccg ttt ttc cgg cga aaa 96
   Leu Asn Phe Gln Gly Asn Met Val Asp Val Pro Phe Phe Arg Arg Lys
   20 25 30
   gac aag gtt gtt ttc gtc atg gga gcc acc gga acc ggc aaa tct cgt 144
   Asp Lys Val Val Phe Val Met Gly Ala Thr Gly Thr Gly Lys Ser Arg
   35 40 45
   cta gcc att gac cta gcc act cgt ttt ccg gcg gag att gta aac tcc 192
   Leu Ala Ile Asp Leu Ala Thr Arg Phe Pro Ala Glu Ile Val Asn Ser
   50 55 60
   gac aag atc cag gtc tat aaa ggt cta gac att gtg act aac aaa gtc 240
   Asp Lys Ile Gln Val Tyr Lys Gly Leu Asp Ile Val Thr Asn Lys Val
   65 70 75 80
   act cct gag gaa agc ctt ggc gtt cct cac cac ctt ctc ggc acc gtc 288
   Thr Pro Glu Glu Ser Leu Gly Val Pro His His Leu Leu Gly Thr Val
   85 90 95
   cac gac act tac gaa gat ttc acg gcg gag gat ttt cag cgt gaa gca 336
   His Asp Thr Tyr Glu Asp Phe Thr Ala Glu Asp Phe Gln Arg Glu Ala
   100 105 110
   atc agg gcc gtc gag tca atc gtc cag aga gac cgt gtc ccg atc ata 384
   Ile Arg Ala Val Glu Ser Ile Val Gln Arg Asp Arg Val Pro Ile Ile
   115 120 125
   gcc ggt ggt tcc aat tct tac atc gag gct ctg gtc aac gat tgc gtt 432
   Ala Gly Gly Ser Asn Ser Tyr Ile Glu Ala Leu Val Asn Asp Cys Val
   130 135 140
   gac ttc cgg tta agg tat aat tgt tgc ttc ttg tgg gtc gac gtc tct 480
   Asp Phe Arg Leu Arg Tyr Asn Cys Cys Phe Leu Trp Val Asp Val Ser
   145 150 155 160
   aga ccg gtt tta cac tcg ttt gtc tcg gag cga gtt gat aag atg gtt 528
   Arg Pro Val Leu His Ser Phe Val Ser Glu Arg Val Asp Lys Met Val
   165 170 175
   gat atg ggt ctc gtc gac gag gtt cgc cgc atc ttc gat ccg tct tcg 576
   Asp Met Gly Leu Val Asp Glu Val Arg Arg Ile Phe Asp Pro Ser Ser
   180 185 190
   tcg gat tac tcc gct gga att cgc cga gcc att gga gtt cca gag ctc 624
   Ser Asp Tyr Ser Ala Gly Ile Arg Arg Ala Ile Gly Val Pro Glu Leu
   195 200 205
   gac gaa ttt ctc cgt tcg gag atg cgg aat tat ccg gcg gag acg acg 672
   Asp Glu Phe Leu Arg Ser Glu Met Arg Asn Tyr Pro Ala Glu Thr Thr
   210 215 220
   gag aga ctt ctt gaa acg gcg atc gag aag att aaa gag aac act tgt 720
   Glu Arg Leu Leu Glu Thr Ala Ile Glu Lys Ile Lys Glu Asn Thr Cys
   225 230 235 240
   ttg ctt gcg tgt aga caa ttg cag aag att caa agg ctt tac aag cag 768
   Leu Leu Ala Cys Arg Gln Leu Gln Lys Ile Gln Arg Leu Tyr Lys Gln
   245 250 255
   tgg aag tgg aac atg cac cgt gtc gac gcg acg gag gtt ttt ctc cga 816
   Trp Lys Trp Asn Met His Arg Val Asp Ala Thr Glu Val Phe Leu Arg
   260 265 270
   cga gga gaa gaa gct gat gag gct tgg gat aac tca gtg gct cat ccg 864
   Arg Gly Glu Glu Ala Asp Glu Ala Trp Asp Asn Ser Val Ala His Pro
   275 280 285
   agc gca ctc gcc gtc gaa aag ttc ctt agt tac agc gat gac cac cat 912
   Ser Ala Leu Ala Val Glu Lys Phe Leu Ser Tyr Ser Asp Asp His His
   290 295 300
   ttg gaa ggc gcc aat att ctc cta ccg gag atc tct gcc gtt ccg cct 960
   Leu Glu Gly Ala Asn Ile Leu Leu Pro Glu Ile Ser Ala Val Pro Pro
   305 310 315 320
   ctt cca gcc gcc gtg gcg gcg att tcc cgg tga 993
   Leu Pro Ala Ala Val Ala Ala Ile Ser Arg
   325 330
<210> 14
   <211> 330
   <212> PRT
   <213> Arabidopsis thaliana
<400> 14
   Met Lys Pro Cys Met Thr Ala Leu Arg Gln Val Ile Gln Pro Leu Ser
   1 5 10 15
   Leu Asn Phe Gln Gly Asn Met Val Asp Val Pro Phe Phe Arg Arg Lys
   20 25 30
   Asp Lys Val Val Phe Val Met Gly Ala Thr Gly Thr Gly Lys Ser Arg
   35 40 45
   Leu Ala Ile Asp Leu Ala Thr Arg Phe Pro Ala Glu Ile Val Asn Ser
   50 55 60
   Asp Lys Ile Gln Val Tyr Lys Gly Leu Asp Ile Val Thr Asn Lys Val
   65 70 75 80
   Thr Pro Glu Glu Ser Leu Gly Val Pro His His Leu Leu Gly Thr Val
   85 90 95
   His Asp Thr Tyr Glu Asp Phe Thr Ala Glu Asp Phe Gln Arg Glu Ala
   100 105 110
   Ile Arg Ala Val Glu Ser Ile Val Gln Arg Asp Arg Val Pro Ile Ile
   115 120 125
   Ala Gly Gly Ser Asn Ser Tyr Ile Glu Ala Leu Val Asn Asp Cys Val
   130 135 140
   Asp Phe Arg Leu Arg Tyr Asn Cys Cys Phe Leu Trp Val Asp Val Ser
   145 150 155 160
   Arg Pro Val Leu His Ser Phe Val Ser Glu Arg Val Asp Lys Met Val
   165 170 175
   Asp Met Gly Leu Val Asp Glu Val Arg Arg Ile Phe Asp Pro Ser Ser
   180 185 190
   Ser Asp Tyr Ser Ala Gly Ile Arg Arg Ala Ile Gly Val Pro Glu Leu
   195 200 205
   Asp Glu Phe Leu Arg Ser Glu Met Arg Asn Tyr Pro Ala Glu Thr Thr
   210 215 220
   Glu Arg Leu Leu Glu Thr Ala Ile Glu Lys Ile Lys Glu Asn Thr Cys
   225 230 235 240
   Leu Leu Ala Cys Arg Gln Leu Gln Lys Ile Gln Arg Leu Tyr Lys Gln
   245 250 255
   Trp Lys Trp Asn Met His Arg Val Asp Ala Thr Glu Val Phe Leu Arg
   260 265 270
   Arg Gly Glu Glu Ala Asp Glu Ala Trp Asp Asn Ser Val Ala His Pro
   275 280 285
   Ser Ala Leu Ala Val Glu Lys Phe Leu Ser Tyr Ser Asp Asp His His
   290 295 300
   Leu Glu Gly Ala Asn Ile Leu Leu Pro Glu Ile Ser Ala Val Pro Pro
   305 310 315 320
   Leu Pro Ala Ala Val Ala Ala Ile Ser Arg
   325 330
<210> 15
   <211> 1029
   <212> DNA
   <213> Arabidopsis thaliana
<220>
   <221> CDS
   <222> (1) .. (1029)
<400> 15
   atg caa caa ctc atg acc ttg tta tca cca cca ctc tct cat tct tct 48
   Met Gln Gln Leu Met Thr Leu Leu Ser Pro Pro Leu Ser His Ser Ser
   1 5 10 15
   ctc ctt ccc acc gtc act acc aaa ttc ggg tca cca cga tta gtc act 96
   Leu Leu Pro Thr Val Thr Thr Lys Phe Gly Ser Pro Arg Leu Val Thr
   20 25 30
   acg tgc atg ggc cat gca ggg cgt aaa aat atc aag gat aag gtg gtt 144
   Thr Cys Met Gly His Ala Gly Arg Lys Asn Ile Lys Asp Lys Val Val
   35 40 45
   ctc atc aca ggt aca aca ggc aca ggc aag tca cgc ctc tca gtc gat 192
   Leu Ile Thr Gly Thr Thr Gly Thr Gly Lys Ser Arg Leu Ser Val Asp
   50 55 60
   ctt gcc acc cgt ttt ttt ccc gcc gag atc ata aac tcg gac aaa atg 240
   Leu Ala Thr Arg Phe Phe Pro Ala Glu Ile Ile Asn Ser Asp Lys Met
   65 70 75 80
   caa atc tac aag gga ttc gag att gtc aca aat cta atc cca ctg cat 288
   Gln Ile Tyr Lys Gly Phe Glu Ile Val Thr Asn Leu Ile Pro Leu His
   85 90 95
   gag caa gga gga gtc ccg cac cat ctt cta ggt cag ttc cac cca caa 336
   Glu Gln Gly Gly Val Pro His His Leu Leu Gly Gln Phe His Pro Gln
   100 105 110
   gac ggt gaa ctc acc cct gca gag ttc cgt tct ttg gcg aca ctg tcc 384
   Asp Gly Glu Leu Thr Pro Ala Glu Phe Arg Ser Leu Ala Thr Leu Ser
   115 120 125
   atc tct aaa cta att tct agc aag aaa ctc ccg att gta gtt ggt gga 432
   Ile Ser Lys Leu Ile Ser Ser Lys Lys Leu Pro Ile Val Val Gly Gly
   130 135 140
   tcc aac tcc ttc aat cac gct cta ctc gcc gag cgt ttt gac ccg gat 480
   Ser Asn Ser Phe Asn His Ala Leu Leu Ala Glu Arg Phe Asp Pro Asp
   145 150 155 160
   att gat cca ttc tct ccc gga tcg agt ctt tca acg atc tgc tct gac 528
   Ile Asp Pro Phe Ser Pro Gly Ser Ser Leu Ser Thr Ile Cys Ser Asp
   165 170 175
   cta agg tac aaa tgt tgc atc tta tgg gtt gat gtt tta gag ccg gtt 576
   Leu Arg Tyr Lys Cys Cys Ile Leu Trp Val Asp Val Leu Glu Pro Val
   180 185 190
   ctg ttc caa cac ttg tgc aat cgt gtc gac caa atg atc gag tcg gga 624
   Leu Phe Gln His Leu Cys Asn Arg Val Asp Gln Met Ile Glu Ser Gly
   195 200 205
   ttg gtc gag cag ctt gcc gaa ttg tac gac cct gtt gta gat tcg ggt 672
   Leu Val Glu Gln Leu Ala Glu Leu Tyr Asp Pro Val Val Asp Ser Gly
   210 215 220
   cga cga cta ggg gtt cgg aag acg ata gga gta gag gag ttc gac cga 720
   Arg Arg Leu Gly Val Arg Lys Thr Ile Gly Val Glu Glu Phe Asp Arg
   225 230 235 240
   tac ttt aga gta tac cct aag gag atg gac aag gga att tgg gac tta 768
   Tyr Phe Arg Val Tyr Pro Lys Glu Met Asp Lys Gly Ile Trp Asp Leu
   245 250 255
   gcg aga aag gcg gcg tac gag gag aca gtg aag ggg atg aaa gag agg 816
   Ala Arg Lys Ala Ala Tyr Glu Glu Thr Val Lys Gly Met Lys Glu Arg
   260 265 270
   aca tgt cgg ttg gtg aag aag cag aaa gag aag atc atg aag ctg ata 864
   Thr Cys Arg Leu Val Lys Lys Gln Lys Glu Lys Ile Met Lys Leu Ile
   275 280 285
   aga ggt ggt tgg gag att aag agg ctt gac gct acg gcg gca att atg 912
   Arg Gly Gly Trp Glu Ile Lys Arg Leu Asp Ala Thr Ala Ala Ile Met
   290 295 300
   gct gag ctg aat caa agt acg gca aag gga gaa gga aag aat ggg aga 960
   Ala Glu Leu Asn Gln Ser Thr Ala Lys Gly Glu Gly Lys Asn Gly Arg
   305 310 315 320
   gag att tgg gaa aaa cac att gtg gat gaa agt gtc gag att gtc aag 1008
   Glu Ile Trp Glu Lys His Ile Val Asp Glu Ser Val Glu Ile Val Lys
   325 330 335
   aag ttt ttg ttg gaa gtt tag 1029
   Lys Phe Leu Leu Glu Val
   340
<210> 16
   <211> 342
   <212> PRT
   <213> Arabidopsis thaliana
<400> 16
   Met Gln Gln Leu Met Thr Leu Leu Ser Pro Pro Leu Ser His Ser Ser
   1 5 10 15
   Leu Leu Pro Thr Val Thr Thr Lys Phe Gly Ser Pro Arg Leu Val Thr
   20 25 30
   Thr Cys Met Gly His Ala Gly Arg Lys Asn Ile Lys Asp Lys Val Val
   35 40 45
   Leu Ile Thr Gly Thr Thr Gly Thr Gly Lys Ser Arg Leu Ser Val Asp
   50 55 60
   Leu Ala Thr Arg Phe Phe Pro Ala Glu Ile Ile Asn Ser Asp Lys Met
   65 70 75 80
   Gln Ile Tyr Lys Gly Phe Glu Ile Val Thr Asn Leu Ile Pro Leu His
   85 90 95
   Glu Gln Gly Gly Val Pro His His Leu Leu Gly Gln Phe His Pro Gln
   100 105 110
   Asp Gly Glu Leu Thr Pro Ala Glu Phe Arg Ser Leu Ala Thr Leu Ser
   115 120 125
   Ile Ser Lys Leu Ile Ser Ser Lys Lys Leu Pro Ile Val Val Gly Gly
   130 135 140
   Ser Asn Ser Phe Asn His Ala Leu Leu Ala Glu Arg Phe Asp Pro Asp
   145 150 155 160
   Ile Asp Pro Phe Ser Pro Gly Ser Ser Leu Ser Thr Ile Cys Ser Asp
   165 170 175
   Leu Arg Tyr Lys Cys Cys Ile Leu Trp Val Asp Val Leu Glu Pro Val
   180 185 190
   Leu Phe Gln His Leu Cys Asn Arg Val Asp Gln Met Ile Glu Ser Gly
   195 200 205
   Leu Val Glu Gln Leu Ala Glu Leu Tyr Asp Pro Val Val Asp Ser Gly
   210 215 220
   Arg Arg Leu Gly Val Arg Lys Thr Ile Gly Val Glu Glu Phe Asp Arg
   225 230 235 240
   Tyr Phe Arg Val Tyr Pro Lys Glu Met Asp Lys Gly Ile Trp Asp Leu
   245 250 255
   Ala Arg Lys Ala Ala Tyr Glu Glu Thr Val Lys Gly Met Lys Glu Arg
   260 265 270
   Thr Cys Arg Leu Val Lys Lys Gln Lys Glu Lys Ile Met Lys Leu Ile
   275 280 285
   Arg Gly Gly Trp Glu Ile Lys Arg Leu Asp Ala Thr Ala Ala Ile Met
   290 295 300
   Ala Glu Leu Asn Gln Ser Thr Ala Lys Gly Glu Gly Lys Asn Gly Arg
   305 310 315 320
   Glu Ile Trp Glu Lys His Ile Val Asp Glu Ser Val Glu Ile Val Lys
   325 330 335
   Lys Phe Leu Leu Glu Val
   340
<210> 17
   <211> 990
   <212> DNA
   <213> Arabidopsis thaliana
<220>
   <221> CDS
   <222> (1) .. (990)
<400> 17
   atg aag ttc tca atc tca tca ctg aag cag gta caa cca atc ttg tgc 48
   Met Lys Phe Ser Ile Ser Ser Leu Lys Gln Val Gln Pro Ile Leu Cys
   1 5 10 15
   ttc aag aac aag cta tct aag gtc aac gtc aac tct ttt ctc cat ccc 96
   Phe Lys Asn Lys Leu Ser Lys Val Asn Val Asn Ser Phe Leu His Pro
   20 25 30
   aaa gaa aaa gtc atc ttt gtg atg gga gct acc gga tcg ggt aag tct 144
   Lys Glu Lys Val Ile Phe Val Met Gly Ala Thr Gly Ser Gly Lys Ser
   35 40 45
   cgt ctc gcc atc gac cta gca act cgt ttt caa gga gag atc ata aac 192
   Arg Leu Ala Ile Asp Leu Ala Thr Arg Phe Gln Gly Glu Ile Ile Asn
   50 55 60
   tcc gac aag att caa ctt tac aag ggc cta gac gtc cta aca aac aaa 240
   Ser Asp Lys Ile Gln Leu Tyr Lys Gly Leu Asp Val Leu Thr Asn Lys
   65 70 75 80
   gtc acc cct aaa gaa tgc cga ggc gtg cct cac cac ttg ctt gga gta 288
   Val Thr Pro Lys Glu Cys Arg Gly Val Pro His His Leu Leu Gly Val
   85 90 95
   ttc gac tcc gaa gcc gga aac cta acg gcc acc cag tat agc cgc ctt 336
   Phe Asp Ser Glu Ala Gly Asn Leu Thr Ala Thr Gln Tyr Ser Arg Leu
   100 105 110
   gcg tca caa gca atc tcg aaa ctc tca gcg aac aac aag ctt ccc ata 384
   Ala Ser Gln Ala Ile Ser Lys Leu Ser Ala Asn Asn Lys Leu Pro Ile
   115 120 125
   gta gcc ggt gga tca aac tct tac atc gaa gca ctt gtt aat cat tcc 432
   Val Ala Gly Gly Ser Asn Ser Tyr Ile Glu Ala Leu Val Asn His Ser
   130 135 140
   tcg ggg ttt tta tta aac aac tac gat tgt tgt ttc att tgg gtc gac 480
   Ser Gly Phe Leu Leu Asn Asn Tyr Asp Cys Cys Phe Ile Trp Val Asp
   145 150 155 160
   gtt tcc tta ccc gta ctt aac tcc ttt gtc tca aaa cgt gtc gac cgc 528
   Val Ser Leu Pro Val Leu Asn Ser Phe Val Ser Lys Arg Val Asp Arg
   165 170 175
   atg atg gaa gca gga tta ctc gaa gaa gta aga gaa gtg ttc aat cca 576
   Met Met Glu Ala Gly Leu Leu Glu Glu Val Arg Glu Val Phe Asn Pro
   180 185 190
   aaa gcg aat tac tcc gta ggg ata cga cga gct atc gga gtc ccc gag 624
   Lys Ala Asn Tyr Ser Val Gly Ile Arg Arg Ala Ile Gly Val Pro Glu
   195 200 205
   ctc cat gaa tat tta cgt aac gaa tct cta gtg gac cgt gcc aca aaa 672
   Leu His Glu Tyr Leu Arg Asn Glu Ser Leu Val Asp Arg Ala Thr Lys
   210 215 220
   agt aaa atg ctt gac gta gcc gtt aaa aat atc aaa aag aac act gag 720
   Ser Lys Met Leu Asp Val Ala Val Lys Asn Ile Lys Lys Asn Thr Glu
   225 230 235 240
   att tta gct tgt cga cag tta aaa aag att caa cgg ctt cac aag aag 768
   Ile Leu Ala Cys Arg Gln Leu Lys Lys Ile Gln Arg Leu His Lys Lys
   245 250 255
   tgg aag atg tct atg cat cgt gtt gac gcc act gag gtg ttc ttg aaa 816
   Trp Lys Met Ser Met His Arg Val Asp Ala Thr Glu Val Phe Leu Lys
   260 265 270
   cgc aac gta gaa gaa caa gac gag gct tgg gag aat ctt gta gcg aga 864
   Arg Asn Val Glu Glu Gln Asp Glu Ala Trp Glu Asn Leu Val Ala Arg
   275 280 285
   cca agc gag aga atc gtc gat aag ttt tat aat aat aat aac caa ctg 912
   Pro Ser Glu Arg Ile Val Asp Lys Phe Tyr Asn Asn Asn Asn Gln Leu
   290 295 300
   aaa aat gat gat gtt gag cac tgt ttg gcg gca tct tac ggc gga gga 960
   Lys Asn Asp Asp Val Glu His Cys Leu Ala Ala Ser Tyr Gly Gly Gly
   305 310 315 320
   agt gga agt aga gcc cac aat atg ata tga 990
   Ser Gly Ser Arg Ala His Asn Met Ile
   325
<210> 18
   <211> 329
   <212> PRT
   <213> Arabidopsis thaliana
<400> 18
   Met Lys Phe Ser Ile Ser Ser Leu Lys Gln Val Gln Pro Ile Leu Cys
   1 5 10 15
   Phe Lys Asn Lys Leu Ser Lys Val Asn Val Asn Ser Phe Leu His Pro
   20 25 30
   Lys Glu Lys Val Ile Phe Val Met Gly Ala Thr Gly Ser Gly Lys Ser
   35 40 45
   Arg Leu Ala Ile Asp Leu Ala Thr Arg Phe Gln Gly Glu Ile Ile Asn
   50 55 60
   Ser Asp Lys Ile Gln Leu Tyr Lys Gly Leu Asp Val Leu Thr Asn Lys
   65 70 75 80
   Val Thr Pro Lys Glu Cys Arg Gly Val Pro His His Leu Leu Gly Val
   85 90 95
   Phe Asp Ser Glu Ala Gly Asn Leu Thr Ala Thr Gln Tyr Ser Arg Leu
   100 105 110
   Ala Ser Gln Ala Ile Ser Lys Leu Ser Ala Asn Asn Lys Leu Pro Ile
   115 120 125
   Val Ala Gly Gly Ser Asn Ser Tyr Ile Glu Ala Leu Val Asn His Ser
   130 135 140
   Ser Gly Phe Leu Leu Asn Asn Tyr Asp Cys Cys Phe Ile Trp Val Asp
   145 150 155 160
   Val Ser Leu Pro Val Leu Asn Ser Phe Val Ser Lys Arg Val Asp Arg
   165 170 175
   Met Met Glu Ala Gly Leu Leu Glu Glu Val Arg Glu Val Phe Asn Pro
   180 185 190
   Lys Ala Asn Tyr Ser Val Gly Ile Arg Arg Ala Ile Gly Val Pro Glu
   195 200 205
   Leu His Glu Tyr Leu Arg Asn Glu Ser Leu Val Asp Arg Ala Thr Lys
   210 215 220
   Ser Lys Met Leu Asp Val Ala Val Lys Asn Ile Lys Lys Asn Thr Glu
   225 230 235 240
   Ile Leu Ala Cys Arg Gln Leu Lys Lys Ile Gln Arg Leu His Lys Lys
   245 250 255
   Trp Lys Met Ser Met His Arg Val Asp Ala Thr Glu Val Phe Leu Lys
   260 265 270
   Arg Asn Val Glu Glu Gln Asp Glu Ala Trp Glu Asn Leu Val Ala Arg
   275 280 285
   Pro Ser Glu Arg Ile Val Asp Lys Phe Tyr Asn Asn Asn Asn Gln Leu
   290 295 300
   Lys Asn Asp Asp Val Glu His Cys Leu Ala Ala Ser Tyr Gly Gly Gly
   305 310 315 320
   Ser Gly Ser Arg Ala His Asn Met Ile
   325
<210> 19
   <211> 993
   <212> DNA
   <213> Arabidopsis thaliana
<220>
   <221> CDS
   <222> (1) .. (993)
<400> 19
   atg caa aat ctt acg tcc aca ttc gtc tct cct tcc atg atc ccg atc 48
   Met Gln Asn Leu Thr Ser Thr Phe Val Ser Pro Ser Met Ile Pro Ile
   1 5 10 15
   act tct ccg cgg ctg cga ctg cca cca cca cga tca gta gtt ccc atg 96
   Thr Ser Pro Arg Leu Arg Leu Pro Pro Pro Arg Ser Val Val Pro Met
   20 25 30
   act acc gtt tgc atg gaa caa tca tac aag caa aaa gtg gtt gtg atc 144
   Thr Thr Val Cys Met Glu Gln Ser Tyr Lys Gln Lys Val Val Val Ile
   35 40 45
   atg gga gcc acc gga tca ggc aag tca tgc ctc tca atc gat cta gca 192
   Met Gly Ala Thr Gly Ser Gly Lys Ser Cys Leu Ser Ile Asp Leu Ala
   50 55 60
   act cgt ttc tct ggc gag atc gtc aat tcc gac aag att caa ttc tac 240
   Thr Arg Phe Ser Gly Glu Ile Val Asn Ser Asp Lys Ile Gln Phe Tyr
   65 70 75 80
   gat gga ttg aag gtc act acg aat caa atg agc atc ctt gag aga tgt 288
   Asp Gly Leu Lys Val Thr Thr Asn Gln Met Ser Ile Leu Glu Arg Cys
   85 90 95
   gga gtc cct cac cat ctc ctt ggt gag ctc cct ccg gat gat agc gaa 336
   Gly Val Pro His His Leu Leu Gly Glu Leu Pro Pro Asp Asp Ser Glu
   100 105 110
   cta act acc tcc gaa ttc cgc tct ttg gcg tcg cgg tcc atc tcc gag 384
   Leu Thr Thr Ser Glu Phe Arg Ser Leu Ala Ser Arg Ser Ile Ser Glu
   115 120 125
   att act gct cgt gga aac ctc ccg att ata gct ggt gga tca aac tcc 432
   Ile Thr Ala Arg Gly Asn Leu Pro Ile Ile Ala Gly Gly Ser Asn Ser
   130 135 140
   ttc att cat gct ctc ctt gtc gac cgt ttt gac ccc aaa acc tat cca 480
   Phe Ile His Ala Leu Leu Val Asp Arg Phe Asp Pro Lys Thr Tyr Pro
   145 150 155 160
   ttc tct tct gag aca tcc atc tct tcc ggc ttg agg tac gag tgt tgc 528
   Phe Ser Ser Glu Thr Ser Ile Ser Ser Gly Leu Arg Tyr Glu Cys Cys
   165 170 175
   ttc ctt tgg gtg gat gtc tca gtg tcg gtc ctg ttc gag tac ctc tcg 576
   Phe Leu Trp Val Asp Val Ser Val Ser Val Leu Phe Glu Tyr Leu Ser
   180 185 190
   aaa cgt gtc gac cag atg atg gag tca ggg atg ttc gag gag cta gcc 624
   Lys Arg Val Asp Gln Met Met Glu Ser Gly Met Phe Glu Glu Leu Ala
   195 200 205
   ggt ttc tac gac ccg aga tat tcc ggg tcc gca atc cga gcc cac ggg 672
   Gly Phe Tyr Asp Pro Arg Tyr Ser Gly Ser Ala Ile Arg Ala His Gly
   210 215 220
   att cac aag acc ata gga ata ccc gag ttc gac cgg tac ttc agc tta 720
   Ile His Lys Thr Ile Gly Ile Pro Glu Phe Asp Arg Tyr Phe Ser Leu
   225 230 235 240
   tac ccg cct gag aga aag cag aag atg tcc gaa tgg gac caa gca aga 768
   Tyr Pro Pro Glu Arg Lys Gln Lys Met Ser Glu Trp Asp Gln Ala Arg
   245 250 255
   aag ggg gcg tat gac gaa gct gtc caa gag atc aaa gag aac aca tgg 816
   Lys Gly Ala Tyr Asp Glu Ala Val Gln Glu Ile Lys Glu Asn Thr Trp
   260 265 270
   agg ctt gcg aag aag cag att gag agg atc atg aag ctg aaa agc agc 864
   Arg Leu Ala Lys Lys Gln Ile Glu Arg Ile Met Lys Leu Lys Ser Ser
   275 280 285
   gga tgg gac att cag agg ttg gac gct acg ccg tca ttt gga aga tcg 912
   Gly Trp Asp Ile Gln Arg Leu Asp Ala Thr Pro Ser Phe Gly Arg Ser
   290 295 300
   tca aga gag att tgg gac aat act gtt ttg gat gaa agc atc aag gtt 960
   Ser Arg Glu Ile Trp Asp Asn Thr Val Leu Asp Glu Ser Ile Lys Val
   305 310 315 320
   gtg aaa cgc ttc ttg gtg aaa gac aaa gtg tga 993
   Val Lys Arg Phe Leu Val Lys Asp Lys Val
   325 330
<210> 20
   <211> 330
   <212> PRT
   <213> Arabidopsis thaliana
<400> 20
   Met Gln Asn Leu Thr Ser Thr Phe Val Ser Pro Ser Met Ile Pro Ile
   1 5 10 15
   Thr Ser Pro Arg Leu Arg Leu Pro Pro Pro Arg Ser Val Val Pro Met
   20 25 30
   Thr Thr Val Cys Met Glu Gln Ser Tyr Lys Gln Lys Val Val Val Ile
   35 40 45
   Met Gly Ala Thr Gly Ser Gly Lys Ser Cys Leu Ser Ile Asp Leu Ala
   50 55 60
   Thr Arg Phe Ser Gly Glu Ile Val Asn Ser Asp Lys Ile Gln Phe Tyr
   65 70 75 80
   Asp Gly Leu Lys Val Thr Thr Asn Gln Met Ser Ile Leu Glu Arg Cys
   85 90 95
   Gly Val Pro His His Leu Leu Gly Glu Leu Pro Pro Asp Asp Ser Glu
   100 105 110
   Leu Thr Thr Ser Glu Phe Arg Ser Leu Ala Ser Arg Ser Ile Ser Glu
   115 120 125
   Ile Thr Ala Arg Gly Asn Leu Pro Ile Ile Ala Gly Gly Ser Asn Ser
   130 135 140
   Phe Ile His Ala Leu Leu Val Asp Arg Phe Asp Pro Lys Thr Tyr Pro
   145 150 155 160
   Phe Ser Ser Glu Thr Ser Ile Ser Ser Gly Leu Arg Tyr Glu Cys Cys
   165 170 175
   Phe Leu Trp Val Asp Val Ser Val Ser Val Leu Phe Glu Tyr Leu Ser
   180 185 190
   Lys Arg Val Asp Gln Met Met Glu Ser Gly Met Phe Glu Glu Leu Ala
   195 200 205
   Gly Phe Tyr Asp Pro Arg Tyr Ser Gly Ser Ala Ile Arg Ala His Gly
   210 215 220
   Ile His Lys Thr Ile Gly Ile Pro Glu Phe Asp Arg Tyr Phe Ser Leu
   225 230 235 240
   Tyr Pro Pro Glu Arg Lys Gln Lys Met Ser Glu Trp Asp Gln Ala Arg
   245 250 255
   Lys Gly Ala Tyr Asp Glu Ala Val Gln Glu Ile Lys Glu Asn Thr Trp
   260 265 270
   Arg Leu Ala Lys Lys Gln Ile Glu Arg Ile Met Lys Leu Lys Ser Ser
   275 280 285
   Gly Trp Asp Ile Gln Arg Leu Asp Ala Thr Pro Ser Phe Gly Arg Ser
   290 295 300
   Ser Arg Glu Ile Trp Asp Asn Thr Val Leu Asp Glu Ser Ile Lys Val
   305 310 315 320
   Val Lys Arg Phe Leu Val Lys Asp Lys Val
   325 330
<210> 21
   <211> 125
   <212> PRT
   <213> Saccharomyces cerevisiae
<400> 21
   Glu Thr Ser Val Lys Ile Leu Val Val Glu Asp Asn His Val Asn Gln
   1 5 10 15
   Glu Val Ile Lys Arg Met Leu Asn Leu Glu Gly Ile Glu Asn Ile Glu
   20 25 30
   Leu Ala Cys Asp Gly Gln Glu Ala Phe Asp Lys Val Lys Glu Leu Thr
   35 40 45
   Ser Lys Gly Glu Asn Tyr Asn Met Ile Phe Met Asp Val Gln Met Pro
   50 55 60
   Lys Val Asp Gly Leu Leu Ser Thr Lys Met Ile Arg Arg Asp Leu Gly
   65 70 75 80
   Tyr Thr Ser Pro Ile Val Ala Leu Thr Ala Phe Ala Asp Asp Ser Asn
   85 90 95
   Ile Lys Glu Cys Leu Glu Ser Gly Met Asn Gly Phe Leu Ser Lys Pro
   100 105 110
   Ile Lys Arg Pro Lys Leu Lys Thr Ile Leu Thr Glu Phe
   115 120 125
<210> 22
   <211> 118
   <212> PRT
   <213> Escherichia coli
<400> 22
   Asn Asp Asp Met Met Ile Leu Val Val Asp Asp His Pro Ile Asn Arg
   1 5 10 15
   Arg Leu Leu Ala Asp Gln Leu Gly Ser Leu Gly Tyr Gln Cys Lys Thr
   20 25 30
   Ala Asn Asp Gly Val Asp Ala Leu Asn Val Leu Ser Lys Asn His Ile
   35 40 45
   Asp Ile Val Leu Ser Asp Val Asn Met Pro Asn Met Asp Gly Tyr Arg
   50 55 60
   Leu Thr Gln Arg Ile Arg Gln Leu Gly Leu Thr Leu Pro Val Ile Gly
   65 70 75 80
   Val Thr Ala Asn Ala Leu Ala Glu Glu Lys Gln Arg Cys Leu Glu Ser
   85 90 95
   Gly Met Asp Ser Cys Leu Ser Lys Pro Val Thr Leu Asp Val Ile Lys
   100 105 110
   Gln Ser Leu Thr Leu Tyr
   115
<210> 23
   <211> 31
   <212> DNA
   <213> Artificial Sequence
<220>
   <223> Description of Artificial Sequence:Designed oligonucleotide primer for PCR
<400> 23
   accatgaact gggcactcaa caatcatcaa g 31
<210> 24
   <211> 30
   <212> DNA
   <213> Artificial Sequence
<220>
   <223> Description of Artificial Sequence:Designed oligonucleotide primer for PCR
<400> 24
   ggattacgac gaaggtgaga taggattagg 30
<210> 25
   <211> 24
   <212> DNA
   (213) Artificial Sequence
<220>
   <223> Description of Artificial Sequence:Designed oligonucleotide primer for PCR
<400> 25
   tcagatatga actgggcact caac 24
<210> 26
   <211> 24
   <212> DNA
   <213> Artificial Sequence
<220>
   <223> Description of Artificial Sequence:Designed oligonucleotide primer for PCR
<400> 26
   ctcaatgctt ttgttccttg actc 24
<210> 27
   (211) 32
   <212> DNA
   <213> Artificial Sequence
<220>
   <223> Description of Artificial Sequence:Designed oligonucleotide primer for PCR
<400> 27
   tccccgcgga aaatgttctt acggttaggt ag 32
<210> 28
   <211> 33
   <212> DNA
   <213> Artificial Sequence
<220>
   <223> Description of Artificial Sequence:Designed oligonucleotide primer for PCR
<400> 28
   tcggtcgact tatgattctg tatctgaagg cga 33
<210> 29
   <211> 32
   <212> DNA
   <213> Artificial Sequence
<220>
   <223> Description of Artificial Sequence:Designed oligonucleotide primer for PCR
<400> 29
   gatcccagct agctagggcc ctaccgcggg ga 32
<210> 30
   <211> 32
   <212> DNA
   <213> Artificial Sequence
<220>
   <223> Description of Artificial Sequence:Designed oligonucleotide primer for PCR
<400> 30
   ctagtccccg cggtagggcc ctagctagct gg 32
<210> 31
   <211> 31
   <212> DNA
   <213> Artificial Sequence
<220>
   <223> Description of Artificial Sequence:Designed oligonucleotide primer for PCR
<400> 31
   tccccgcgga aaatgtctat aacttgtgag c 31
<210> 32
   <211> 33
   <212> DNA
   <213> Artificial Sequence
<220>
   <223> Description of Artificial Sequence:Designed oligonucleotide primer for PCR
<400> 32
   ctagctagct taacaaggtt caaagaatct tgc 33
<210> 33
   <211> 33
   <212> DNA
   <213> Artificial Sequence
<220>
   <223> Description of Artificial Sequence:Designed oligonucleotide primer for PCR
<400> 33
   tccccgcgga aaatgaaagc acgaggtgag agg 33
<210> 34
   <211> 32
   <212> DNA
   <213> Artificial Sequence
<220>
   <223> Description of Artificial Sequence:Designed oligonucleotide primer for PCR
<400> 34
   ctagctagct taacaaggtt caaagaattt gc 32
<210> 35
   <211> 30
   <212> DNA
   <213> Artificial Sequence
<220>
   <223> Description of Artificial Sequence:Designed oligonucleotide primer for PCR
<400> 35
   aaaatgaagt gtaatgacaa aatggttgtg 30
<210> 36
   <211> 27
   <212> DNA
   <213> Artificial Sequence
<220>
   <223> Description of Artificial Sequence:Designed oligonucleotide primer for PCR
<400> 36
   gacatttgtt ttgcggtgat attagtc 27
<210> 37
   <211> 34
   <212> DNA
   <213> Artificial Sequence
<220>
   <223> Description of Artificial Sequence:Designed oligonucleotide primer for PCR
<400> 37
   ttatacatat gaagccatgc atgacggctc taag 34
<210> 38
   <211> 28
   <212> DNA
   <213> Artificial Sequence
<220>
   <223> Description of Artificial Sequence:Designed oligonucleotide primer for PCR
<400> 38
   cgggatcctc accgggaaat cgccgcca 28

## Description

### BACKGROUND OF THE INVENTION

### FIELD OF THE INVENTION

The present invention relates to a method for analyzing agonist-activity and antagonist-activity of an examinee to cytokinin receptor.

### DESCRIPTION OF THE RELATED ART

Cytokinins are plant hormones relevant to cell division and differentiation of higher plants and are known as important physiologically active substances having functions of inducing division of cells of higher plants, differentiating callus or pith to stems and leaves, preventing ethiolating and defoliating, preventing dropping of fruits, breaking dominance of apical buds, and the like [Cytokinins: Chemistry, Activity, and Function, CRC press (1994)]. In recent years, it has been found that the key enzyme of cytokinin biosynthesis of plants is isopentenyltransferase and that this enzyme transfers an isopentenyl group of dimethylallyl diphosphate to adenosine-5'-triphosphate and/or adenosine-5'-diphosphate [Plant & Cell Physiol. (2001) 42, 677-685; J. Biol. Chem. (2001) 276, 26405-26410; and W02002072818A1].

Document WO 99/06579 describes methods for inhibiting the unwanted action of a hormone in plants comprising the regulated local expression in the plant of one or more genes coding for or leading to hormone antagonists and/or molecules facilitating the action of the hormone of the hormone antagonist at the site of the hormone action and/or molecules interfering with the biosynthesis or catabolism of the hormone, wherein for regulated local expression of one or more antagonists and/or molecular facilitating the action of the hormone antagonists the plant is transformed with a gene or the genes that encodes or encode an enzyme or the enzymes that is or are involved in a keystep or the keysteps in the pathway that leads to the synthesis of the hormone antagonist. One of these hormone antagonists is cytokinin.

Document EP 1241182 A2 describes a method for analyzing antagonist-activity to a cytokinin receptor and a method for analyzing agonist-activity to a cytokinin receptor.

Substances having the activity of controlling biosynthesis of cytokinins can be used as plant growth regulators, for example, agents for preventing the dropping of fruits such as apples and oranges, agents for preventing plants such as rice plants and wheat from falling down by regulating the height of such plants, agents for increasing sweetness of fruits after harvest, and agents for suppressing axillary buds of tobaccoes or roses.

As a method for finding such substances having the activity of controlling biosynthesis of cytokinins, a method, in which examinee substances are directly sprayed to plants to observe and evaluate the physiological changes of the plants, can be employed.

The aforementioned method has a problem in that it is necessary to prepare the examinee substances in amounts sufficient to directly spray them to plants and it takes a great deal of time to grow the plants and to observe and evaluate the physiological changes of the plants after spraying of the examinee substances. Therefore, there has been a demand for development of various methods for quickly finding substances having the activity of controlling biosynthesis of cytokinins with small amounts of examinee substances.

### SUMMARY OF THE INVENTION

Inventors of the present invention have intensively investigated in such situations and they have found a transformed cell co-expressing a cytokinin receptor and a cytokinin biosynthesis enzyme for use in analyzing the activity of controlling biosynthesis of cytokinins of examinee substances and quickly searching substances having the activity of controlling biosynthesis of cytokinins with small amounts of samples, leading to the completion of the present invention.

The present invention provides:
1. A cell transformed with
   a polynucleotide comprising a nucleotide sequence encoding an amino acid sequence of a cytokinin receptor and
   a polynucleotide comprising a nucleotide sequence encoding an amino acid sequence of a cytokinin biosynthesis enzyme (hereinafter sometimes referred to as a transformed cell of the present invention);
2. The transformed cell according to claim 1, wherein said cytokinin receptor is selected from the group consisting of:
   (a) a cytokinin receptor having the amino acid sequence represented by SEQ ID No: 2;
   (b) a cytokinin receptor having the amino acid sequence represented by SEQ ID No: 4;
   (c) a cytokinin receptor having the amino acid sequence represented by SEQ ID No: 6;
   (d) a partially transmembrane region-deleted type cytokinin receptor;
   (e) a cytokinin receptor having the amino acid sequence represented by amino acids 196 to 1176 of SEQ ID No: 4;
   (f) a cytokinin receptor having the amino acid sequence represented by amino acids 50 to 1176 of SEQ ID No: 4;
   (g) a cytokinin receptor having the amino acid sequence represented by amino acids 32 to 1036 of SEQ ID No: 6;
   (h) a chimera-type cytokinin receptor comprising an extracellular region of the cytokinin receptor, transmembrane regions of the cytokinin receptor, a histidine kinase region of the cytokinin receptor, and a receiver region of the histidine kinase, wherein said extracellular region, said transmembrane regions and said histidine kinase region are homogeneous to one another and the receiver region is heterogeneous thereto;
   (i) a cytokinin receptor having the amino acid sequence of (a), (b), (c), (e), (f), or (g) with deletion, substitution, or addition of one or a plurality of amino acids; and
   (j) a cytokinin receptor having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequence represented by SEQ ID Nos: 2, 4, or 6;
3. The transformed cell according to claim 1, wherein said cytokinin biosynthesis enzyme is a isopentenyltransferase;
4. The transformed cell according to claim 1, wherein said cytokinin biosynthesis enzyme is selected from the group consisting of:
   (a) a cytokinin biosynthesis enzyme having the amino acid sequence represented by SEQ ID No: 8;
   (b) a cytokinin biosynthesis enzyme having the amino acid sequence represented by SEQ ID No: 10;
   (c) a cytokinin biosynthesis enzyme having the amino acid sequence represented by SEQ ID No: 12;
   (d) a cytokinin biosynthesis enzyme having the amino acid sequence represented by SEQ ID No: 14;
   (e) a cytokinin biosynthesis enzyme having the amino acid sequence represented by SEQ ID No: 16;
   (f) a cytokinin biosynthesis enzyme having the amino acid sequence represented by SEQ ID No: 18;
   (g) a cytokinin biosynthesis enzyme having the amino acid sequence represented by SEQ ID No: 20;
   (h) a cytokinin biosynthesis enzyme having the amino acid sequence represented by SEQ ID Nos: 8, 10, 12, 14, 16, 18, or 20 with deletion, substitution, or addition of one or a plurality of amino acids; and
   (i) a cytokinin biosynthesis enzyme having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequence represented by SEQ ID Nos: 8, 10, 12, 14, 16, 18, or 20;
5. The transformed cell according to claim 1, wherein said cell is yeast; and
6. The transformed cell according to claim 1, wherein said cell is budding yeast.

Further scope of applicability of the present invention will become apparent from the detailed description given hereinafter. However, it should be understood that the detailed description and specific examples, while indicating preferred embodiments of the invention, are given by way of illustration only.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

### DETAILED DESCRIPTION OF THE PREFERRED EMBODIMENTS

Hereinafter, the present invention will be described in detail.

A cytokinin biosynthetic pathway in plants has been considered to include isopentenylation of some kind of adenine structure, because cytokinins contain an isopentenyl group or a hydroxylated isopentenyl group. In recent years, it has been found that the key step of cytokinin biosynthesis in plants is isopentenylation of adenosine-5'-triphosphate and/or adenosine-5'-diphosphate with dimethylallyl diphosphate as isopentenyl donor, more concretely, transfer of an isopentenyl group from dimethylallyl diphosphate to N6 of adenosine-5'-triphosphate and/or adenosine-5'-diphosphate. An enzyme which can catalyze the isopentenylation, more concretely, an enzyme which can catalyze the transfer of the isopentenyl group, such as isopentenyltransferase, is a representative example of cytokinin biosynthesis enzyme to be used in the present invention.

Practical examples of the cytokinin biosynthesis enzymes are a cytokinin biosynthesis enzyme having the amino acid sequence represented by SEQ ID No: 8; a cytokinin biosynthesis enzyme having the amino acid sequence represented by SEQ ID No: 10; a cytokinin biosynthesis enzyme having the amino acid sequence represented by SEQ ID No: 12; a cytokinin biosynthesis enzyme having the amino acid sequence represented by SEQ ID No: 14; a cytokinin biosynthesis enzyme having the amino acid sequence represented by SEQ ID No: 16; a cytokinin biosynthesis enzyme having the amino acid sequence represented by SEQ ID No: 18; a cytokinin biosynthesis enzyme having the amino acid sequence represented by SEQ ID No: 20; a cytokinin biosynthesis enzyme having the amino acid sequence represented by SEQ ID Nos: 8, 10, 12, 14, 16, 18, or 20 with deletion, substitution, or addition of one or a plurality of amino acids; a cytokinin biosynthesis enzyme having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequence represented by SEQ ID Nos: 8, 10, 12, 14, 16, 18, or 20 ; a cytokinin biosynthesis enzyme having an amino acid sequence encoded by a nucleotide sequence of a polynucleotide, wherein said polynucleotide hybridizes under a stringent condition to a polynucleotide that has a nucleotide sequence complementary to the nucleotide sequence represented by SEQ ID Nos: 7, 9, 11, 13, 15, 17, or 19; and the like. Incidentally, the phrase, "a plurality of amino acids", means more particularly about 2 to 20 amino acids and for example, 2 to 10 amino acids and 2 to 5 amino acids may be exemplified. Also, the phrase, "the amino acid sequence ... with deletion, substitution, or addition of one or a plurality of amino acids", means as examples those having the amino acid sequences of which 80% or higher, preferably 90% or higher, and more preferably 95% or higher are identical with the sequences of the amino acids before the deletion, substitution or addition of amino acids (i.e. amino acid sequence identity of 80% or higher, particularly 90% or higher, and more preferably 95% or higher).

Those phrases, "the amino acid sequence ... with deletion, substitution, or addition of one or a plurality of amino acids" or "the amino acid sequences of which 80% or higher ... are identical" include amino acid sequences of the proteins provided by the intracellular processing to which proteins having the amino acid sequences represented by SEQ ID Nos: 8, 10, 12, 14, 16, 18, or 20 are subjected, and the natural variations caused by differences in type of organisms from which the proteins are derived, differences in individual bodies, differences in tissues, and the like.

A cytokinin receptor is a protein having functions of controlling the propagation and differentiation of cells of higher plants based on the intracellular signal transduction mechanism so-called Two-Component regulatory system (or Histidine to Aspartic acid phosphorelay system) while being specifically bonded with cytokinins such as purine type cytokinins, e.g. kinetine, zeatine, and the like, and urea type cytokinins, e.g. N-phenyl-N' - (4-pyridyl) urea and the like. The cytokinin receptor to be used in the present invention belongs to the histidine kinase family and is protein composed of extracellular regions, transmembrane regions, histidine kinase regions (regions having histidine kinase activity in the cell and holding Histidine residue to be an active site), and receiver regions (regions having a reception part for phosphate group transfer and holding Aspartic acid residue to be an active site).

Practical examples of the cytokinin receptor are a cytokinin receptor having the amino acid sequence represented by SEQ ID No: 2; a cytokinin receptor having the amino acid sequence represented by SEQ ID No: 4; a cytokinin receptor having the amino acid sequence represented by SEQ ID No: 6; a cytokinin receptor having the amino acid sequence represented by SEQ ID No: 2 with deletion, substitution, or addition of one or a plurality of amino acids; a cytokinin receptor having the amino acid sequence represented by SEQ ID No: 4 with deletion, substitution, or addition of one or a plurality of amino acids; a cytokinin receptor having the amino acid sequence represented by SEQ ID No: 6 with deletion, substitution, or addition of one or a plurality of amino acids; a cytokinin receptor having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequences represented by SEQ ID No: 2; a cytokinin receptor having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequences represented by SEQ ID No: 4; a cytokinin receptor having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequences represented by SEQ ID No: 6; a cytokinin receptor having an amino acid sequence encoded by a nucleotide sequence of a polynucleotide, wherein said polynucleotide hybridizes under a stringent condition to a polynucleotide that has a nucleotide sequence complementary to the nucleotide sequence represented by SEQ ID Nos: 1, 3, or 5; cytokinin receptors of partially transmembrane region-deleted type which will be described later; chimera-type cytokinin receptors which will be described later; and the like. Incidentally, the phrase, "a plurality of amino acids", means more particularly about 2 to 20 amino acids and for example, 2 to 10 amino acids and 2 to 5 amino acids may be exemplified. Also, the phrase, "the amino acid sequences ... with deletion, substitution, or addition of one or a plurality of amino acids", means as examples those having the amino acid sequences of which 80% or higher, preferably 90% or higher, and more preferably 95% or higher are identical with the sequences of the amino acids before the deletion, substitution or addition of amino acids (i.e. amino acid sequence identity of 80% or higher, particularly 90% or higher, and more preferably 95% or higher).

Those phrases, "the amino acid sequence ... with deletion, substitution or addition of amino acids" or "the amino acid sequences of which 80% or higher ... are identical" include amino acid sequences of the proteins provided by the intracellular processing to which proteins having the amino acid sequences represented by SEQ ID Nos: 2, 4, and 6 are subjected, and the natural variations caused by differences in type of organisms from which the proteins are derived, differences in individual bodies, differences in tissues, and the like.

The phrase, "sequence identity" , in the present invention means the identity and homology between two DNA sequences and between two protein sequences. The sequence identity may be determined by comparing two sequences aligned in the optimum states in a region of the sequences of the comparison objects. The DNA or proteins, the comparison objects, may have addition or deletion (e.g. gap and the like) in the optimum alignment of two sequences. Regarding such sequence identity, computation may be performed using, for example, Vector NTI by producing alignment by utilizing Clustal W algorithm [Nucleic Acid Res., 22 (22): 4673-4680 (1994)]. Incidentally, the sequence identity may be measured by a sequence analysis software, practically Vector NTI, GENETYX-MAC and analysis tools provided in public databases. The aforementioned public databases may be, in general, accessible in, for example, the webpage (http://www.ddbj.nig.ac.jp) of the DNA Data Bank of Japan (the international databank operated within the Center for Information Biology and DNA Data Bank of Japan).

Regarding the phrase, "hybridizes under a stringent condition" , hybridization in this case maybe performed according to a conventional method described in, for example, Molecular Cloning 2nd edition, written by Sambrook J., Frisch E. F., Maniatis T., issued by Cold Spring Harbor Laboratory press. As the "stringent condition", there can be mentioned, for example, the conditions under which a hybrid is formed in a solution of 6 x SSC (a solution containing 1. 5 M NaCl and 0.15 M trisodium citrate is defined as 10 x SSC) at 65°C and then the hybrid is washed with 1 x SSC at a room temperature. The salt concentration in the washing step may be selected, for example, from the condition of 1 x SSC at a room temperature (a low stringent condition) to 0.1 x SSC at a room temperature (a high stringent condition). The temperature in the washing step may be selected, for example, from a room temperature (a low stringent condition) to 68°C (a high stringent condition). Further, both of the salt concentration and the temperature may be changed.

Production of a cell that is transformed with a polynucleotide comprising a nucleotide sequence encoding an amino acid sequence of a cytokinin receptor and a polynucleotide comprising a nucleotide sequence encoding an amino acid sequence of a cytokinin biosynthesis enzyme will be described below.

A cell transformed with a polynucleotide comprising a nucleotide sequence encoding an amino acid sequence of a cytokinin receptor and a polynucleotide comprising a nucleotide sequence encoding an amino acid sequence of a cytokinin biosynthesis enzyme may be obtained by introducing into a host cell, for example, in the following manner, a polynucleotide comprising a nucleotide sequence encoding an amino acid sequence of a cytokinin receptor and a polynucleotide comprising a nucleotide sequence encoding an amino acid sequence of a cytokinin biosynthesis enzyme.

### (1) Preparation of cDNA

At first, the total RNA is prepared from plants such as higher plants according to the method described in, for example, Molecular Cloning 2nd edition written by J., Sambrook, E., F., Frishch, T., Maniastis.

Concretely, for example, after a part of tissues are sampled from a higher plant, the tissues are frozen in liquified nitrogen and successively physically milled using a mortar and pestle or the like. The total RNA may then be obtained by methods in which (a) the resulting milled product is mixed with a solution containing guanidine hydrochloride with phenol or a solution containing SDS with phenol to obtain the total RNA or (b) the resulting milled product is mixed with a solution containing guanidine thiocyanate and further with CsCl and then subjected to centrifugal separation to obtain the total RNA. Examples of the higher plant include a monocotyledonous plant, e.g. rice, corn, barley, wheat and the like and a dicotyledonous plant, e.g. tobaccos, soybean, Arabidopsis, and the like. For the aforementioned process, commercialized kits such as ISOGEN (produced by Nippon Gene Co.), RNeasy Total RNA Purification Kit (produced by QIAGEN Co.), and the like may be employed.

Next, mRNA is prepared from the total RNA. For example, the preparation may be carried out by a method utilizing the hybridization of oligo-dT chains bonded with cellulose or latex and poly(A) chains of mRNA. For the operation, for example, commercialized kits such as mRNA Purification Kit (produced by Amersham Pharmacia Co.), OLIGOTEX^{™} dT30 super (Oligo(dT)latex beads) (produced by Takara Shuzo Co. Ltd.), and the like may be employed.

Further, cDNA is produced using the mRNA (mRNA having poly(A) chains) may be prepared in the following manner. For example, oligo-dT chains or random primers are annealed with mRNA and then reacted with reverse transcriptase to produce cDNA. Further, the cDNA is reacted with, for example, RNaseH, DNA polymerase I to produce double-stranded cDNA. For the operation, for example, the following commercialized kits may be employed: SMART™ PCR cDNA Synthesis Kit (produced by Clontech Co.), cDNA Synthesis Kit (produced by Takara Shuzo Co. Ltd.), cDNA Synthesis Kit (produced by Amersham Pharmacia Co.), ZAP-cDNA Synthesis Kit (produced by Stratagene Co.) and the like.

### (2) Cloning

A polynucleotide comprising a nucleotide sequence encoding an amino acid sequence of a cytokinin receptor may be obtained by a polymerase chain reaction (hereinafter referred as to PCR) from the produced cDNA using, for example, a polynucleotide having partial nucleotide sequence of the nucleotide sequence of SEQ ID Nos: 1, 3 or 5 as a primer or by a hybridization method using a polynucleotide having partial nucleotide sequence of the nucleotide sequence of SEQ ID Nos: 1, 3 or 5 as a probe. In a similar manner, a polynucleotide comprising a nucleotide sequence encoding an amino acid sequence of a cytokinin biosynthesis enzyme may be obtained by a PCR using, for example, a polynucleotide having partial nucleotide sequence of the nucleotide sequence of SEQ ID Nos: 7, 9, 11, 13, 15, 17 or 19 as a primer or by a hybridization method using a polynucleotide having partial nucleotide sequence of the nucleotide sequence of SEQ ID Nos: 7, 9, 11, 13, 15, 17 or 19 as a probe.

In the case of employing PCR, polynucleotides usable as a primer set is those designed and synthesized based on the nucleotide sequences of about 20 bp to 40 bp, for example, the nucleotide sequences selected respectively from 5'-non-coding regions and 3'-non-coding regions of the nucleotide sequence represented by SEQ ID Nos: 1, 3, or 5. Examples of the primer set are sets of the polynucleotide of nucleotide sequence represented by SEQ ID No: 23 and the polynucleotide of nucleotide sequence represented by SEQ ID No: 24. The PCR solution to be used may be prepared by adding reaction solutions instructed by the kit to 250 ng of cDNA. The conditions of the PCR may properly be changed depending on the primer set to be used and, for example, concrete conditions include as follows: keeping at 94°C for 2 minutes, and then at about 8°C for 3 minutes, and further repeating 40 cycles each of which comprises steps of keeping at 94°C for 30 seconds, at 55°C for 30 seconds, and at 72°C for 4 minutes; and repeating 5 to 10 cycles each of which comprises steps of keeping at 94°C for 5 seconds and at 72°C for 4 minutes, and further repeating about 20 to 40 cycles each of which comprises steps of keeping at 94°C for 5 seconds and at 70°C for 4 minutes. For the operation, the following commercialized kits may be, for example, employed: HERCULASE^{™} (DNA polymerase)(produced by Stratagene Co., Ltd.), DNA polymerase contained in Advantage cDNA PCR Kit (Clontech Co.), TAKARA Ex Taq (Takara Shuzo Co., Ltd.), PLATINUM^{™} PCR SUPER (thermostable DNA polymerase in a PCR reaction mix) (Lifetech Oriental Co.), and the like.

In the case of employing hybridization, cloning may be carried out according to a method described in, for example, "Cloning and Sequence", Experimental Manual of Plant Biotechnology (edited by Watanabe and Sugiura, Noson Bunka Publisher, 1989)

The probe to be used may be obtained by preparing polynucleotide (with the chain length of about 200 nucleotides to 500 nucleotides) having partial nucleotide sequences of the nucleotide sequence represented by SEQ ID Nos: 1, 3 or 5 and labelling the polynucleotide with radioisotope markers or fluorescent markers according to the known methods using, for example, Random Primed DNA Labelling Kit (Boehringer Co.), Random Primer DNA Labelling Kit Ver. 2 (Takara Shuzo Co., Ltd.), ECL Direct Nucleic Acid Labelling and Detection System (Amersham Pharmacia Co.), Megaprime DNA-labelling system (Amersham Pharmacia Co.) and the like.

Examples of the hybridization conditions include stringent conditions and the following conditions may be exemplified: keeping at 65°C in the presence of 6 x SSC (0.9 M NaCl and 0.09 M trisodium citrate), 5 x Denhard's solution [0.1% (w/v) ficoll 400, 0.1% (w/v) polyvinylpyrrolidone, 0.1% BSA], 0.5% (w/v) SDS and 100 µg/ml degenerated salmon sperm DNA or in DIG EASY Hyb solution (Boeringer-Mannheim Co.) containing 100 µg/ml of degenerated salmon sperm DNA, successively keeping at a room temperature for 15 minutes two times in the presence of 1 x SSC (0.15 M NaCl and 0.015 M trisodium citrate) and 0.5% (w/v) SDS, and further keeping at 68°C for 30 minutes in the presence of 0.1 x SSC (0.015 MNaCl and 0.0015 M trisodium citrate) and 0.5% SDS.

- In order to obtain a polynucleotide coding the cytokinin receptor of Arabidopsis, PCR can be carried out employing TAKARA LA taq^{™} (thermostable DNA polymerase) (Takara Shuzo Co., Ltd.) and using a solution containing cDNA library phage of Arabidopsis (about 1,000,000 pfu) as a template and a polynucleotide having the nucleotide sequence represented by SEQ ID No: 25 and a polynucleotide having the nucleotide sequence represented by SEQ ID No: 26 as a primer set to amplify and obtain DNA to be a probe. In order to obtain a polynucleotide coding the cytokinin biosynthesis enzyme of Arabidopsis, PCR can be carried out employing TAKARA LA taq^{™} (thermostable DNA polymerase) (Takara Shuzo Co., Ltd.) and using a solution containing cDNA library phage of Arabidopsis (about 1,000,000 pfu) as a template and polynucleotides having partial nucleotide sequence of the nucleotide sequence of SEQ ID Nos: 7, 9, 11, 13, 15, 17 or 19 as a primer set to amplify and obtain DNA to be a probe. The PCR solution to be used may be prepared by adding to 250 ng of cDNA library, the reaction solutions instructed by the kit.

The PCR conditions may be as follows: keeping at 94°C for 2 minutes, and then at 8°C for 3 minutes, and further repeating 40 cycles each of which comprising keeping at 94°C for 30 seconds, at 55°C for 30 seconds, and at 68°C for 5 minutes.

Using the amplified and obtained DNA as a template, a ³²P-labeled probe may be produced employing Megaprime DNA-labelling system kit (Amersham Pharmacia Co.) and using the reaction solutions instructed by the kit. Using the probe obtained in such a manner, colony hybridization is carried out by a conventional method, practically the hybridization is carried out keeping at 65°C in the presence of 6 x SSC (0.9 M NaCl and 0.09 M trisodium citrate), 5 x Denhard's solution [0.1% (w/v) ficoll 400, 0.1% (w/v) polyvinylpyrrolidone, 0.1% BSA], 0.5% (w/v) SDS and 100 µg/ml degenerated salmon sperm DNA or in the DIG EASY Hyb solution (Boeringer-Mannheim Co.) containing 100 µg/ml of degenerated salmon sperm DNA, successively keeping at a room temperature for 15 minutes two times in the presence of 1 x SSC (0.15 M NaCl and 0.015 M trisodium citrate) and 0.5% (w/v) SDS, and further keeping at 68 °C for 30 minutes in the presence of 0.1 x SSC (0.015 M NaCl and 0.0015 M sodium citrate) and 0.5% SDS to obtain clone hybridized with the probe.

Further, a polynucleotide coding the cytokinin receptor may be prepared based on, for example, the nucleotide sequence represented by SEQ ID No: 1, 3 or 5 by chemical synthesis of polynucleotides according to a conventional method such as phosphite-triester method (Hunkapiller, M. et al., Nature, 310, 105, 1984). In a similar manner, a polynucleotide coding the cytokinin biosynthesis enzyme may be prepared based on, for example, the nucleotide sequence represented by SEQ ID No: 7, 9, 11, 13, 15, 17 or 19 by chemical synthesis of polynucleotides.

The polynucleotide comprising a nucleotide sequence encoding an amino acid sequence of the cytokinin receptor and the polynucleotide comprising a nucleotide sequence encoding an amino acid sequence of a cytokinin biosynthesis enzyme, obtained in such a manner, may be cloned in a vector by a conventional method described in, "Molecular Cloning: A Laboratory Manual 2nd edition" (1989), Cold Spring Harbor Laboratory Press; "Current Protocols In Molecular Biology" (1987), John Wiley & Sons, Inc. ISBNO-471-50338-X, and the like. The vector to be used may be, for example, pBlue Script II vector (produced by Stratagene Co.), pUC18/19 vector (produced by Takara Shuzo Co., Ltd.), TA cloning vector (produced by Invitrogen Co.), and the like

Incidentally, the nucleotide sequence of cloned polynucleotide may be confirmed by, the Maxam Gilbert method (described in, for example, Maxam, A., M & W. Gilbert, Proc. Natl. Acad. Sci. USA, 74, 560, 1977, and the like), the Sanger method (described in, for example, Sanger, F. & A.R. Coulson, J. Mol. Biol. , 94, 441, 1975, Sanger, F. &NicklenandA.R. Coulson. , Proc. Natl. Acd. Sci. USA, 74, 5463, 1977, and the like). For the process, the following commercialized kits may be, for example, employed: Thermo Sequenase II dye terminator cycle sequencing kit (produced by Amersham Pharmacia Co.), BigDye Terminator Cycle Sequencing FS Ready Reaction Kit (produced by PE Biosystems Japan Co.), and the like.

### (3) Construction of expression vector

An expression vector of a polynucleotide comprising a nucleotide sequence encoding an amino acid sequence of the cytokinin receptor and an expression vector of a polynucleotide comprising a nucleotide sequence encoding an amino acid sequence of the cytokinin biosynthesis enzyme may be constructed according to a conventional method described in, for example, Molecular Cloning 2nd edition written by J., Sambrook, E., F., Frisch, & T., Maniastis, published by Cold Spring Harbor Laboratory Press.

Usable are vectors to be used in host cells to be transformed, for example, independently replicating vectors which contain genetic information possible to be duplicated in the host cells and further are possible to be isolated from the host cells and purified and may have detectable marker. More practically, in the case of using bacteria such as E. coli as the host cells, for example, Plasmid pUC 119 (produced by Takara Shuzo Co., Ltd.), Phagemid pBluescript II (Stratagene Co.) and the like may be used. In the case of using yeast as the host cells, for example, Plasmid pACT2 (Clontech Co.) and the like may be used. In the case of using plant cells as the host cells, for example, a polynucleotide comprising a nucleotide sequence encoding an amino acid sequence of the cytokinin receptor or a polynucleotide comprising a nucleotide sequence encoding an amino acid sequence of the cytokinin biosynthesis enzyme may be integrated with plasmid pBI221 (Clontech Co.) to construct the vectors.

An expression vector possible to express a polynucleotide comprising a nucleotide sequence encoding an amino acid sequence of the cytokinin receptor in a host cell may be constructed by integrating a promoter with the aforementioned vectors in the upstream of the polynucleotide comprising a nucleotide sequence encoding an amino acid sequence of the cytokinin receptor in a binding manner of enabling to function in the host cell. In a similar manner, an expression vector possible to express a polynucleotide comprising a nucleotide sequence encoding an amino acid sequence of the cytokinin biosynthesis enzyme in a host cell may be constructed by integrating a promoter with the aforementioned vectors in the upstream of the polynucleotide comprising a nucleotide sequence encoding an amino acid sequence of the cytokinin biosynthesis enzyme in a binding manner of enabling to function in the host cell. In these cases, the phrase, "in a binding manner of enabling to function", means that the polynucleotide comprising a nucleotide sequence encoding an amino acid sequence of the cytokinin receptor or the polynucleotide comprising a nucleotide sequence encoding an amino acid sequence of the cytokinin biosynthesis enzyme, and the promoter are bonded as to express the polynucleotide comprising a nucleotide sequence encoding an amino acid sequence of the cytokinin receptor or the polynucleotide comprising a nucleotide sequence encoding an amino acid sequence of the cytokinin biosynthesis enzyme in the host cell under the control of the promoter. Usable as the promoter possible to function in the host cell in the case of using E. coli as the host cell are, for example, a promoter (*lacP*) of lactose operon of E. coli, a promoter *(trpP)* of triptophan operon, a promoter *(argP)* of arginine operon, a promoter *(galP)* of galactose operon, a tac-promoter, T7-promoter, T3-promoter, λ-phage promoter, (λ-pL, λ-pR) and the like. In the case of using yeast as the host cell, it may be prepared by a conventional genetic engineering method [described in Method in Enzymology 101 part (p.192-201) by Ammerer, et. al.] from *ADH1* promoter (the *ADH1* promoter is available from the yeast expression vector pAAH5 which contains the *ADH1* promoter and its terminator and which may be obtained from Washington Research Foundation). The *ADH1* promoter is included in US patent application number 299,733 of Washington Research Foundation and in the case that it is used for industrial and commercial purposes in USA., it is required to obtain permission from the patent holder. In the case of using a plant cell as the host cell, usable examples are a nopaline synthesis enzyme gene (*NOS*) promoter, an octopine synthesis enzyme gene (*OCT*) promoter, a cauliflower mosaic virus (CaMV)-derived 19S promoter, a CaMV-derived 35S promoter and the like.

Further, in the case of integrating a polynucleotide comprising a nucleotide sequence encoding an amino acid sequence of the cytokinin receptor or a polynucleotide comprising a nucleotide sequence encoding an amino acid sequence of the cytokinin biosynthesis enzyme into a vector already having a promoter possible to function in a host cell, the polynucleotide comprising a nucleotide sequence encoding an amino acid sequence of the cytokinin receptor or the polynucleotide comprising a nucleotide sequence encoding an amino acid sequence of the cytokinin biosynthesis enzyme is inserted in the downstream of the promoter and the polynucleotide comprising a nucleotide sequence encoding an amino acid sequence of the cytokinin receptor or the polynucleotide comprising a nucleotide sequence encoding an amino acid sequence of the cytokinin biosynthesis enzyme is inserted in a manner of enabling to function. For example, the aforementioned plasmid PACT 2 for yeast comprises the *ADH1* promoter and therefore, an expression vector possible to express the polynucleotide comprising a nucleotide sequence encoding an amino acid sequence of the cytokinin receptor or the polynucleotide comprising a nucleotide sequence encoding an amino acid sequence of the cytokinin biosynthesis enzyme in yeast, such as CG1945 (Clontech Co.) may be constructed by inserting the polynucleotide comprising a nucleotide sequence encoding an amino acid sequence of the cytokinin receptor or the polynucleotide comprising a nucleotide sequence encoding an amino acid sequence of the cytokinin biosynthesis enzyme in the downstream of the *ADH1* promoter of the plasmid pACT2.

### (4) Production of a transformed cell

A transformed cell to be used in the present invention may be produced by introducing the constructed expression vector into a host cell by a conventional method. As the host cell to be used for production of the transformed cell, examples are bacteria, yeast, plant cell and the like. As bacteria, examples are bacteria belonging to E. coli, Serratia, Bacillus, Brevibacterium, Corynebacterium, Microbacterium and the like. As yeast, examples are budding yeast and fission yeast. More particularly, examples are yeast belonging to Saccharomyces, Schizosaccharomyces and the like. As plant cell, examples are BY-2 strain, which is a cultured cell of tobacco, and BMS strain, which is a cultured cell of corn (Black Mexican Sweet), and the like.

As the method for introducing the expression vector into the aforementioned host cell, conventional introduction methods are employed according to the host cell to be transformed. For example, in the case of using bacteria as the host cell, the aforementioned expression vector may be introduced into a host cell by employing a conventional introduction method such as a calcium chloride method and an electroporationmethod described in, "Molecular Cloning" (by J. Sambrook, et. al Cold Spring Harbor 1989). In the case of using yeast as a host cell, the aforementioned expression vector may be introduced into the host cell by employing Yeast transformation kit (produced by Clontech Co.) based on a lithium method. Further, in the case of using a plant cell as a host cell, the aforementioned expression vector may be introduced into the host cell by a conventional introduction method, for example, Agrobacterium infection method (Japanese Examined Patent Application No. 2 - 58917, Japanese Laid-Open Patent Application No. 60 - 70080), an electroporation method into a protoplast (Japanese Laid-Open Patent Application No. 60 - 251887, Japanese Laid-Open Patent Application No. 5 - 68575), a particle gun method (Japanese Laid-Open Patent Application No. 6 - 508316, Japanese Laid-Open Patent Application No. 63 - 258525).

A transformed cell in which a cytokinin receptor of partially transmembrane region-deleted type, i.e. a cytokinin receptor of a transmembrane quantity variation type is introduced, will be described below.

A cytokinin receptor to be used in the present invention includes a cytokinin receptor wherein said cytokinin receptor has at least one transmembrane region but less than that in its natural form (commonly 2 to 4 transmembrane region) (incidentally, in the present invention, such cytokinin receptors are sometimes referred as to cytokinin receptors of partially transmembrane region-deleted type.) In this case, the phrase, its natural form" means cytokinin receptors having an amino acid sequence most frequently existing among organisms having the similar nomenclature and generally called also as wild-type cytokinin receptor.

Such cytokinin receptors of partially transmembrane regions-deleted type are cytokinin receptors whose transmembrane region structure may be determined by employing structure assumption software available in http: //www.ch.embnet.org/software/TMPRED_form.html and whose transmembrane regions are partially deleted, for example, in 1 to 2 sites, and are less in number than the number of the transmembrane regions of the natural type cytokinin receptors (i.e. natural form).

More particularly, examples of such cytokinin receptors include a cytokinin receptor having the amino acid sequence from amino acid number 196 to 1176 among the amino acid sequence represented by SEQ ID No: 4 (2 transmembrane regions) ; a cytokinin receptor having the amino acid sequence from amino acid number 50 to 1176 among the amino acid sequence represented by SEQ ID No: 4 (3 transmembrane regions); a cytokinin receptor having the amino acid sequence from amino acid number 32 to 1036 among the amino acid sequence represented by SEQ ID No: 6 (2 transmembrane regions); a cytokinin receptor having the amino acid sequence derived from the amino acid sequences of these cytokinin receptors wherein one or a plurality of amino acids are deleted, substituted or added, for example, cytokinin receptors having the amino acid sequence derived from the amino acid sequences in which one methionine is added to the amino-terminal and the like.

The DNA coding the cytokinin receptor may be constructed as to hold transmembrane regions in a less number than the number of the transmembrane regions of the natural type cytokinin receptors by partially deleting the transmembrane regions by a conventional genetic engineering technique.

A transformed cell in which the polynucleotide comprising a nucleotide sequence encoding an amino acid sequence of the partially transmembrane region-deleted type cytokinin receptor and the polynucleotide comprising a nucleotide sequence encoding an amino acid sequence of the cytokinin biosynthesis enzyme are introducedmay be produced according to the aforementionedmethod, "Production of a cell that is transformed with a polynucleotide comprising a nucleotide sequence encoding an amino acid sequence of a cytokinin receptor and a polynucleotide comprising a nucleotide sequence encoding an amino acid sequence of a cytokinin biosynthesis enzyme".

A transformed cell to express chimera-type cytokinin receptor will be described below.

A cytokinin receptor to be used in the present invention also includes a chimera-type cytokinin receptor comprising an extracellular region of the cytokinin receptor, transmembrane regions of the cytokinin receptor, a histidine kinase region of the cytokinin receptor, and a receiver region for a histidine kinase. The chimera-type cytokinin receptor has the extracellular region, the transmembrane regions, and the histidine kinase region homogeneous to one another and the receiver region for the histidine kinase heterogeneous to these regions. For example, the chimera-type cytokinin receptor may have said extracellular region, transmembrane regions, and histidine kinase region derived from a cytokinin receptor selected from CRE1, AHK2, and AHK3, and have the receiver region derived from the osmosensor SLN1 in budding yeast.

The histidine kinase protein has the following sequence in common in plants, e.g. higher plants, and microorganism. That is, histidine kinase protein is composed of an extracellular region, transmembrane region (generally about 2 to 4), histidine kinase region having histidine kinase activity and holding Histidine residue to be an active site, and receiver region having a reception part for a phosphoryl group transfer and holding Aspartic acid residue to be an active site. In the chimera-type cytokinin receptor, it is preferable that the extracellular region, the transmembrane regions, and the histidine kinase region are all derived from the identical type of cytokinin receptor, whereas the receiver regions are derived from a histidine kinase protein that is different from type of the former cytokinin receptor.

It is sufficient for the receiver region of the chimera-type cytokinin receptor to have a function of receiving signals transmitted from the histidine kinase region and transmitting them to the next step and any of receiver region may be usable as long as they can complement or improve the intrinsic functions of the receiver region of a histidine kinase protein comprising the homogeneously derived extracellular region, transmembrane regions, and histidine kinase region.

As such a receiver region, usable are, for example, a receiver region of histidine kinase proteins derived from microorganism (e.g. a receiver region of histidine kinase protein derived from a microorganism such as yeast, E. coli) and more particularly receiver region of histidine kinase protein coded in *SLN1* gene derived from budding yeast (e.g. the amino acid sequence represented by SEQ ID No: 21), receiver region of histidine kinase protein coded in Chey gene derived from Salmonella, receiver region of histidine kinase protein coded in *RcsC* gene, which is a hybrid sensor of E. coli [Maeda T, et al. Nature: 369 242-245, (1994): e.g. the amino acid sequence represented by SEQ ID No: 22], receiver region of histidine kinase protein coded in Phks gene relevant to cell cycle control of fission yeast [Shieh, JC, et al., Gene Dev. 11, 1008-1022 (1997)].

In the above, histidine kinase regions of the cytokinin receptor may include the region present C-terminally downstream from the particular transmembrane region which is the most C-terminally downstream transmembrane region of the cytokinin receptor. Said histidine kinase region typically has five conserved motifs which are common to generic histidine kinases as described in Annual Review of Genetics 23:311-336 (1989), Microbiological Reviews 53(4):450-490(1989), Science 262:539-544(1993), and the like. Examples of the region includes a region having the amino acid sequence from amino acid number 587 to 844 among the amino acid sequence represented by SEQ ID No: 4 in a case of AHK2, a region having the amino acid sequence from amino acid number 450 to 700 among the amino acid sequence represented by SEQ ID No: 6 in a case of AHK3 and a region having the amino acid sequence from amino acid number 449 to 714 among the amino acid sequence represented by SEQ ID No: 2 in a case of CRE1.

Receiver regions of the cytokinin receptor may include the region existing between the histidine kinase region and the C-terminal end of the cytokinin receptor. Said region having three conserved motifs which are common to generic histidine kinases as described in Annual Review of Genetics 23:311-336 (1989), Science 262:539-544(1993), and the like. Examples of the region includes a region having the amino acid sequence from amino acid number 891 to 1163 among the amino acid sequence represented by SEQ ID No: 4 in a case of AHK2, a region having the amino acid sequence from amino acid number 746 to 1018 among the amino acid sequence represented by SEQ ID No: 6 in a case of AHK3 and a region having the amino acid sequence from amino acid number 763 to 1038 among the amino acid sequence represented by SEQ ID No: 2 in a case of CRE1.

In addition, a sensor region for cytokinin means, for example, a region which is a part of any of the extracellular regions of the cytokinin receptor, wherein said sensor region is present between a transmembrane region next to the histidine kinase region and a transmembrane region secondary close to the histidine kinase region. Said sensor region typically has 50% or more identity and homology between three cytokinin receptors of AHK2, AHK3 and CRE1 as described in Plant and Cell Physiology 42(2):231-235(2001) and the like. Examples of the region includes a region having the amino acid sequence from amino acid number 259 to 536 among the amino acid sequence represented by SEQ ID No: 4 in a case of AHK2, a region having the amino acid sequence from amino acid number 120 to 399 among the amino acid sequence represented by SEQ ID No: 6 in a case of AHK3 and a region having the amino acid sequence from amino acid number 132 to 398 among the amino acid sequence represented by SEQ ID No: 2 in a case of CRE1.

A polynucleotide comprising a nucleotide sequence encoding an amino acid sequence of the chimera-type cytokinin receptor may be constructed by respectively producing polynucleotides encoding each of the extracellular region of the cytokinin receptor, transmembrane regions of the cytokinin receptor, histidine kinase region of the cytokinin receptor, and receiver region for the histidine kinase protein, joining the polynucleotides by a conventional genetic engineering technique so as to prevent appearance of any termination codon in the middle while using a proper linker so as to prevent frame shift. Incidentally, one polynucleotide may be utilized to provide the polynucleotide encoding the extracellular region of the cytokinin receptor and transmembrane regions of the cytokinin receptor, or the polynucleotide encoding the extracellular region of the cytokinin receptor, transmembrane regions of the cytokinin receptor, and histidine kinase region of the cytokinin receptor.

The polynucleotides coding amino acid sequences of the aforementioned respective regions may respectively be produced by known methods. For example, in the case of production by PCR, at first, the oligonucleotides (5' side primers) having the nucleotide sequences of 5' terminal regions of respective regions to be amplified and oligonucleotides (3' side primers) having complementary nucleotide sequences to the nucleotide sequences of 3' terminal are designed and synthesized. The primers may be oligonucleotides of about 14 nucleotides to about 35 nucleotides in general and are preferable to contain in the 5' side of the primers, restriction enzyme recognition sequences usable at the time of ligating the polynucleotides amplified by the PCR to one another or these polynucleotides to vectors. Then, using the primers and the cDNA library as a template, amplification reactions may be carried out in the conventional reaction conditions employed for the PCR. As the template to be used in the case of producing the polynucleotide coding amino acid sequence of the extracellular region, the transmembrane regions of the cytokinin receptor or the histidine kinase region of the cytokinin receptor, usable is cDNA library derived from plants such as higher plants. Also as the template to be used in the case of producing the polynucleotides coding amino acid sequence of the receiver regions of the histidine kinase, usable is the total DNA or cDNA library derived from microorganism prepared by a conventional method.

The transformed cell in which the polynucleotide comprising a nucleotide sequence encoding an amino acid sequence of the chimera-type cytokinin receptor and the polynucleotide comprising a nucleotide sequence encoding an amino acid sequence of the cytokinin biosynthesis enzyme are introduced may be produced according to the aforementioned, "Production of a cell that is transformed with a polynucleotide comprising a nucleotide sequence encoding an amino acid sequence of a cytokinin receptor and a polynucleotide comprising a nucleotide sequence encoding an amino acid sequence of a cytokinin biosynthesis enzyme".

Intracellular signal transduction system relevant to cytokinin will be described below.

Measurement of the existence or the quantity of intracellular signal transduction from the cytokinin receptor expressed in the transformed cell produced by the above-described manner may be carried out by utilizing the intracellular signal transduction system originally present in the host cell used for production of the transformed cell. The existence or the quantity of intracellular signal transduction can be determined by employing the quantity of the cell growth of the transformed cell as an indicator. Alternatively, a regulator (and/or a mediator), which is a downstream signal transducer in the so-called Two-Component regulatory system, can be additionally introduced and expressed in the host cell and the expressed system may be used in the intracellular signal transduction system. Usable as the Two-Component regulatory system, for example, are Two-Component regulatory systems corresponding to 5 types of ethylene receptors; ETR1, ETR2, ERS1, ERS2, and EIN4; present in Arabidopsis [Chang et al., Science 262: 539-544 (1993), Hua et al., Science 269: 1712-1714 (1995), Sakai et al. , Plant Cell Physiol 39: 1232-1239 (1998)] and AtHK1 having sensor functions to the osmotic pressure [Urao, Plant ell 11: 1743-1754 (1999)].

As the host cell to be used for production of such a transformed cell, usable are host cells improved as to have histidine kinase activity lower than the intrinsic histidine kinase activity of the host cells. For example, it includes the host cells improved as to have histidine kinase activity lower than the intrinsic histidine kinase activity of the host cell by deleting one or more of histidine kinases. The phrase, "histidine kinase activity lower than" means that the quantity of a phosphoryl group transfer from Histidine residue (the active site of histidine kinase regions having histidine kinase activity) to Aspartic acid residue (the active site of receiver regions having a reception part) is decreased. Such improvements in the host cell may provide a change of the quantity of the cell growth, change of the morphology, change of the shape, change of the quantity of the biosynthesis of specific compound, change of the quantity of the metabolism of specific compound in the transformed cell. More particularly, the following strain [Maeda T et al., Nature 369: 242-245 (1994)] may be exemplified: a strain obtained by defecting the *SLN1* gene coding the protein having the osmotic pressure sensor function and derived from the budding yeast such as Saccharomyces cerevisiae and the like. Since the strain is defected in the histidine kinase protein existing in Saccharomyces cerevisiae, there can be more clearly detected the existence or the quantity of the intracellular signal transduction from the cytokinin receptor expressed in the transformed cell by using the quantity of the cell growth of the transformed cell as an indicator. Further, other preferable example includes an E. coli strain defective in the *RcsC* gene that encodes a hybrid sensor, and a fission yeast strain defective in *Phks* gene that is relevant to the cell cycle.

Method for analyzing the activity of controlling biosynthesis of cytokinin will be described below.

In the method for analyzing the activity of controlling biosynthesis of cytokinin, examples of the first step of bringing an examinee substance into contact with a transformed cell into which a polynucleotide comprising a nucleotide sequence encoding an amino acid sequence of a cytokinin receptor and a polynucleotide comprising a nucleotide sequence encoding an amino acid sequence of a cytokinin biosynthesis enzyme are introduced include a method for culturing the transformed cell in a culture medium containing the examinee substance. In order to culture the transformed cell, the following cultures are usable: liquid-phase culture for culturing the transformed cell in a liquid culture medium and a solid-phase culture for culturing the transformed cell in a solid culture medium produced by adding agar or the like to the liquid culture medium. The concentration of an examinee substance in the culture medium is about 1 nM to about 1 mM and preferably about 10 nM to about 100 µM. The culture time is, for example, 1 hour to 3 days and preferably 25 hours to 2 days.

Following the above-described first step, the existence or the quantity of intracellular signal transduction from the cytokinin receptor expressed in the transformed cell may be measured. Using the measured value as an indicator, the activity of the examinee substance to control biosynthesis of cytokinin may be analyzed.

More particularly, for example, in the case of using a transformed cell of the present invention (that is, a transformed cell, wherein growth of said cell is directly controlled by intracellular signal transduction from the cytokinin receptor) produced using TM182 (*s1n1*Δ) [Maeda T et al, Nature 369: 242-245 (1994)], a *SLN1* genetically defected strain in which *PTP2* Tyrosine phosphatase gene [Ota et al. Proc. N. A. Sci., USA, 89, 2355-2359 (1992)] is introduced, as a host cell, the activity of controlling biosynthesis of cytokinin may be measured using, as an indicator, the quantity of the cell growth of the transformed cell in a culture medium (an agar culture medium or a liquid culture medium) containing glucose as a carbon source, for example, a DOHLU + Glu culture medium. In the case of the DOHLU + Glu culture medium to which the examinee substance is added, the examinee substance found capable of changing growth of the transformed cell can be evaluated as a substance having the activity of controlling biosynthesis of cytokinin, as compared with growth of the transformed cell in the DOHLU + Glu culture medium to which the examinee substance is not added (a culture medium containing no substance having the activity of controlling biosynthesis of cytokinin). Incidentally, as a control to verify that the change of cell growth of the transformed cell is not resulted from activity of the examinee substance directly controlling signal transduction from cytokinin receptor, such as agonist-activity or antagonist-activity to the cytokinin receptor, TM182 *(s1n1* Δ) transformed with only a polynucleotide comprising a nucleotide sequence encoding an amino acid sequence of a cytokinin receptor may be cultured in DOLU + Glu culture medium with or without the examinee substance. In a case where the examinee substance dose not affect growth of the control cell, the examinee substance may not directly affect signal transduction system from cytokinin receptor. As a control to verify that the change of cell growth of the transformed cell is not resulted from activity of the examinee substance which affects somewhere not involved in biosynthesis of cytokinin nor signal transduction from cytokinin receptor, TM182 *(s1n1* Δ) may be cultured in culture medium containing galactose as the carbon source in place of glucose, such as DOLU + Gal culture medium with or without the examinee substance. In a case where the examinee substance dose not affect growth of the control cell [TM182 *(s1n1 Δ*)], the examinee substance may not affect somewhere not involved in biosynthesis of cytokinin nor signal transduction from cytokinin receptor.

Further, in the case of using a transformed cell of the present invention (that is, a transformed cell, wherein growth of said cell is directly controlled by the intracellular signal transduction from the cytokinin receptor) produced by employing fission yeast as a host cell, such as a Phks genetically defected strain, the fission pattern of the fission yeast may be observed with a microscope. In this case, when the examinee substance is added to the culture medium, the examinee substance found capable of changing fission and propagation of the transformed cell can be evaluated as a substance having the activity of controlling biosynthesis of cytokinin, as compared with fission and propagation of the transformed cell in a medium to which the examinee substance is not added.

Furthermore, in the case of using a transformed cell of the present invention produced by employing as the host cell an E. coli defective in RcsC gene into which cps-LacZ is introduced, the X-Gal coloring may be observed in an agar culture medium or a liquid culture medium [Suzuki et al. Plant Cell Physiol 42: 107-113 (2001)]. In this case, when the culture medium contains the examinee substance, the examinee substance found capable of changing coloring of the transformed cell can be evaluated as a substance having the activity of controlling biosynthesis of cytokinin, as compared with coloring of the transformed cell in a medium to which the examinee substance is not added.

The activity of the examinee substance for controlling biosynthesis of cytokinin can be detected by evaluating the activity of two or more examinee substances. The activity of controlling biosynthesis of cytokinin can be provided for the evaluation by conducting the aforementioned method for analyzing the activity of controlling biosynthesis of cytokinin. For example, the evaluation of the activity of controlling biosynthesis of cytokinin can be based on the difference between the two or more examinee substances, which are obtained by comparison of the existence or the quantity of the intracellular signal transduction. In utilizing the two or more different examinee substances, the examinee substances are typically utilized independently in different systems. It is preferable that at least one of the two or more examinee substances has no activity of controlling biosynthesis of cytokinin.

Moreover, a substance with the activity of controlling biosynthesis of cytokinin maybe searched by selecting substances having the activity of controlling biosynthesis of cytokinin based on the activity evaluated by the aforementioned detection methods.

Further, a substance selected by the above described detection methods may be utilized as an active ingredient of a plant growth regulator.

The plants to be the objects to be treated with the aforementioned plant growth regulator are, for example, decorative plants such as flowering plants and ornamental foliage plants; cultivating plants such as crop, vegetable, fruit and the like; fibrous plants; trees; lawn and the like.

The growth regulator is generally mixed with a solid carrier, a liquid carrier, and the like and further, if needed, mixed with a surfactant and other auxiliary agents for the formulation of an agricultural and horticultural agent and formulated in an emulsion agent, a hydrating agent, a suspension agent, a solution agent and the like. In these agricultural and horticultural agents, an agonist-active substance or an antagonist-active substance to the cytokinin receptor may be contained generally in 0.5 to 90 % by weight and preferably in 1 to 80 % by weight.

Usable as the solid carrier to be used for the formulation of an agricultural and horticultural agent are, for example, clays (kaolinite, kieselguhr, synthesized hydrated silicon oxide, intercalated clays, bentonite, acidic white clay, and the like), talc, other inorganic minerals (sericite, quartz powder, sulfur powder, activated carbon, calcium carbonate, and the like), chemical fertilizers (ammonium sulfate, ammonium phosphate, ammonium nitrate, ammonium chloride, urea, and the like) in finely powdered state or in granular state and usable as the liquid carrier are, for example, water, alcohols (methanol, ethanol and the like), ketones (acetone, methyl ethyl ketone, cylohexanone and the like), aromatic hydrocarbons (toluene, xylene, ethylbenzene, methylnaphthalene and the like), non-aromatic hydrocarbons (hexane, cyclohexane, kerosene and the like), esters (ethyl acetate, butyl acetate and the like), nitriles (actonitrile, isobutylnitrile and the like), ethers (dioxane, diisopropyl ether and the like), acid amides (dimethylformamide, dimethylacetamide and the like), halohydrocarbons (dichloroethane, trichloroethane and the like), etc.

As the surfactant, usable are, for example, alkylsulfuric acid esters, alkylsulfonic acid salts, alkylarylsulfonic acid salts, alkylaryl ethers and their polyoxyethylene compounds, polyethylene glycols, polyhydric alcohol esters, sugaralcohols and the like.

As other auxiliary agents for the formulation of agricultural and horticultural agents, usable are solidification agents and dispersanst such as casein, gelatin, polysaccharides (starch, acacia, cellulose derivatives, alginic acid and the like), lignin derivatives, bentonite, synthesized water-soluble polymer [poly(vinyl alcohol), poly(vinyl pyrolidone), poly(acrylic acid) and the like] and the like, and stabilizers such as PAP (acidic isopropyl phosphate), BHT (2,6-tert-butyl-4-methylphenol), BHA (2-/3-tert-butyl-4-methoxyphenol), plant oils, mineral oils, aliphatic acids, aliphatic acid esters and the like.

The substance with the activity of controlling biosynthesis of cytokinin made to be agricultural and horticultural agents is used as it is or diluted with water to carry out treatment for the stem and leave parts, branch and leave parts, and flower and fruit parts of plants by spraying, for fruits by immersion, and for fruits by application. The plant growth regulator is used for the object plants to carry out the treatment once or a plurality of times.

In the case of using the plant growth regulator for the purpose of suppressing the dropping of fruits, the plant growth regulator is diluted with water and the resulting diluted agent is sprayed to the fruit parts and branch and leave parts before harvest.

In the case of using the plant growth regulator for the purpose of suppressing the ball dropping of cotton, the plant growth regulator is diluted with water and the resulting diluted agent is sprayed to the balls and stem and leave parts of cotton before harvest.

The plant growth regulator may be used for the treatment of growing plants or of plants after harvest.

The application amount of the substance with the activity of controlling biosynthesis of cytokinin in an agricultural and horticultural agent is generally 1 to 8000 g per 1 hectare, although it is changed depending on the state of the agricultural and horticultural agent, the timing for the treatment, the method for the treatment, the site for the treatment, and the object plant to be treated. Also in the case of using the plant growth regulator while diluting the agent with water, the concentration of the agent is generally 0.0001 to 1000 mM and preferably 0.001 to 10 mM, although it is changed depending on the state of the agricultural and horticultural agent, the timing for the treatment, the method for the treatment, the site for the treatment, and the object plant to be treated.

Next, hereinafter given are formulation examples of an agricultural and horticultural agent produced from a substance with the activity of controlling biosynthesis of cytokinin and used as a plant growth regulator. The parts in the following description of the examples denotes the parts by weight.

### Formulation example 1

A hydrating agent was obtained by sufficiently pulverizing and mixing 50 parts of a substance with the activity of controlling biosynthesis of cytokinin, 3 parts of calcium ligninsulfonate, 2 parts of sodium laurylsulfate, and 45 parts of synthesized hydrated silicon oxide.

### Formulation example 2

A hydrating agent was obtained by sufficiently pulverising and mixing 70 parts of a substance with the activity of controlling biosynthesis of cytokinin, 3 parts of calcium ligninsulfonate, 2 parts of sodium laurylsulfate, and 25 parts of synthesized hydrated silicon oxide.

### Formulation example 3

An emulsion agent was obtained by mixing 40 parts of a substance with the activity of controlling biosynthesis of cytokinin, 3 parts of polyoxyethylene sorbitane monoolate, 2 parts of CMC (carboxymethyl cellulose), and 52 parts of water and wet-pulverizing the resultant mixture to be 5 µm or smaller in the particle size.

The present invention makes it possible to provide a transformed cell co-expressing a cytokinin receptor and a cytokinin biosynthesis enzyme. Further, by using such a transformed cell, it is possible to provide a method for analyzing the activity of controlling biosynthesis of cytokinins. Furthermore, by using such an analysis method, it is possible to provide a method for quickly finding substances having the activity of controlling biosynthesis of cytokinins with small amounts of samples.

### EXAMPLES

Hereinafter, although the present invention will be described in details with the reference to examples, the present invention is not at all restricted to these examples.

### (Example 1) Preparation of a polynucleotide comprising a nucleotide sequence encoding an amino acid sequence of a cytokinin receptor and preparation of an expression vector of the polynucleotide (Part 1): production of Arabidopsis cDNAphage library for CRE1 cloning

Seeds of Arabidopsis thaliana ecotype Wassilewskija were sterilized with 70% of ethyl alcohol for 1 minute and were further sterilized with 1. 5% of sodium hypochlorite for 10 minutes. The resulting seeds were well washed with sterilized water and then were cultured for 2 weeks in GM culture medium [4.3 g Murashige and Skoog's basal salt mixture, 1% sucrose, 10 ml of 5% MES-KOH (pH 5.7), 0.3% PHYTAGEL^{™} (gellan gum) (SIGMA)] to obtain 5g of the plant. After the plant was frozen in liquified nitrogen and physically milled with a mortar and a pestle. The resulting milled product was mixed with a mixed solution of 10 ml of an extraction buffer [200 mM Tris-HCl (pH 8.5), 100 mM NaCl, 10 mM EDTA, 0.5% SDS, 14 mM β-mercaptoethanol] and 10 g of phenol. After being mixed by a Vortex mixer, the resulting mixture was mixed further with 10 ml of chloroform and vigorously stirred and subjected to centrifugal separation at 10,000 rotation for 20 minutes. The recovered aqueous layer was mixed with LiCl in the concentration to be 2M of the final concentration, left still at -80°C for 3 hours, thawed and subjected to centrifugal separation at 10,000 rotation for 20 minutes to recover a precipitate. The recovered precipitate was dissolved in 2 ml of TE [10 mM Tris-HCl (pH 8.0), 1 mM EDTA] and then further mixed with 0.2 ml of 3 M sodium acetate (pH 5.2) and 5 ml ethanol and subjected to centrifugal separation to recover RNA as a precipitate. Further, the precipitate (RNA) was subjected to treatment with OLIGOTEX™ dT30 super (oligo d(T) latex beads) (Nippon Rosch Co.) to extract RNA integrated with polyA.

The production of phage cDNA library from the extracted RNA integrated with polyA was carried out employing ZAP-cDNAR Synthesis Kit (Stratagene Co.) according to the instruction. The potency of the produced phage cDNA library was 500,000 PFU.

### (Example 2) Preparation of a polynucleotide comprising a nucleotide sequence encoding an amino acid sequence of a cytokinin receptor and preparation of an expression vector of the polynucleotide (Part 1): production of DNA probe of CRE1

The PCR was carried out employing TAKARA LA TAQ™ (thermostable DNA polymerase)(Takara Shuzo Co. , Ltd.) and using a phage solution (about 1,000,000 PFU) of the phage cDNA library produced in the example 1 as a template and a polynucleotide having the nucleotide sequence represented by SEQ ID No: 25 and a polynucleotide having the nucleotide sequence represented by SEQ ID No: 26 as the primers to amplify DNA. The procedure will be described in details below.

A PCR solution was prepared by adding a reaction composition containing dNTP and the like to the phage 1,000,000 pft and respective primer each in 0.2 µM according to the instruction of the kit and the desired DNA fragment was amplified in PCR conditions: keeping at 94°C for 2 minutes, and further repeating 40 cycles each of which comprised keeping at 94°C for 30 seconds, at 55°C for 30 seconds, and at 68°C for 5 minute. Next, using the amplified DNA fragment as a template, a probe labelled with ³²P was prepared employing Megaprime DNA-labelling system kit (Amersham Pharmacia Co.). Incidentally, reaction solution (25 µl) was prepared by adding 2.0 MBq of ³²P dCTP to 25 ng of amplified DNA fragment and adding a reaction composition instructed by the kit. The labelling reaction was carried out at 37°C for 10 minutes.

### (Example 3) Preparation of a polynucleotide comprising a nucleotide sequence encoding an amino acid sequence of a cytokinin receptor and preparation of an expression vector of the polynucleotide (Part 1): production of phage cDNA clone holding CRE1 gene

The cloning of desired CRE1 gene was carried out by plaque hybridization using the probe prepared by the example 2. Detailed description will be given below.

Using the phage cDNA library produced in the example 1 and according to the instruction of ZAP-cDNAR Synthesis Kit, plaque was produced. DNAwas adsorbed on a nitrocellulose filter from the produced plaque and then treated with UV rays to be fixed on the filter. Using the filter prepared in such a manner, hybridized phage cDNA clone was obtained by keeping at 65°C in the presence of 6 x SSC (0. 9 M NaCl and 0.09 M trisodium citrate), 5 x Denhard's solution [0.1% (w/v) ficoll 400, 0.1% (w/v) polyvinylpyrrolidone, 0.1% BSA], 0.5% (w/v) SDS and 100 µg/ml degenerated salmon sperm DNA or in DIG EASY Hyb solution (Boeringer-Mannheim Co.) containing 100 µg/ml of degenerated salmon sperm DNA, successively keeping at a room temperature for 15 minutes two times in the presence of 1 x SSC (0.15 M NaCl and 0.015 M trisodium citrate) and 0.5% (w/v) SDS, and further keeping at 68°C for 30 minutes in the presence of 0.1 x SSC (0.015 M NaCl and 0.0015 M trisodium citrate) and 0.5% SDS.

### (Example 4) Preparation of a polynucleotide comprising a nucleotide sequence encoding an amino acid sequence of a cytokinin receptor and preparation of an expression vector of the polynucleotide (Part 1): cloning of CRE1 cDNA

Using the cDNA of the phage cDNA clone obtained by the example 3 as a template and a polynucleotide having the nucleotide sequence represented by SEQ ID No: 23 and a polynucleotide having the nucleotide sequence represented by SEQ ID No: 24 as primers, DNA having the nucleotide sequence represented by SEQ ID No: 1 was amplified by PCR. Detailed description will be given below.

The PCR was carried out employing Herculase Enhanced DNA Polymerase (Stratagene Co., Ltd.) in the reaction conditions of keeping at 94°C for 1 minute, and further repeating 25 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of cDNA of the phage cDNA clone and respective primer each in 100 ng according to the instruction of the kit.

The desired DNA fragment was amplified in such a manner.

### (Example 5) Preparation of a polynucleotide comprising a nucleotide sequence encoding an amino acid sequence of a cytokinin receptor and preparation of an expression vector of the polynucleotide (Part 1): construction of CRE1 expression vector

After p415CYC1, a yeast expression vector, [Munberg et al. Gene: 156 119-122 (1995), available from ATCC library (No. 873821)] was digested with a restriction enzyme Sma I and then using T4 DNA ligase, DNA having the nucleotide sequence represented by SEQ ID No: 1 and obtained by the example 4 was ligated downstream of the CYC 1 promoter sequence of the expression vector p415CYCl as to be integrated to express the desired protein in yeast. The constructed DNA was confirmed to be in the right direction and its nucleotide sequence was confirmed to be the nucleotide sequence represented by SEQ ID No: 1 by an automatic DNA sequencer and then the expression plasmid p415CYC-CRE 1 was obtained.

### (Example 6) Preparation of a polynucleotide comprising a nucleotide sequence encoding an amino acid sequence of a cytokinin receptor and preparation of an expression vector of the polynucleotide (Part 2): cloning of AHK3 (AAF99730) cDNA

Seeds of Arabidopsis thaliana ecotype Wassilewskija were sterilized with 70% of ethyl alcohol for 1 minute and further sterilized with 1. 5% of sodium hypochlorite for 10 minutes. The resulting seeds were well washed with sterilized water and then cultured for 2 weeks in GM culture medium [4.3 g Murashige and Skoog's basal salt mixture, 1% sucrose, 10 ml of 5% MES-KOH (pH 5.7), 0.3% PHYTAGEL^{™} (gellan gum) (SIGMA)] to obtain 5g of the plant. After the plant was frozen in liquified nitrogen and physically milled with a mortar and a pestle. The resulting milled product was mixed with a mixed solution of 10 ml of an extraction buffer [200 mM Tris-HCl (pH 8.5), 100 mM NaCl, 10 mM EDTA, 0.5% SDS, 14 mM β-mercaptoethanol and 10 g of phenol. After being mixed by a Voltex mixer, the resulting mixture was mixed further with 10 ml of chloroform and vigorously stirred and subjected to centrifugal separation at 10,000 rotation for 20 minutes. The recovered aqueous Layer was mixed with LiCl in the concentration to be 2M of the final concentration, left still at -80°C for 3 hours, thawed and subjected to centrifugal separation at 10,000 rotation for 20 minutes to recover a precipitate. The recovered precipitate was dissolved in 2 ml of TE [10 mM Tris-HCl (pH 8.0), 1 mM EDTA] and then further mixed with 0.2 ml of 3 M sodium acetate (pH 5.2) and 5 ml ethanol and subjected to centrifugal separation to recover RNA as a precipitate. Further, 40 µg of the precipitate (RNA) was mixed with 30 unit of FPLC pure^{™} DnaseI (RNase free-DNase I) (Amersham-Pharmacia) and 60 unit of Superace (Ambion) to remove mixed genome DNA and the resulting RNA was subjected to the phenol/chloroform treatment and ethanol treatment to purify the RNA. Next, using the purified RNA as a template and oligo (dT) 12-18 (Amersham-Pharmacia) as a primer, RT-PCR was carried out. The RT-PCR was carried out employing Superscript II (GIBCO BRL Co.) at 42°C for 40 minutes. Incidentally the PT-PCR solution was prepared according to the method described in instruction of the Superscript II.

The desired cDNA was amplified in such a manner.

Using the amplified cDNA as a template and a polynucleotide having the nucleotide sequence represented by SEQ ID No: 27 and a polynucleotide having the nucleotide sequence represented by SEQ ID No: 28 as primers, DNA having the nucleotide sequence represented by SEQ ID No: 5 was amplified by PCR. The PCR was carried out using Herculase Enhanced DNA Polymerase (Stratagene Co., Ltd.) in the reaction conditions of keeping at 94°C for 1 minute, and further repeating 41 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of the template DNA and respective primer each in 100 ng according to the instruction of the kit.

The desired DNA fragment was amplified in such a manner.

### (Example 7) Preparation of a polynucleotide comprising a nucleotide sequence encoding an amino acid sequence of a cytokinin receptor and preparation of an expression vector of the polynucleotide (Part 2): construction of pHM-1

After p415CYC1 [Munberg et al. Gene: 156 119-122 (1995); available from ATCC library (No. 87382)] was digested with the restriction enzyme Spe I and BamH I, synthesized DNA fragments (linkers) having the nucleotide sequence represented by SEQ ID Nos: 29 and 30 were inserted to the expression vector p415CYC1 using T4 DNA ligaseas to newly add the restriction enzyme sites Sac II, Apa I, Nhe I to the plasmid p415CYC1 and construct pHM-1

### (Example 8) Preparation of a polynucleotide comprising a nucleotide sequence encoding an amino acid sequence of a cytokinin receptor and preparation of an expression vector of the polynucleotide (Part 2): construction of AHK3 expression vector

After the pHM-1 was digested with the restriction enzyme Sal I and Sac II, and then using T4 DNA ligase, DNA having the nucleotide sequence represented by SEQ ID No: 5 was introduced downstream of the CYC1 promoter sequence of the expression vector pHM-1 as to be integrated to express the desired protein in yeast. The constructed DNA was confirmed to be right in the direction and its nucleotide sequence was confirmed to be right in the sequence by an automatic DNA sequencer and thus the expression plasmid p415CYC-AHK3 was obtained.

### (Example 9) Preparation of a polynucleotide comprising a nucleotide sequence encoding an amino acid sequence of a cytokinin receptor and preparation of an expression vector of the polynucleotide (Part 2): cloning of AHK2 (BAB09274) cDNA

Using the cDNA prepared in the example 6 (the cDNA prepared by reverse transcription of the total RNA) as a template and a polynucleotide having the nucleotide sequence represented by SEQ ID No: 31 and a polynucleotide having the nucleotide sequence represented by SEQ ID No: 32 as primers, DNA having the nucleotide sequence represented by SEQ ID No: 3 was amplified by PCR. The PCR was carried out employing TAKARA Pfu Turbo denature (Takara Shuzo Co., Ltd.) in the reaction conditions of keeping at 94°C for 1 minute, and further repeating 30 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of the template DNA and respective primer each in 50 ng according to the instruction of the kit. The desired DNA fragment was amplified in such a manner.

### (Example 10) Preparation of a polynucleotide comprising a nucleotide sequence encoding an amino acid sequence of a cytokinin receptor and preparation of an expression vector of the polynucleotide (Part 2): cloning of AHK2 (BAB09274) ΔcDNA

Using the cDNA prepared in example 6 (the cDNA prepared by reverse transcription of the total RNA) as a template and a polynucleotide having the nucleotide sequence represented by SEQ ID No: 33 and a polynucleotide having the nucleotide sequence represented by SEQ ID No: 34 as primers, PCR was carried out to amplify DNA containing the nucleotide sequence represented by nucleotide numbers 586 to 3531 of SEQ ID No: 3 in which ATG was added to the 5' terminal sites of said nucleotide sequence. The PCR was carried out employing TAKARA Pfu Turbo denature (Takara Shuzo Co. , Ltd.) in the reaction conditions of keeping at 94°C for 1 minute, and further repeating 30 cycles each of which comprised keeping at 94°C for 30 seconds, at 55°C for 30 seconds, and at 72°C for 4 minute. Incidentally, the PCR solution (50 µl) was prepared by adding a reaction composition containing dNTP and the like to 500 ng of the template DNA and respective primer each in 50 ng according to the instruction of the kit. The desired DNA fragment was amplified in such a manner.

### (Example 11) Preparation of a polynucleotide comprising a nucleotide sequence encoding an amino acid sequence of a cytokinin receptor and preparation of an expression vector of the polynucleotide (Part 2): construction of AHK2 and AHK2 Δ expression vectors

After pHM-1 was digested with a restriction enzyme Sac II and Nhe I and then using T4 DNA ligase, DNA fragments obtained by the example 9 and the example 10 were respectively ligated downstream to the CYC 1 promoter sequence of the expression vector pHM-1 as to be integrated to express the desired protein in yeast. The introduced DNA was confirmed to be right in the direction and its nucleotide sequence was confirmed to be right in the sequence by an automatic DNA sequencer and thus the expression plasmid p415CYC-AHK 2 and p415CYC-AHK2Δ were obtained.

### (Reference example 1) Production of transformed cells TM182-CRE1, TM182-AHK2, TM182-AHK2Δ and TM182-AHK3

Transformation of TM182 *(s1n1Δ*), *SLN1* genetically defected strain, [Maeda T et al. Nature: 369 242-245 (1994)] was carried out using the obtained expression plasmid p415CYC-CRE1 (the example 5), p415CYC-AHK2 (the example 11), p415CYC-AHK2Δ (the example 11) and p415CYC-AHK3 (the example 8). The transformation was carried out by employing Polyethylene glycol/lithium acetate (PEG/LiAc)-mediated transformation method according to the description of VII. Library Transformation & Screening Protocols disclosed in MATCHAMAKER Two-Hybrid System 3 User Manual p. 22 from CLONTECH Co. Transformed cells TM182-CRE1, TM182-AHK2, TM182-AHK2A and TM182-AHK3 were produced by selecting transformed cells that were grown in the DOLU + Gal culture medium based on the fact that leucine auxotrophy disappears in the transformed cells.

### (Reference example 2) Response of transformed cells TM182-CRE1, TM182-AHK2, TM182-AHK2Δ and TM182-AHK3 to cytokinin (Part 1)

A culture solution 10 µl (about 800 clones of yeast) of the transformed cells TM182-CRE1, TM182-AHK2, TM182-AHK2Δ and TM182-AHK3 produced by the example 12 was spotted on DOLU + Glu agar media containing 10 µM of trans-zeatin and cultured at 30°C for 30 hours. After incubation, the growth state of the transformed cells was observed and photographed by a digital camera.

As a result, each of the transformed cells TM182-CRE1, TM182-AHK2, TM182-AHK2Δ and TM182-AHK3 showed high growth in the case of using the DOLU + Glu agar culture media containing trans-zeatin as compared with that in the case of using the DOLU + Glu agar culture media containing no trans-zeatin. The results showed that the transformed cells responded to cytokinin and that they were possible to be grown in the DOLU + Glu agar culture media. Further the transformed cells were found possible to be grown in the DOLU + Gal agar culture media independently of the existence trans-zeatin.

### (Reference example 3) Response of transformed cells TM182-CRE1, TM182-AHK2Δ and TM182-AHK2 to cytokinin (Part 2)

Culture solutions of the transformed cells TM182-CRE1, TM182-AHK2Δ and TM182-AHK2 produced by the reference example 1 were spotted on DOLU + Glu agar media containing cytokinin in a variety of concentrations (1 nM, 10 nM, 100 nM, 1 µM, 10 µM, 100 µM) and cultured at 30°C for 30 hours. After incubation, the growth state of the transformed cells was observed and photographed by a digital camera. The lowest observed supply concentrations of trans-zeatin and cis-zeatin in which the respective transformed cells could be grown were shown in Table 1.

**Table 1**

| cytokinin | TM182-CRE1 | TM182-AHK2 | TM182-AHK2Δ |
|---|---|---|---|
| trans-zeatin | 10µM | 1µM | 100nM |
| cis-zeatin | no growth | 10µM | 1µM |

### (Reference example 4) Method for searching substance having agonist-activity to cytokinin receptor (Part 1)

The transformed cells TM182-AHK2, TM182-AHK2Δ and TM182-AHK3 produced in the reference example 1 were inoculated in 200 ml of DOLU + Gal culture media and pre-cultured at 30°C for 36 hours. The pre-cultured solutions were diluted with DOLU + Glu media as to become 0.100 as the optical density (OD₆₀₀) and further the resultants were diluted with DOLU + Glu media at a dilution rate of 1/200 to obtain diluted pre-cultured solutions.

An assay plate was prepared by filling respective wells of a 96-well plate with 20 µl of solutions which were prepared by diluting DMSO solution (10mM) of each examinee substance with DOLU + Glu medium at a dilution rate of 1/100, said solutions containing each examinee substance to be 100 µM at the final concentration. Simultaneously, an assay plate only filled with 20 µl of solutions which were prepared by diluting DMSO solution with DOLU + Glu medium, said solutions containing no examinee substance, was prepared as the blank sections.

The diluted pre-cultured solutions were added in 200 µl each into the respective wells of both assay plates and cultured at 30°C for 24 hours and then the optical density (OD₆₂₀) of each well was measured by a plate reader. The agonist-activity of the examinee substances to the cytokinin receptor was detected by comparing the optical density measured in the testing sections to which the examinee substances were added with the optical density measured in the blank sections. The optical density of the culture solutions of the transformed cells in the testing sections to which the examinee substances were added were shown in Table 2. Compound B showing the higher optical density measured in the testing sections to which the examinee substances were added than that in the blank sections were selected as agonist-active substance to the cytokinin receptor.

**Table 2**

| Examinee substance | TM182-AHK2 | TM182-AHK2Δ | TM182-AHK3 |
|---|---|---|---|
| DMSO | 0.01 | 0 | 0.51 |
| Compound A^{*1} | 0.01 | 0 | 0.54 |
| Compound B^{*2} | 0.45 | 0.89 | 0.88 |

| | | | |
|---|---|---|---|
| ^{*1} Abscisic acid (=negative control) ^{*2} 6-Benzyl aminopurine (=positive control) | | | |

### (Reference example 5) Method for searching substance having agonist-activity to cytokinin receptor (Part 2)

The transformed cells TM182-AHK2, TM182-AHK2Δ and TM182-AHK3 produced in the reference example 1 are inoculated in 200 ml of DOLU + Gal culture media and cultured at 30°C for 30 hours to obtain pre-cultured solutions.

The pre-cultured transformed cells (TM182-AHK2, TM182-AHK2A and TM182-AHK3) are spot-added in 10 µl to the respective DOLU + Glu agar culture media to which agonist-active substances to the cytokinin receptor selected by the reference example 4 are added while their concentration being changed from 10 nM to 100 µM and then are cultured at 30°C for 30 hours. After incubation, the intensity of the agonist-activity of the examinee substances to the cytokinin receptor is detected and confirmed based on the lowest concentration at which transformed cells (TM182-AHK2, TM182-AHK2A and TM182-AHK3) are observed to grow.

### (Example 12) Preparation of a polynucleotide comprising a nucleotide sequence encoding an amino acid sequence of a cytokinin biosynthesis enzyme and preparation of an expression vector of the polynucleotide: Cloning of AtIPT4 cDNA)

Seeds of Arabidopsis thaliana ecotype Wassilewskija were sterilized with 70% ethyl alcohol for 1 minute, and were further sterilized with 1.5% sodium hypochlorite for 10 minutes. These seeds were well washed with sterilized water, and then were cultured for 20 days in GM culture medium (4.3 g Murashige and Skoog's basal salt mixture, 1% sucrose, 10 mL of 5% MES-KOH (pH 5.7), 0.3% PHYTAGEL™ (gellan gum) (SIGMA)) to obtain 5 g of plants. The plants were frozen in liquified nitrogen, and were then physically milled in a 1.5 mL tube. To the milled product, 100 µL of 2xCTAB (2%(w/v) Cetyl trimethyl ammonium bromide (hereinafter, referred to as "CTAB") , 0.1M Tris-HCl (pH 8.0), 1.4M NaCl, 1% (w/v) PVP) was added. The resulting mixture was left standing at 60°C for 30 minutes, and then 100 µL of a mixed solution of chloroform and isoamyl alcohol (chloroform: isoamyl alcohol = 24: 1) was added. The resulting mixture was stirred with a Voltex mixer, and was then subjected to centrifugal separation at 15,000 rpm at 20°C for 20 minutes. After centrifugal separation, 75 µL of the resulting supernatant was placed in a 0.6 mL tube, in which 75 µL of isoamyl alcohol had been already placed, and the resulting mixture was left standing at 25°C for 20 minutes. Thereafter, the mixture was subjected to centrifugal separation at 15,000 rpm at 20°C for 20 minutes to recover a precipitate. To the recovered precipitate, 300 µL of 70% ethanol was added, and the resulting mixture was stirred with a Voltex mixer. The mixture was subjected to centrifugal separation at 15,000 rpm at 4°C for 15 minutes to recover a precipitate. The recovered precipitate was left standing at 37°C for 5 minutes, and then 30 µL of T10E0.2 (10mM Tris-HCl (pH 8.0), 0.2mM EDTA (pH 8.0)) was added to dissolve the precipitate. In this way, a genomic DNA solution was obtained. Next, PCR was carried out using the obtained genomic DNA as a template and a polynucleotide having the nucleotide sequence represented by SEQ ID No: 35 and a polynucleotide having the nucleotide sequence represented by SEQ ID No: 36 as primers to amplify DNA having the nucleotide sequence represented by SEQ ID No: 11. The PCR was carried out employing Herculase Enhanced DNA Polymerase (Stratagene Co., Ltd.) under the amplifying conditions of keeping at 94°C for 1 minute, repeating 40 cycles each including keeping at 94°C for 30 seconds, at 50°C for 30 seconds, and at 72°C for 60 seconds, and final keeping at 72°C for 1 minute. A PCR solution (50 µL) was prepared by adding a reaction composition such as dNTP and the like to 100 ng of the template DNA and 100 ng of each of the primers according to the instruction of the kit. In this way, a desired DNA fragment was amplified.

### (Example 13) Preparation of a polynucleotide comprising a nucleotide sequence encoding an amino acid sequence of a cytokinin biosynthesis enzyme and preparation of an expression vector of the polynucleotide: construction of AtIPT4 expression vector

After p423ADH, a yeast expression vector, (Munberg et al. Gene: 156 119-122 (1995), available from ATCC library (No. 87371) was digested with a restriction enzyme Sma I and then using T4 DNA ligase, DNA having the nucleotide sequence represented by SEQ ID No: 11 and obtained by the example 12 was ligated downstream of the ADH promoter sequence of the expression vector p423ADH as to be integrated to express the desired protein in yeast. The constructed DNA was confirmed to be in the right direction and its nucleotide sequence was confirmed to be the nucleotide sequence represented by SEQ ID No: 11 by an automatic DNA sequencer and then the expression plasmid p423ADH-IPT4 was obtained.

In a similar manner, after p423CYC1, a yeast expression vector, (Munberg et al. Gene: 156 119-122 (1995), available from ATCC library (No. 87379) was digested with a restriction enzyme Sma I and then using T4 DNA ligase, DNA having the nucleotide sequence represented by SEQ ID No: 11 and obtained by the example 12 was ligated downstream of the CYC 1 promoter sequence of the expression vector p423CYC1 as to be integrated to express the desired protein in yeast. The constructed DNA was confirmed to be in the right direction and its nucleotide sequence was confirmed to be the nucleotide sequence represented by SEQ ID No: 11 by an automatic DNA sequencer and then the expression plasmid p423CYC1-IPT4 was obtained.

### (Example 14) Preparation of a polynucleotide comprising a nucleotide sequence encoding an amino acid sequence of a cytokinin biosynthesis enzyme and preparation of an expression vector of the polynucleotide: cloning of AtIPT5 cDNA

In a similar manner to Example 12, PCR was carried out by using the genomic DNA obtained from the plants of Arabidopsis thaliana ecotype Wassilewskija as a template and a polynucleotide having a nucleotide sequence represented by SEQ ID No: 37 and a polynucleotide having a nucleotide sequence represented by SEQ ID No: 38 as primers to amplify DNA having a nucleotide sequence represented by SEQ ID No:13. The PCR was carried out employing Herculase Enhanced DNA Polymerase (Stratagene Co., Ltd.) under the amplifying conditions of keeping at 94°C for 1 minute, repeating 35 cycles each including keeping at 94°C for 30 seconds, at 55°C for 30 seconds, and at 72°C for 90 seconds, and final keeping at 72°C for 1 minute. A PCR solution (50 µL) was prepared by adding a reaction composition such as dNTP and the like to 100 ng of the template DNA and 100 ng of each of the primers according to the introduction of the kit. In this way, a desired DNA fragment was amplified.

### (Example 16) Production of transformed cells of the present invention: Production of transformed cells TM182-CYCl-CYC1IPT4, TM182-CYC1-ADHIPT4, TM182-CYC1-CYC1IPT5, TM182-CYC1-ADHIPT5, TM182-CRE1-CYC1IPT4, TM182-CRE1-ADHIPT4, TM182-CRE1-CYC1IPT5, TM182-CRE1-ADHIPT5, TM182-AHK2-CYC1IPT4, TM182-AHK2-ADHIPT4, TM182-AHK2-CYC1IPT5, and TM182-AHK2-ADHIPT5

Transformation of TM182 (S1n1Δ), *SLN1* genetically defected strain, [Maeda T et al. Nature: 369 242-245 (1994)] was carried out by using any one of the three expression plasmids, commercially available p415CYC1, p415CYC-CRE1 obtained in Example 5, and p415CYC-AHK2 obtained in Example 11, and any one of the four expression plasmids, p423ADH-IPT4 and p423CYC1-IPT4 obtained in Example 13, and p423ADH-IPT5 and p423CYC1-IPT5 obtained in Example 15 together (that is, by using two expression plasmids at the same time). The transformation was carried out by employing Polyethylene glycol/lithium acetate (PEG/LiAc)-mediated transformation method according to the description of VII. Library Transformation & Screening Protocols disclosed in MATCHAMAKER Two-Hybrid System 3 User Manual p. 22 from CLONTECH Co. Twelve kinds of transformed cells TM182-CYC1-CYC1IPT4 to which p415CYC1 and p423CYC1-IPT4 are introduced, TM182-CYC1-ADHIPT4 to which p415CYC1 and p423ADH-IPT4 are introduced, TM182-CYC1-CYC1IPT5 to which p415CYC1 and p423CYC1-IPT5 are introduced, TM182-CYC1-ADHIPT5 to which p415CYC1 and p423ADH-IPT5 are introduced, TM182-CRE1-CYC1IPT4 to which p415CYC-CRE1 and p423CYC1-IPT4 are introduced, TM182-CRE1-ADHIPT4 to which p415CYC-CRE1 and p423ADH-IPT4 are introduced, TM182-CRE1-CYC1IPT5 to which p415CYC-CRE1 and p423CYC1-IPT5 are introduced, TM182-CRE1-ADHIPT5 to which p415CYC-CRE1 and p423ADH-IPT5 are introduced, TM182-AHK2-CYC1IPT4 to which p415CYC-AHK2 and p423CYC1-IPT4 are introduced, TM182-AHK2-ADHIPT4 to which p415CYC-AHK2 and p423ADH-IPT4 are introduced, TM182-AHK2-CYC1IPT5 to which p415CYC-AHK2 and p423CYC1-IPT5 are introduced, and TM182-AHK2-ADHIPT5 to which p415CYC-AHK2 and p423ADH-IPT5 are introduced were produced by selecting transformed cells that were grown in DOHLU+Gal culture medium based on the fact that leucine auxotrophy and histidine auxotrophy disappear in the transformed cells.

### (Example 17) Production of transformed cells of the present invention: production of transformed cells TM182-AHK2Δ-CYC1IPT4, TM182-AHK2Δ-ADHIPT4, TM182-AHK2Δ-CYC1IPT5, TM182-AHK2Δ-ADHIPT5, TM182-AHK3-CYC1IPT4, TM182-AHK3-ADHIPT4, TM182-AHK3-CYC1IPT5, and TM182-AHK3-ADHIPT5

Transformation of TM182 (S1n1Δ), *SLN1* genetically defected strain, [Maeda T et al. Nature: 369 242-245 (1994)] is carried out by using any one of the two expression plasmids, p415CYC-AHK2Δ obtained in Example 11 and p415CYC-AHK3 obtained in Example 8, and any one of the four expression plasmids, p423ADH-IPT4 and p423CYC1-IPT4 obtained in Example 13, and p423ADH-IPT5 and p423CYC1-IPT5 obtained in Example 15 together (that is, by using two expression plasmids at the same time). The transformation is carried out by employing Polyethylene glycol/lithium acetate (PEG/LiAc)-mediated transformation method according to the description of VII. Library Transformation & Screening Protocols disclosed in MATCHAMAKER Two-Hybrid System 3 User Manual p. 22 from CLONTECH Co. Eight kinds of transformed cells TM182-AHK2Δ-CYC1IPT4 to which p415CYC-AHK2Δ and p423CYC1-IPT4 are introduced, TM182-AHK2Δ-ADHIPT4 to which p415CYC-AHK2Δ and p423ADH-IPT4 are introduced, TM182-AHK2Δ-CYClIPT5 to which p415CYC-AHK2Δ and p423CYC1-IPT5 are introduced, TM182-AHK2Δ-ADHIPT5 to which p415CYC-AHK2Δ and p423ADH-IPT5 are introduced, TM182-AHK3-CYC1IPT4 to which p415CYC-AHK3 and p423CYC1-IPT4 are introduced, TM182-AHK3-ADHIPT4 to which p415CYC-AHK3 and p423ADH-IPT4 are introduced, TM182-AHK3-CYC1IPT5 to which p415CYC-AHK3 and p423CYC1-IPT5 are introduced, and TM182-AHK3-ADHIPT5 to which p415CYC-AHK3 and p423ADH-IPT5 are introduced are produced by selecting transformed cells that are grown in DOHLU+Gal culture medium based on the fact that leucine auxotrophy and histidine auxotrophy disappear in the transformed cells.

### (Example 18) Use of transformed cells of the present invention: culture of transformed cells TM182-CYC1-CYC1IPT4, TM182-CYC1-ADHIPT4, TM182-CYC1-CYC1IPT5, TM182-CYC1-ADHIPT5, TM182-CRE1-CYC1IPT4, TM182-CRE1-ADHIPT4, TM182-AHK2-CYC1IPT4, TM182-AHK2-ADHIPT4, TM182-AHK2-CYC1IPT5, and TM182-AHK2-ADHIPT5

10 µL of the culture solution (containing about 800 clones of yeast) of each of the transformed cells TM182-CYC1-CYC1IPT4, TM182-CYC1-ADHIPT4, TM182-CYC1-CYC1IPT5, TM182-CYC1-ADHIPT5, TM182-CRE1-CYC1IPT4, TM182-CRE1-ADHIPT4, TM182-AHK2-CYC1IPT4, TM182-AHK2-ADHIPT4, TM182-AHK2-CYC1IPT5, and TM182-AHK2-ADHIPT5 produced in Example 16 was spotted on DOHLU+Glu agar culture medium, and was cultured at 30°C for 30 hours. After incubation, the growth state of the transformed cells was observed and photographed by a digital camera.

As a result, the transformed cells TM182-CYC1-CYC1IPT4, TM182-CYCl-ADHIPT4, TM182-CYC1-CYC1IPT5, and TM182-CYCl-ADHIPTS were not observed to grow, but the transformed cells TM182-CRE1-CYC1IPT4, TM182-CRE1-ADHIPT4, TM182-AHK2-CYC1IPT4, TM182-AHK2-ADHIPT4, TM182-AHK2-CYC1IPT5, and TM182-AHK2-ADHIPT5 were observed to grow. Further, in a case where DOHLU+Glu agar culture medium containing 10 µM trans-zeatin was used, the transformed cells TM182-CYC1-CYC1IPT4, TM182-CYC1-ADHIPT4, TM182-CYC1-CYC1IPT5, and TM182-CYC1-ADHIPT5 were not observed to grow, but the transformed cells TM182-CRE1-CYC1IPT4, TM182-CRE1-ADHIPT4, TM182-AHK2-CYC1IPT4, TM182-AHK2-ADHIPT4, TM182-AHK2-CYC1IPT5, and TM182-AHK2-ADHIPT5 were observed to grow. This indicates that the transformed cells TM182-CRE1-CYC1IPT4, TM182-CRE1-ADHIPT4, TM182-AHK2-CYC1IPT4, TM182-AHK2-ADHIPT4, TM182-AHK2-CYC1IPT5, and TM182-AHK2-ADHIPT5 respond to cytokinins synthesized by themselves and can grow on DOHLU+Glu agar culture medium. It was also confirmed that all of the ten kinds of transformed cells could grow in DOHLU+Gal culture medium regardless of the presence or absence of trans-zeatin.

### (Example 19) Use of transformed cells of the present invention: culture of transformed cells TM182-CYC1-CYC1IPT4, TM182-CYC1-ADHIPT4, TM182-CYC1-CYC1IPT5, TM182-CYC1-ADHIPT5, TM182-CRE1-CYC1IPT4, and TM182-CRE1-ADHIPT5

Each of the transformed cells TM182-CYC1-CYC1IPT4, TM182-CYC1-ADHIPT4, TM182-CYC1-CYC1IPT5, TM182-CYC1-ADHIPT5, TM182-CRE1-CYC1IPT4, and TM182-CRE1-ADHIPT5 obtained in Example 16 was inoculated in 200 mL of DOHLU+Gal culture medium, and was pre-cultured at 30°C for 36 hours. The thus obtained culture solutions were diluted with DOHLU+Glu culture medium so that OD₆₀₀ became 0.200, and then the diluted ones were further diluted with DOHLU+Glu culture medium at a dilution rate of 1/200 to obtain diluted pre-cultured solutions.

A 96-well assay plate was prepared by filling each well with 1 µL of a DMSO solution of an examinee substance (Abscisic acid or 6-Benzyl aminopurine). In this regard, it is to be noted that the DMSO solution of a examinee substance was prepared so that the concentration thereof became 200 ppm. Simultaneously, an assay plate was prepared by filling each well with 1 µL of DMSO as a blank section.

Each of the diluted pre-cultured solutions was added in a volume of 100 µL per each well of both of the assay plates, and was cultured at 30°C for 24 hours. After incubation, the turbidity of each well was measured using a plate reader. The absorbance (OD₆₀₀) of each of the culture solutions of the transformed cells containing the examinee substance was shown in Table 3. The transformed cells TM182-CYC1-CYC1IPT4, TM182-CYC1-ADHIPT4, TM182-CYC1-CYC1IPT5, and TM182-CYC1-ADHIPT5 were not observed to grow whichever examinee substance was added. On the other hand, the transformed cells TM182-CRE1-CYC1IPT4 and TM182-CRE1-ADHIPT5 were observed to grow whichever examinee substance was added. Further, the growth speeds of the transformed cells TM182-CRE1-CYC1IPT4 and TM182-CRE1-ADHIPT5 in the culture medium to which 6-Benzyl aminopurine was added were faster than those in the culture medium to which only DMSO was added. This indicates that the transformed cells TM182-CRE1-CYC1IPT4 and TM182-CRE1-ADHIPT5 respond to cytokinins synthesized by the cells and can grow in DOHLU+Glu culture medium and that the growth speeds of the transformed cells TM182-CRE1-CYC1IPT4 and TM182-CRE1-ADHIPT5 are increased or decreased depending on the amount of cytokinin.

**Table 3**

| Examinee substance | TM182-CYC1-CYC1-IPT4 | TM182-CYC1-ADH-IPT4 | TM182-CYC1-CYC1-IPT5 | TM182-CYC1-ADH-IPT5 | TM182-CRE1-CYC1-IPT4 | TM182-CRE1-ADH-IPT5 |
|---|---|---|---|---|---|---|
| DMSO | 0.042 | 0.040 | 0.041 | 0.043 | 0.106 | 0.129 |
| Abscisic acid | 0.042 | 0.040 | 0.041 | 0.043 | 0.102 | 0.131 |
| 6-Benzyl aminopurine | 0.042 | 0.041 | 0.043 | 0.043 | 0.260 | 0.293 |

### (Example 20) Use of transformed cells of the present invention: culture of transformed cells TM182-CYC1-CYC1IPT4, TM182-CYC1-ADHIPT4, TM182-CYC1-CYC1IPT5, TM182-CYC1-ADHIPT5, TM182-CRE1-ADHIPT4, TM182-AHK2-CYC1IPT4, TM182-AHK2-CYC1IPT5 and TM182-AHK2-ADHIPT5

Each of the transformed cells TM182-CYC1-CYC1IPT4, TM182-CYC1-ADHIPT4, TM182-CYC1-CYC1IPT5, TM182-CYC1-ADHIPT5, TM182-CRE1-ADHIPT4, TM182-AHK2-CYC1IPT4, TM182-AHK2-CYC1IPT5 and TM182-AHK2-ADHIPT5 obtained in Example 16 was inoculated in 200 mL of DOHLU+Gal culture medium, and was pre-cultured at 30°C for 36 hours. The thus obtained culture solutions were diluted with DOHLU+Glu culture medium so that OD₆₀₀ became 0.200, and then the diluted ones were further diluted with DOHLU+Glu culture medium at a dilution rate of 1/200 to obtain diluted pre-cultured solutions.

A 96-well assay plate was prepared by filling each well with 1 µL of a DMSO solution of an examinee substance (Abscisic acid or 6-Benzyl aminopurine). In this regard, it is to be noted that the DMSO solution of a examinee substance was prepared so that the concentration thereof became 200 ppm. Simultaneously, an assay plate was prepared by filling each well with 1 µL of DMSO as a blank section.

Each of the diluted pre-cultured solutions was added in a volume of 100 µL per each well of both of the assay plates, and was cultured at 30°C for 24 hours. After incubation, the turbidity of each well was measured using a plate reader. The absorbance (OD₆₀₀) of each of the culture solutions of the transformed cells containing the examinee substance was shown in Table 4. The transformed cells TM182-CYC1-CYC1IPT4, TM182-CYC1-ADHIPT4, TM182-CYC1-CYC1IPT5 and TM182-CYC1-ADHIPT5 were not observed to grow whichever examinee substance was added. On the other hand, the transformed cells TM182-CRE1-ADHIPT4, TM182-AHK2-CYC1IPT4, TM182-AHK2-CYC1IPT5 and TM182-AHK2-ADHIPT5 were observed to grow whichever examinee substance was added. Further, the growth speeds of the transformed cells TM182-CRE1-ADHIPT4, TM182-AHK2-CYC1IPT4, TM182-AHK2-CYC1IPT5 and TM182-AHK2-ADHIPT5 in the culture medium to which 6-Benzyl aminopurine was added were faster than those in the culture medium to which only DMSO was added. This indicates that the transformed cells TM182-CRE1-ADHIPT4, TM182-AHK2-CYC1IPT4, TM182-AHK2-CYC1IPT5 and TM182-AHK2-ADHIPT5 respond to cytokinins synthesized by the cells and can grow in DOHLU+Glu culture medium and that the growth speeds of the transformed cells TM182-CRE1-ADHIPT4, TM182-AHK2-CYC1IPT4, TM182-AHK2-CYC1IPT5 and TM182-AHK2-ADHIPT5 are increased or decreased depending on the amount of cytokinin.

**Table 4**

| Examinee substance | TM182-CYC1-CYC1-IPT4 | TM182-CYC1-ADH-IPT4 | TM182-CYC1-CYC1-IPT5 | TM182-CYC1-ADH-IPT5 | TM182-CRE1-ADH-IPT4 | TM182-AHK2-CYC1-IPT4 | TM182-AHK2-CYC1-IPT5 | TM182-AHK2-ADH-IPT5 |
|---|---|---|---|---|---|---|---|---|
| DMSO | 0.035 | 0.035 | 0.035 | 0.035 | 0.044 | 0.199 | 0.071 | 0.301 |
| Abscisic acid | 0.035 | 0.035 | 0.036 | 0.035 | 0.045 | 0.194 | 0.069 | 0.297 |
| 6-Benzyl amino-purine | 0.034 | 0.034 | 0.035 | 0.035 | 0.058 | 0.339 | 0.430 | 0.427 |

### (Example 21) Use of transformed cells of the present invention: method for searching substances having activity of controlling cytokinin biosynthesis

The transformed cell TM182-CRE1-ADHIPT5 obtained in Example 16 is inoculated in 200 mL of DOHLU+Gal culture medium, and is then pre-cultured at 30°C for 36 hours. The culture solution is diluted with DOHLU+Glu culture medium so that OD₆₀₀ become 0.200. The diluted one is further diluted with DOHLU+Glu culture medium at a dilution ratio of 1/200 to obtain a diluted pre-cultured solution. Similarly, TM182 *(sln1 Δ*), a *SLN1* genetically defected strain in which *PTP2* Tyrosine phosphatase gene is introduced, is inoculated in 200 mL of DOLU+Gal culture medium, and is then pre-cultured at 30°C for 36 hours. The culture solution is diluted with DOLU+Gal culture medium so that OD₆₀₀ become 0.100. The diluted one is further diluted with DOLU+Gal culture medium at a dilution ratio of 1/200 to obtain a diluted pre-cultured solution. Further, the transformed cell TM182-CRE1 obtained in Reference Example 1 is inoculated in 200 mL of DOLU+Gal culture medium, and is then pre-cultured at 30°C for 36 hours. The culture solution is diluted with DOLU+Glu culture medium so that OD₆₀₀ become 0.100. The diluted one is further diluted with DOLU+Glu culture medium at a dilution ratio of 1/200 to obtain a diluted pre-cultured solution. A diluted pre-cultured solution of transformed cell TM182-CRE1 to which 6-Benzyl aminopurine (cytokinin) is added is also prepared so that the concentration of the 6-Benzyl aminopurine become 2 ppm.

A 96-well assay plate is prepared by filling each well with 1 µL of a DMSO solution of a examinee substance. In this regard, it is to be noted that the DMSO solution of a examinee substance is prepared so that the concentration thereof become 200 ppm. Simultaneously, an assay plate is prepared by filling each well with 1 µL of DMSO as a blank section.

Each of the diluted pre-cultured solutions is added in a volume of 100 µL per each well of both of the assay plates, and is cultured at 30°C for 24 hours. After incubation, the turbidity of each well is measured using a plate reader. In a case where it is confirmed that the growth speed of the transformed cell TM182-CRE1-ADHIPT5 in a well containing a examinee substance is increased or decreased as compared with that in a well of the blank section to which only DMSO is added, the examinee substance contained in the well can be selected as a substance having the activity of controlling cytokinin biosynthesis. Incidentally, in a case where, in a well containing the examinee substance which is confirmed to increase or decrease the growth speed of the transformed cell TM182-CRE1-ADHIPT5 as compared with that in the blank section to which only DMSO is added, the growth speed of the transformed cell TM182-CRE1 is not increased or decreased as compared with that in the blank section to which only DMSO is added, the examinee substance can be selected as a substance that has the activity of controlling cytokinin biosynthesis and that may not directly affect signal transduction system from cytokinin receptor. Further, in a case where, in a well containing the examinee substance which is confirmed to increase or decrease the growth speed of the transformed cell TM182-CRE1-ADHIPT5 as compared with that in the blank section to which only DMSO is added, the growth speed of the transformed cell TM182 *(s1n1 Δ*) is not increased or decreased as compared with that in the blank section to which only DMSO is added, the examinee substance can be selected as a substance that has the activity of controlling cytokinin biosynthesis and that may not affect somewhere not involved in biosynthesis of cytokinin nor signal transduction from cytokinin receptor.

Hereinafter, the medium compositions to be used in the present invention will be described:

### (a) DOLU + Glu culture medium

| | |
|---|---|
| Bacto-yeast nitrogen base without amino acids | 6.7g |
| Glucose | 20g |
| Drop-out mix (x) | 2.0g |
| Distilled water | 1000ml |

### (b)DOLU + Gal culture medium

| | |
|---|---|
| Bacto-yeast nitrogen base without amino acids | 6.7g |
| Galactose | 20g |
| Drop-out mix (x) | 2.0g |
| Distilled water | 1000ml |

### (x) Drop-out mix: Drop-out mix is a combination of the following ingredients.

| | | | |
|---|---|---|---|
| Adenine | 0.5g | Lysine | 2.0g |
| Alanine | 2.0g | Methionine | 2.0g |
| Arginine | 2.0g | para-Aminobenzoic acid | 0.2g |
| Asparagine | 2.0g | Phenylalanine | 2.0g |
| Aspartic acid | 2.0g | Proline | 2.0g |
| Cysteine | 2.0g | Serine | 2.0g |
| Glutamine | 2.0g | Threonine | 2.0g |
| Glutamic acid | 2.0g | Trytophan | 2.0g |
| Glycine | 2.0g | Tyrosine | 2.0g |
| Histidine | 2.0g | Valine | 2.0g |
| Inositol | 2.0g | Isoleucine | 2.0g |

### (c) DOLU + Glu agar culture medium

A solid culture medium prepared by adding 2% (W/V) of agar into the culture medium (a)

### (d) DOLU + Gal agar culture medium

A solid culture medium prepared by adding 2% (W/V) of agar into the culture medium (c)

### (e) DOHLU + Glu culture medium

| | |
|---|---|
| Bacto-yeast nitrogen base without amino acids | 6.7g |
| Glucose | 20g |
| Drop-out mix (y) | 2.0g |

| | |
|---|---|
| Distilled water | 1000ml |

### (f)DOHLU + Gal culture medium

- Bacto-yeast nitrogen base without amino acids: 6.7g
- Galactose: 20g
- Drop-out mix (y): 2.0g
- Distilled water: 1000ml

### (y) Drop-out mix: Drop-out mix is a combination of the following ingredients.

| | | | |
|---|---|---|---|
| Adenine | 0.5g | Lysine | 2.0g |
| Alanine | 2.0g | Methionine | 2.0g |
| Arginine | 2.0g | para-Aminobenzoic acid | 0.2g |
| Asparagine | 2.0g | Phenylalanine | 2.0g |
| Aspartic acid | 2.0g | Proline | 2.0g |
| Cysteine | 2.0g | Serine | 2.0g |
| Glutamine | 2.0g | Threonine | 2.0g |
| Glutamic acid | 2.0g | Trytophan | 2.0g |
| Glycine | 2.0g | Tyrosine | 2.0g |
| Inositol | 2.0g | Valine | 2.0g |
| Isoleucine | 2.0g | | |

### (g) DOHLU + Glu agar culture medium

A solid culture medium prepared by adding 2% (W/V) of agar into the culture medium (e)

### (h) DOHLU + Gal agar culture medium

A solid culture medium prepared by adding 2% (W/V) of agar into the culture medium (f)

### Free text in Sequence Listing

SEQ ID No: 23
Designed oligonucleotide primer for PCR
SEQ ID No: 24
Designed oligonucleotide primer for PCR
SEQ ID No: 25
Designed oligonucleotide primer for PCR
SEQ ID No: 26
Designed oligonucleotide primer for PCR
SEQ ID No: 27
Designed oligonucleotide primer for PCR
SEQ ID No: 28
Designed oligonucleotide primer for PCR
SEQ ID No: 29
Designed oligonucleotide primer for PCR
SEQ ID No: 30
Designed oligonucleotide primer for PCR
SEQ ID No: 31
Designed oligonucleotide primer for PCR
SEQ ID No: 32
Designed oligonucleotide primer for PCR
SEQ ID No: 33
Designed oligonucleotide primer for PCR
SEQ ID No: 34
Designed oligonucleotide primer for PCR
SEQ ID No: 35
Designed oligonucleotide primer for PCR
SEQ ID No: 36
Designed oligonucleotide primer for PCR
SEQ ID No: 37
Designed oligonucleotide primer for PCR
SEQ ID No: 38
Designed oligonucleotide primer for PCR

## Claims

1. A cell transformed with
a polynucleotide comprising a nucleotide sequence encoding an amino acid sequence of a cytokinin receptor and
a polynucleotide comprising a nucleotide sequence encoding an amino acid sequence of a cytokinin biosynthesis enzyme,
wherein said transformed cell is produced by introducing said polynucleotides into a host cell that is defective in one or more histidine kinases.

2. The transformed cell according to claim 1, wherein said cytokinin receptor is selected from the group consisting of:
(a) a cytokinin receptor having the amino acid sequence represented by SEQ ID NO: 2;
(b) a cytokinin receptor having the amino acid sequence represented by SEQ ID NO: 4;
(c) a cytokinin receptor having the amino acid sequence represented by SEQ ID NO: 6;
(d) a partially transmembrane region-deleted type cytokinin receptor;
(e) a cytokinin receptor having the amino acid sequence represented by amino acids 196 to 1176 of SEQ ID NO: 4;
(f) a cytokinin receptor having the amino acid sequence represented by amino acids 50 to 1176 of SEQ ID NO: 4;
(g) a cytokinin receptor having the amino acid sequence represented by amino acids 32 to 1036 of SEQ ID NO: 6;
(h) a chimera-type cytokinin receptor comprising an extracellular region of the cytokinin receptor, a histidine kinase region of the cytokinin receptor, and a receiver region of the histidine kinase, wherein said extracellular region said transmembrane regions and said histidine kinase region are homogenous to one another and the receiver region is heterogeneous thereto;
(i) a cytokinin receptor having the amino acid sequence of (a), (b), (c), (e), (f), or (g) with deletion, substitution, or addition of one or a plurality of amino acids; and
(j) a cytokinin receptor having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequence represented by SEQ ID NOs: 2, 4 or 6.

3. The transformed cell according to claim 1, wherein said cytokinin biosynthesis enzyme is a isopentenyltransferase.

4. The transformed cell according to claim 1, wherein said cytokinin biosynthesis enzyme is selected from the group consisting of:
(a) a cytokinin biosynthesis enzyme having the amino acid sequence represented by SEQ ID NO: 8;
(b) a cytokinin biosynthesis enzyme having the amino acid sequence represented by SEQ ID NO: 10;
(c) a cytokinin biosynthesis enzyme having the amino acid sequence represented by SEQ ID NO: 12;
(d) a cytokinin biosynthesis enzyme having the amino acid sequence represented by SEQ ID NO: 14;
(e) a cytokinin biosynthesis enzyme having the amino acid sequence represented by SEQ ID NO: 16;
(f) a cytokinin biosynthesis enzyme having the amino acid sequence represented by SEQ ID NO: 18;
(g) a cytokinin biosynthesis enzyme having the amino acid sequence represented by SEQ ID NO: 20;
(h) a cytokinin biosynthesis enzyme having the amino acid sequence represented by SEQ ID NOs: 8, 10, 12, 14, 16, 18, or 20 with deletion, substitution, or addition of one or a plurality of amino acids, and
(i) a cytokinin biosynthesis enzyme having an amino acid sequence encoded by a nucleotide sequence of a polynucleotide, wherein said polynucleotide hybridizes under a stringent condition to a polynucleotide that has a nucleotide sequence complementary to a nucleotide sequence encoding the amino acid sequence represented by SEQ ID NOs: 8, 10, 12, 14, 16, 18, or 20.

5. The transformed cell according to claim 1, wherein said cell is yeast.

6. The transformed cell according to claim 1, wherein said cell is budding yeast.

## Patentansprüche

1. Zelle, die transformiert ist
mit einem Polynucleotid, umfassend eine Nucleotidsequenz, die eine Aminosäuresequenz eines Cytokininrezeptors codiert, und
einem Polynucleotid, umfassend eine Nucleotidsequenz, die eine Aminosäuresequenz des Cytokinin-Biosyntheseenzyms codiert,
wobei die transformierte Zelle durch Einführen der Polynucleotide in eine Wirtszelle erzeugt wird, die fehlerhaft in einer oder mehreren Histidinkinasen ist.

2. Transformierte Zelle gemäß Anspruch 1, wobei der Cytokininrezeptor ausgewählt ist aus der Gruppe bestehend aus:
(a) einem Cytokininrezeptor, der die Aminosäuresequenz wie dargestellt in SEQ ID NO: 2 hat;
(b) einem Cytokininrezeptor, der die Aminosäuresequenz wie dargestellt in SEQ ID NO: 4 hat;
(c) einem Cytokininrezeptor, der die Aminosäuresequenz wie dargestellt in SEQ ID NO: 6 hat;
(d) einem Cytokininrezeptor vom partiell Transmembranregion-deletierten Typ;
(e) einem Cytokininrezeptor, der die Aminosäuresequenz wie dargestellt in Aminosäuren 196 bis 1176 von SEQ ID NO: 4 hat;
(f) einem Cytokininrezeptor, der die Aminosäuresequenz wie dargestellt in Aminosäuren 50 bis 1176 von SEQ ID NO: 4 hat;
(g) einem Cytokininrezeptor, der die Aminosäuresequenz wie dargestellt in Aminosäuren 32 bis 1036 von SEQ ID NO: 6 hat;
(h) einem Cytokininrezeptor vom Chimärentyp, umfassend eine extrazelluläre Region des Cytokininrezeptors, eine Histidinkinaseregion des Cytokininrezeptors, und eine Empfängerregion der Histidinkinase, wobei die extrazelluläre Region, die Transmembranregionen und die Histidinkinaseregion zueinander homogen sind und die Empfängerregion heterogen dazu ist;
(i) einem Cytokininrezeptor, der die Aminosäuresequenz von (a), (b), (c), (e), (f) oder (g) mit Deletion, Substitution oder Addition einer oder einer Mehrzahl von Aminosäuren hat; und
(j) einem Cytokininrezeptor, der eine Aminosäuresequenz hat, die durch eine Nucleotidsequenz eines Polynucleotids codiert wird, wobei das Polynucleotid unter einer stringenten Bedingung an ein Polynucleotid hybridisiert, das eine Nucleotidsequenz hat, die komplementär zu einer Nucleotidsequenz ist, die die Aminosäuresequenz wie dargestellt in SEQ ID NO: 2, 4 oder 6 codiert.

3. Transformierte Zelle gemäß Anspruch 1, wobei das Cytokinin-Biosyntheseenzym eine Isopentenyltransferase ist.

4. Transformierte Zelle gemäß Anspruch 1, wobei das Cytokinin-Biosyntheseenzym ausgewählt ist aus der Gruppe bestehend aus:
(a) einem Cytokinin-Biosyntheseenzym, das die Aminosäuresequenz wie dargestellt in SEQ ID NO: 8 hat;
(b) einem Cytokinin-Biosyntheseenzym, das die Aminosäuresequenz wie dargestellt in SEQ ID NO: 10 hat;
(c) einem Cytokinin-Biosyntheseenzym, das die Aminosäuresequenz wie dargestellt in SEQ ID NO: 12 hat;
(d) einem Cytokinin-Biosyntheseenzym, das die Aminosäuresequenz wie dargestellt in SEQ ID NO: 14 hat;
(e) einem Cytokinin-Biosyntheseenzym, das die Aminosäuresequenz wie dargestellt in SEQ ID NO: 16 hat;
(f) einem Cytokinin-Biosyntheseenzym, das die Aminosäuresequenz wie dargestellt in SEQ ID NO: 18 hat;
(g) einem Cytokinin-Biosyntheseenzym, das die Aminosäuresequenz wie dargestellt in SEQ ID NO: 20 hat;
(h) einem Cytokinin-Biosyntheseenzym, das die Aminosäuresequenz wie dargestellt in SEQ ID NO: 8, 10, 12, 14, 16, 18 oder 20 mit Deletion, Substitution oder Addition einer oder einer Mehrzahl von Aminosäuren hat; und
(i) einem Cytokinin-Biosyntheseenzym, das die Aminosäuresequenz hat, die durch eine Nucleotidsequenz eines Polynucleotids codiert wird, wobei das Polynucleotid unter einer stringenten Bedingung an ein Polynucleotid hybridisiert, das eine Nucleotidsequenz hat die komplementär zu einer Nucleotidsequenz ist, die die Aminosäuresequenz wie dargestellt in SEQ ID NO: 8, 10, 12, 14, 16, 18 oder 20 codiert.

5. Transformierte Zelle gemäß Anspruch 1, wobei die Zelle Hefe ist.

6. Transformierte Zelle gemäß Anspruch 1, wobei die Zelle sprossende Hefe ist.

## Revendications

1. Cellule transformée avec
un polynucléotide comprenant une séquence de nucléotides encodant une séquence d'acides aminés d'un récepteur de la cytokinine et
un polynucléotide comprenant une séquence de nucléotides encodant une séquence d'acides aminés d'une enzyme de biosynthèse de la cytokinine,
dans laquelle ladite cellule transformée est produite en introduisant lesdits polynucléotides dans une cellule hôte qui manque d'une ou plusieurs histidine kinases.

2. Cellule transformée selon la revendication 1, dans laquelle ledit récepteur de la cytokinine est choisi dans le groupe comprenant :
(a) un récepteur de la cytokinine ayant la séquence d'acides aminés représentée par SEQ ID NO : 2 ;
(b) un récepteur de la cytokinine ayant la séquence d'acides aminés représentée par SEQ ID NO : 4;
(c) un récepteur de la cytokinine ayant la séquence d'acides aminés représentée par SEQ ID NO : 6;
(d) un récepteur de la cytokinine de type à délétion partielle de la région transmembranaire;
(e) un récepteur de la cytokinine ayant la séquence d'acides aminés représentée par les acides aminés 196 à 1176 de SEQ ID NO : 4 ;
(f) un récepteur de la cytokinine ayant la séquence d'acides aminés représentée par les acides aminés 50 à 1176 de SEQ ID NO : 4 ;
(g) un récepteur de la cytokinine ayant la séquence d'acides aminés représentée par les acides aminés 32 à 1036 de SEQ ID NO : 6 ;
(h) un récepteur de la cytokinine de type chimère comprenant une région extracellulaire du récepteur de la cytokinine, une région histidine kinase du récepteur de la cytokinine, et une région réceptrice de l'histidine kinase, dans lequel ladite région extracellulaire, lesdites régions transmembranaires et ladite région histidine kinase sont homogènes entre elles et la région réceptrice est hétérogène avec elles ;
(i) un récepteur de la cytokinine ayant la séquence d'acides aminés de (a), (b), (c), (e), (f), ou (g) avec la délétion, la substitution, ou l'addition d'un ou plusieurs acides aminés ; et
(j) un récepteur de la cytokinine ayant une séquence d'acides aminés encodée par une séquence de nucléotides d'un polynucléotide, dans lequel ledit polynucléotide s'hybride sous une condition stringente à un polynucléotide qui possède une séquence de nucléotides complémentaire d'une séquence de nucléotides encodant la séquence d'acides aminés représentée par les SEQ ID NOs : 2, 4 ou 6.

3. Cellule transformée selon la revendication 1, dans laquelle ladite enzyme de biosynthèse de la cytokinine est une isopentényltransférase.

4. Cellule transformée selon la revendication 1, dans laquelle ladite enzyme de biosynthèse de la cytokinine est choisie dans le groupe comprenant :
(a) une enzyme de biosynthèse de la cytokinine ayant la séquence d'acides aminés représentée par SEQ ID NO : 8 ;
(b) une enzyme de biosynthèse de la cytokinine ayant la séquence d'acides aminés représentée par SEQ ID NO : 10 ;
(c) une enzyme de biosynthèse de la cytokinine ayant la séquence d'acides aminés représentée par SEQ ID NO : 12 ;
(d) une enzyme de biosynthèse de la cytokinine ayant la séquence d'acides aminés représentée par SEQ ID NO : 14 ;
(e) une enzyme de biosynthèse de la cytokinine ayant la séquence d'acides aminés représentée par SEQ ID NO : 16 ;
(f) une enzyme de biosynthèse de la cytokinine ayant la séquence d'acides aminés représentée par SEQ ID NO : 18 ;
(g) une enzyme de biosynthèse de la cytokinine ayant la séquence d'acides aminés représentée par SEQ ID NO : 20 ;
(h) une enzyme de biosynthèse de la cytokinine ayant la séquence d'acides aminés représentée par les SEQ ID NOs : 8, 10, 12, 14, 16, 18, ou 20 avec la délétion, la substitution, ou l'addition d'un ou plusieurs acides aminés, et
(i) une enzyme de biosynthèse de la cytokinine ayant une séquence d'acides aminés encodée par une séquence de nucléotides d'un polynucléotide, dans laquelle ledit polynucléotide s'hybride sous une condition stringente à un polynucléotide qui possède une séquence de nucléotides complémentaire d'une séquence de nucléotides encodant la séquence d'acides aminés représentée par les SEQ ID NOs : 8, 10, 12, 14, 16, 18, ou 20.

5. Cellule transformée selon la revendication 1, dans laquelle ladite cellule est une levure.

6. Cellule transformée selon la revendication 1, dans laquelle ladite cellule est une levure bourgeonnante.
